# EUROPEAN PATENT APPLICATION

(11) **EP 3 875 456 A1**
(43) Date of publication of application: **08.09.2021**
(21) Application number: 20160426.1
(22) Date of filing: 02.03.2020
(51) Int. Cl.: C07D 417/14, C07D 401/14, A61P 25/00, A61P 25/18, A61P 25/22, A61P 25/24

(54) **HETERO-BIFUNCTIONAL PROTEOLYSIS-TARGETING CHIMERAS (PROTACS) FOR THE SELECTIVE DEGRADATION OF FK506-BINDING PROTEINS (FKBPS)**

(71) Applicant: Technische Universität Darmstadt, 64289 Darmstadt (DE)
(72) Inventor: HAUSCH, Felix, 64354 Reinheim (DE); HÄHLE, Andreas, 90425 Nürnberg (DE); MAO, Tianqi, 200020 Shanghai (CN)
(74) Representative: Fuchs Patentanwälte Partnerschaft mbB

(57) **Abstract**

The present invention relates to hetero-bifunctional proteolysis-targeting chimeras (PROTACs), which enable the selective degradation of FK506-binding proteins (FKBPs). The molecules of the present invention consist of a ligand for recruiting an E3 ligase, a linker-system, and a second ligand, which selectively binds to FKBP51. As such, the FKBP is targeted by the PROTAC for degradation. Said hetero-bifunctional PROTACs are useful for the treatment of psychiatric disorders, metabolic disorders, pain diseases, and/or cancers.

## Description

The present invention relates to hetero-bifunctional proteolysis-targeting chimeras (PROTACs), which enable the selective degradation of FK506-binding proteins (FKBPs). The molecules of the present invention consist of a ligand for recruiting an E3 ligase, a linker-system, and a second ligand, which selectively binds to FKBP51 targeted for degradation. Said hetero-bifunctional PROTACs are useful for the treatment of psychiatric disorders, metabolic disorders, pain diseases, and cancers.

### Background of the invention

FK506-binding proteins (FKBPs) are part of the immunophillin family and best known for their immunosuppressive activity as complexes with the natural products FK506 and rapamycin. In addition, FKBPs are prominently expressed in the central nervous system and non-immunosuppressive FKBP ligands have repeatedly displayed neuroprotective and neurotrophic effects.

Among the human FKBPs, FKBP51 has gained particular interest as a regulator of stress-coping behaviour and as a risk factor for stress-related psychiatric disorders, as a risk factor promoting chronic pain states, and a regulator of metabolic processes, thus suggesting FKBP51 inhibition as a potential therapeutic approach for indications related to these biological systems. However, the development of potent drug-like inhibitors for FKBPs in general and for FKBP51 in particular is challenging owing to the shallow FK506 binding site.

In the past, inhibitors of FKBPs have been described. For example bicyclic aza-amides derivatives and stereoisomeric forms, as described in WO/2014/015993. Said bicyclic aza-amides derivatives have been identified as specific inhibitors of FKBPs, including FKBP51, are useful for the treatment of psychiatric disorders and neurodegenerative diseases, disorders and conditions, for treating vision disorders and/or improving vision; for treating memory impairment and/or enhancing memory performance and for treating alopecia and promoting hair growth.

WO2015/110271 describes Diazabicyclo-[4.3.1]-decane derivatives as potent inhibitors of FKBP-function, with very potent binding to certain members of FKBP-family, such as FKBP51, for example.

However, the inhibitors known in the prior art only block the binding pocket and cannot completely inactivate the very complex activities of FKBPs. Thus, despite inhibition still certain activities of FKBP51 could be measured.

Therefore, it is the object of the present invention to provide compounds and/or pharmaceutically acceptable salts thereof, which selectively bind to a defined FKBP-member, such as FKBP 51, and facilitate the degradation of the FKBP by channelling it to the ubiquitin protease degradation pathway. As such, the target protein is eliminated rather than just inhibited, enabling enhanced therapeutic applications of the inventive molecules.

The hetero-bifunctional PROTACs presented herein are the first description of molecules, which enable the selective degradation of FKPB51. The molecules consist of a ligand for recruiting an E3 ligase, an inventive linker-system, and a second ligand, which selectively binds to the FKBP51 to be targeted for degradation.

### Description of the invention

### Proteolysis-targeting chimeras (PROTACS)

In the recent decade, proteolysis-targeting chimeras (PROTACs) has emerged as a useful chemical knockdown strategy and has a promising application prospect in clinical therapies for diseases. PROTACs are a new class of heterobifunctional molecules consisting of a recognition motif for binding the protein of interest (POI), a recognition motif for recruiting an E3 ligase and a linker to tether the first two together. By binding to the substrate and the E3 ligase complex simultaneously, PROTACs are able to bring them into proximity to induce poly-ubiquitination of the substrate, leading to the recognition by the 26S proteasome and proteasomal degradation.

According to the present invention, the proteolysis-targeting chimera (PROTAC) for degradation of FK506 binding proteins (FKBPs), comprising a FKBP-binding moiety and E3-binding moiety connected via a Linker (**L**), are represented by the following general formula 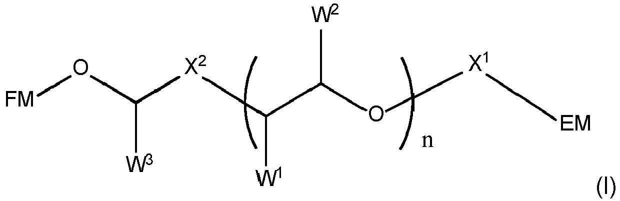
- wherein **n** is an integer from 0 to 10,
- **X¹** represents a bond, -CH₂(C=O)NH-,-CH₂(C=O)NMe-, -CH(CH₃)(C=O)NH-,-CH(CH₃)(C=O)NMe-, -CH₂-,-C(O)-, -(C=O)NH-, -NH(C=O)-, -(C=O)NHCH₂-,-(C=O)NMeCH₂-,-NH(C=O)CH₂-,;
- **X²** represents 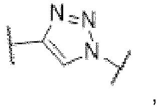 -(C=O)NH- or -CH2NH(C=O)-; -CH₂O-, -CH(CH₃)O-,
- wherein **W¹, W²** and **W³** independently represent -H and/or -CH₃,
- wherein the "FKBP-binding moiety" (**FM**) represents a ligand capable of binding to FK506 binding proteins (FKBPs),
- wherein the "E3-binding moiety" (**EM**) represents a ligand capable of binding an E3 ubiquitin ligase,
and wherein, upon binding of the PROTAC to the FKBP and the E3 ubiquitin ligase, the FKBP is degraded by a ubiquitin mediated proteolysis pathway within a cell;
and enantiomers, stereoisomeric forms, mixtures of enantiomers, anomers, deoxy-forms, diastereomers, mixtures of diastereomers, prodrugs, tautomers, hydrates, solvates and racemates of the above mentioned compounds and pharmaceutically acceptable salts thereof.

The expression "degradation" means herein intracelluar degradation of a target-substrate, preferably an FKBP after being poly-ubiquitinated and recognized by the 26S proteasome. In some parts of this description also the expression "elimination" is used to describe the elimination of the functional substrate from the cell by using the before-mentioned proteasomal degradation pathway. A substrate is envisaged to be "degradated" and/or "eliminated" if less than 25%, less than 15%, less than 10%, less than 5% of the original substrate is detectable or still present in a cell in functional active form after being incubated with the degrading molecule.

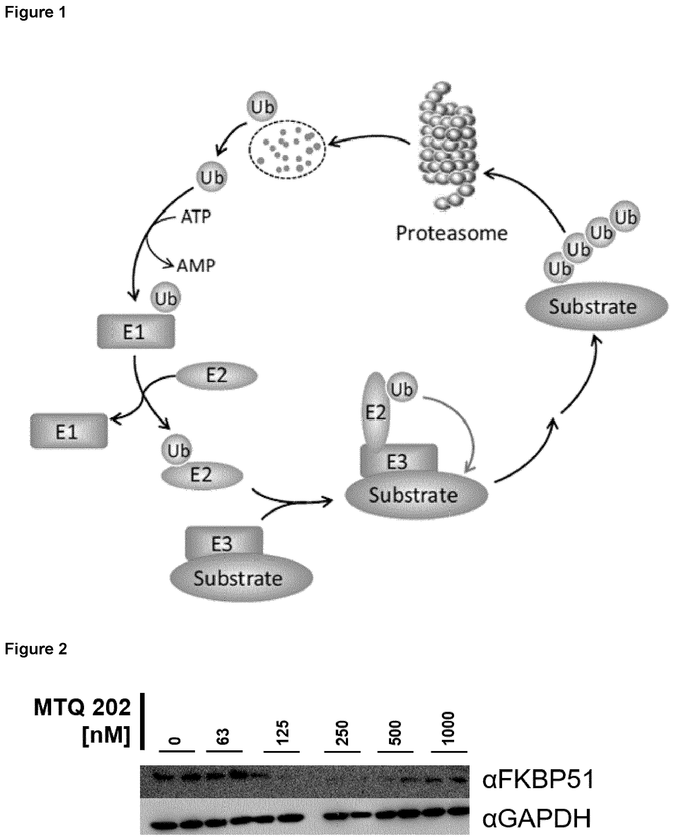

The term "ubiquitin mediated proteolysis pathway" is used for the ubiquitin-proteasome system as depicted exemplarily in Figure 1.

The PROTACs of the present invention also encompass derivatives and stereoisomeric forms, solvates, hydrates and/or pharmaceutically acceptable salts of these compounds as well as pharmaceutical compositions containing at least one of these PROTACs together with pharmaceutically acceptable carrier, excipient and/or diluents.

Said PROTACs have been identified as specifically degrading members of the family of the FK506 binding proteins (FKBPs), especially FKBP51, and are useful for the treatment of psychiatric disorders, such as depression and post-traumatic stress disorder, metabolic disorders such as obesity or diabetes, pain diseases such as chronic pain or fibromyalgia, and cancers such as prostate cancer or glioma or malignant melanoma.

In some embodiments, the integer (n), which refers to the number of glycol units of the linker region, has the value of 1 to 8, in other embodiments 2 to 6, in other embodiments 3 to 5, and yet in another embodiment 4.

### FKBP-binding moiety (FM)

The FK506-binding protein (FKBP) family of immunophilins consists of proteins with a variety of protein-protein interaction domains and versatile cellular functions. This highly conserved protein family binds to immunosuppressive drugs, such as FK506 and rapamycin. This protein family displays peptidyl propyl isomerase (PPIase) activity.

The immunosuppressant drugs FK506 and rapamycin are well known as potent T-cell specific immunosuppressants, and are effective against autoimmunity, transplant or graft rejection, inflammation, allergic responses, other autoimmune or immune-mediated diseases, and infectious diseases.

FK506 and rapamycin apart from binding to FKBP12 also interact and inhibit calcineurin (CaN) and mTOR respectively thereby mediating their immunosuppressive action.

The high molecular weight multidomain homolog FKBP51 acts as co chaperons for the heat shock protein (Hsp90) and modulates the signal transduction of the glucocorticoid receptor by participating in the Heat shock protein 90 (Hsp90) - steroid receptor complex.

FKBP51 has emerged as a key player in human stress biology and plays an important role in depression, obesity, glucose homeostasis, chronic pain states and certain cancers. Human genetic studies have clearly validated FKBP51 as a risk factor for mood disorders like depression. Characterization of FKBP51-deficient mice revealed that FKBP51 plays a prominent role in the feedback control of the hypothalamus-pituitary-adrenal axis, a key stress response system in mammals. Furthermore, FKBP51^{-/-}-mice had an improved sleep profile and enhanced glucose tolerance, were resistant to diet-induced obesity, and were protected from experimentally induced forms of chronic pain. Importantly, no potentially adverse effects were observed in FKBP51^{-/-}-mice so far, suggesting FKBP51 as a novel and safe drug target for depression, obesity or chronic pain.

The immunosuppressive compounds, like FK506, disclosed in the prior art suppress the immune system, by definition, and also exhibit other toxic side effects. Accordingly, there is a need for non-immunosuppressant, small molecule compounds, and compositions and methods for use of such compounds, that are useful in treating psychiatric disorders and neurodegenerative diseases, disorders and conditions.

Further studies led to α-ketoamide analogs of FK506 devoid of immunosuppressive activity. So far there has been only few investigations on the activity of monocyclic, pipecolate or proline-based compounds concerning FKBP51.

Also, the main physiological role of FKBP51 is believed to be the inhibition of glucocorticoid receptor signalling, especially in stressful situations. However, the FKBP51-GR interplay (glucocorticoid receptor interplay) has been difficult to assess pharmacologically, largely due to lack of appropriate chemical probes. The most selective and most wide used compound to study FKBP51 are compounds from the SAFit class such as SAFit2. These compounds block the FK506-binding site on FKBP51, but do not affect other functional domains such as the HSP90-binding domain (TPR domain).

### General Formula

The compound of the present invention is a ligand capable of binding to FK506 binding proteins (FKBPs) (FM) and has the general structure of: 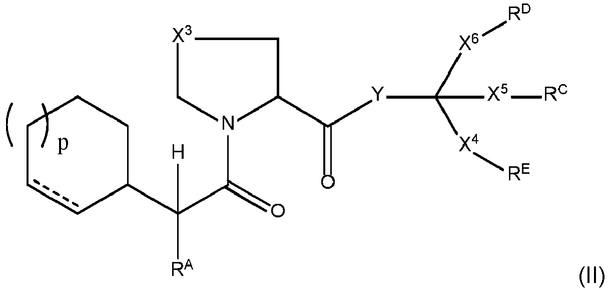 wherein
**X3** represents -CH₂-, -CH₂CH₂-, -CH=CH-, -CH₂-S-, or -S-CH₂-;
**Y** represents -NH-, or -O-;
**p** is an integer of 0 or 1;
" 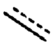 " represents a C=C bond or a C-C bond;
wherein **R^{E}** represents **R^{L}**;
and **R^{A}** represents 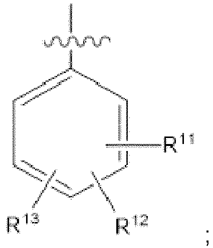
**R^{L}** represents: 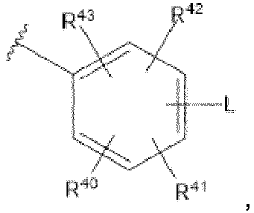 wherein **L** represents the chemical linker **L** depicted below.
**R^{c}** represents: **R²⁷** or 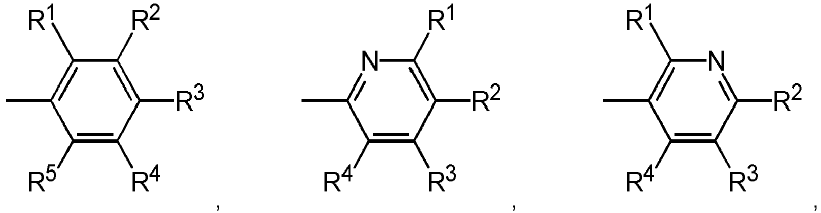 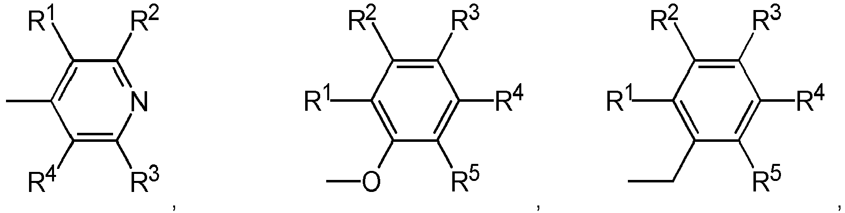 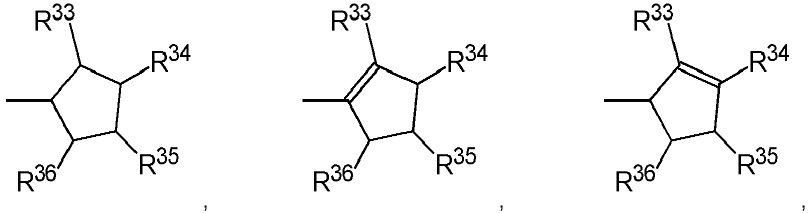 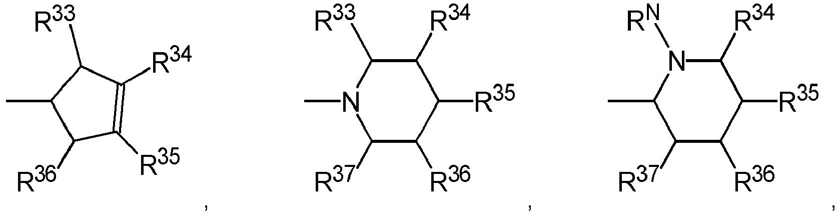 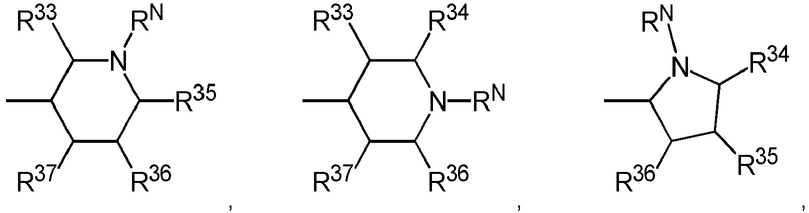 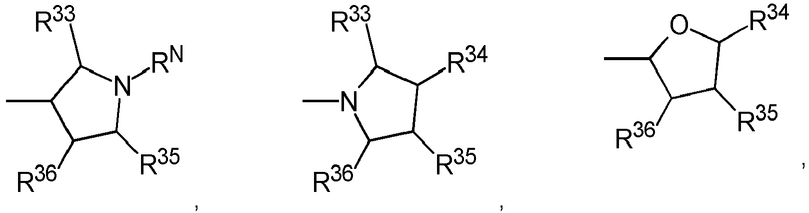 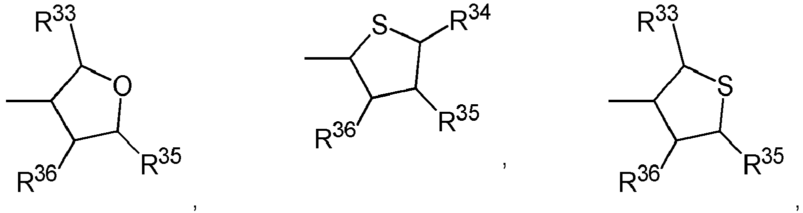 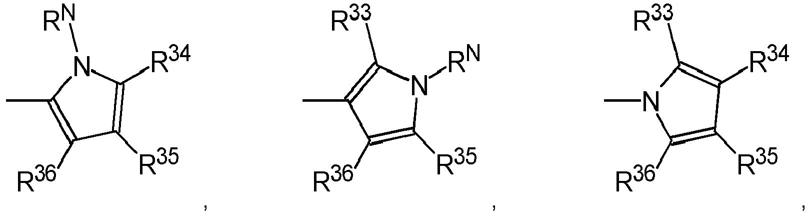 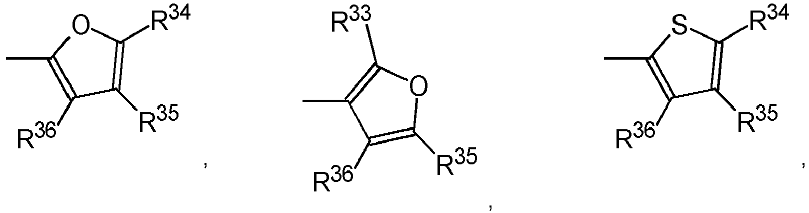 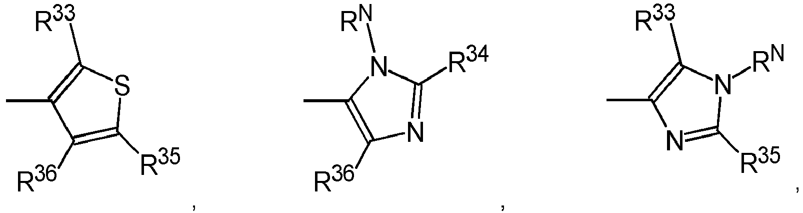 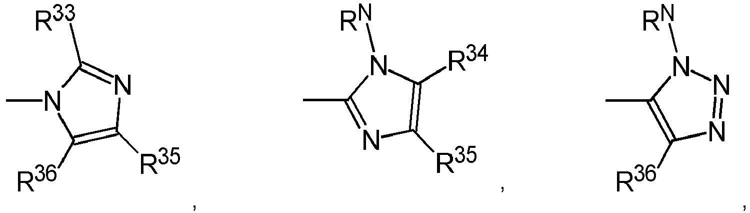 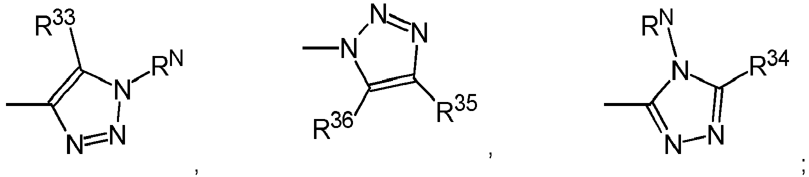
**R^{D}** represents: **R²⁸** or 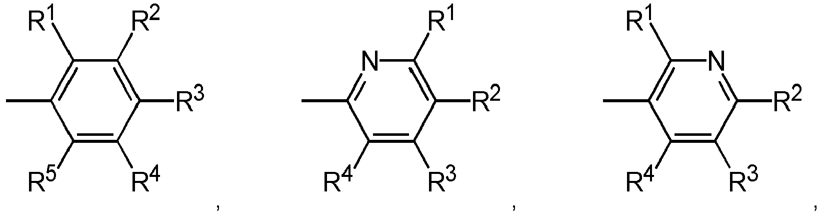 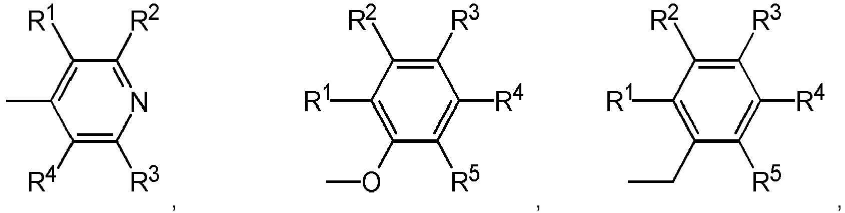 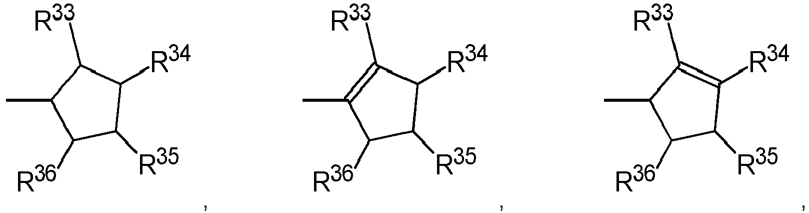 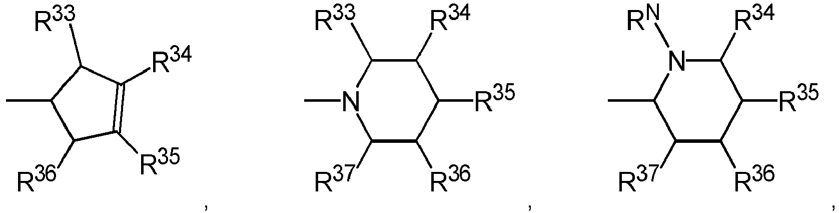 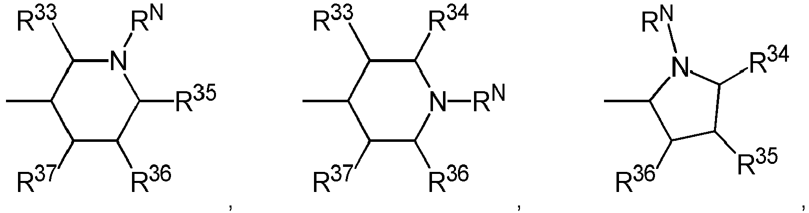 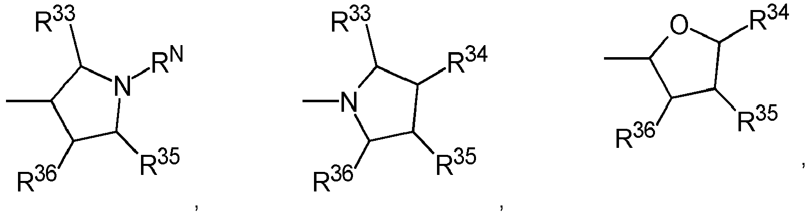 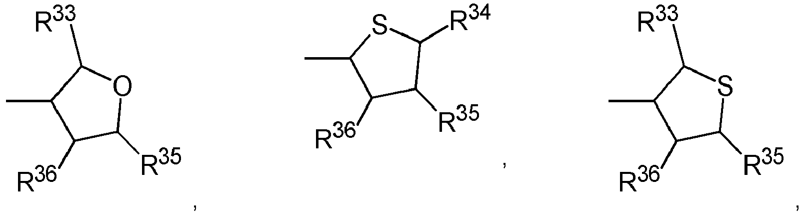 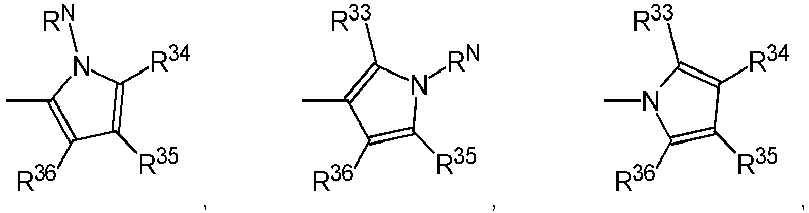 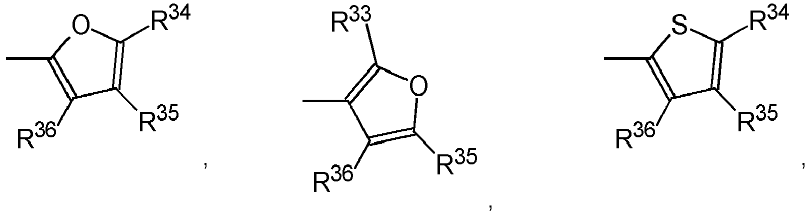 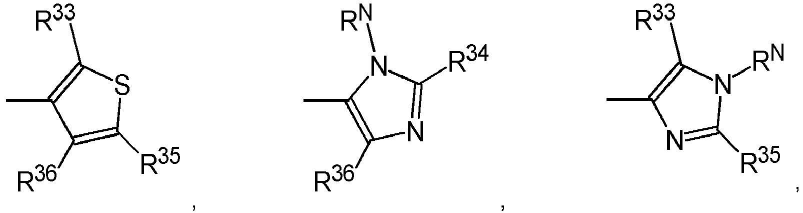 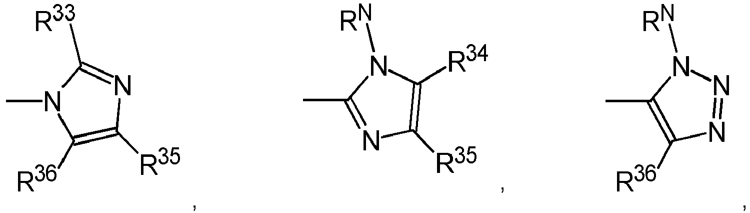 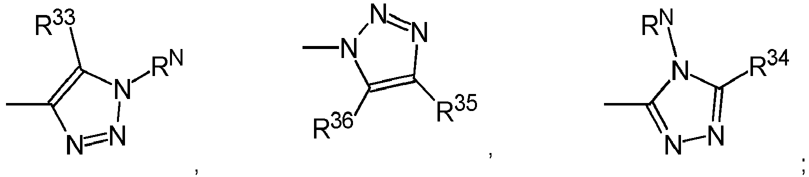
**R¹- R²², R^{18'}** - **R^{22'}, R²⁶** - **R⁴³** represent independently of each other -H, -OH, -OCH₃, -OC₂H₅, -OC₃H₇, -O-cyclo-C₃H₅, -OCH(CH₃)₂, -OC(CH₃)₃, -OC₄H₉, -OCH₂-COOH, -OPh, -OCH₂-Ph, -OCPh₃, -CH₂-OH, -C₂H₄-OH, -C₃H₆-OH, -CH(OH)-CH₂-OH, -CH₂-OCH₃, -C₂H₄-OCH₃, -C₃H₆-OCH₃, -CH₂-OC₂H₅, -C₂H₄-OC₂H₅, -C₃H₆-OC₂H₅, -CH₂-OC₃H₇, -C₂H₄-OC₃H₇, -C₃H₆-OC₃H₇, -CH₂-O-cyclo-C₃H₅, -C₂H₄-O-cyclo-C₃H₅, -C₃H₆-O-cyclo-C₃H₅, -CH₂-OCH(CH₃)₂, -C₂H₄-OCH(CH₃)₂, -C₃H₆-OCH(CH₃)₂, -CH₂-OC(CH₃)₃, -C₂H₄-OC(CH₃)₃, -C₃H₆-OC(CH₃)₃, -CH₂-OC₄H₉, -C₂H₄-OC₄H₉, -C₃H₆-OC₄H₉, -CH₂-OPh, -C₂H₄-OPh, -C₃H₆-OPh, -CH₂-OCH₂-Ph, -C₂H₄-OCH₂-Ph, -C₃H₆-OCH₂-Ph, -SH, -SCH₃, -SC₂H₅, -SC₃H₇, -S-cyclo-C₃H₅, -SCH(CH₃)₂, -SC(CH₃)₃, -NO₂, -F, -CI, -Br, -I, -P(O)(OH)₂, -P(O)(OCH₃)₂, -P(O)(OC₂H₅)₂, -P(O)(OCH(CH₃)₂)₂, -C(OH)[P(O)(OH)₂]₂, -Si(CH₃)₂(C(CH₃)₃), -Si(C₂H₅)₃, -Si(CH₃)₃, -N₃, -CN, -OCN, -NCO, -SCN, -NCS, -CHO, -COCH₃, -COC₂H₅, -COC₃H₇, -CO-cyclo-C₃H₅, -COCH(CH₃)₂, -COC(CH₃)₃, -COOH, -COCN, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CH₂-CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NHCO-OCH₃, -NHCO-OC₂H₅, -NHCO-OC₃H₇, -NHCO-O-cyclo-C₃H₅, -NHCO-OCH(CH₃)₂, -NHCO-OC(CH₃)₃, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -SO₂NH₂, -SO₂NHCH₃, -SO₂NHC₂H₅, -SO₂NHC₃H₇, -SO₂NH-cyclo-C₃H₅, -SO₂NHCH(CH₃)₂, -SO₂NHC(CH₃)₃, -SO₂N(CH₃)₂, -SO₂N(C₂H₅)₂, -SO₂N(C₃H₇)₂, -SO₂N(cyclo-C₃H₅)₂, -SO₂N[CH(CH₃)₂]₂, -SO₂N[C(CH₃)₃]₂, -O-S(=O)CH₃, -O-S(=O)C₂H₅, -O-S(=O)C₃H₇, -O-S(=O)-cyclo-C₃H₅, -O-S(=O)CH(CH₃)₂, -O-S(=O)C(CH₃)₃, -S(=O)(=NH)CH₃, -S(=O)(=NH)C₂H₅, -S(=O)(=NH)C₃H₇, -S(=O)(=NH)-cyclo-C₃H₅, -S(=O)(=NH)CH(CH₃)₂, -S(=O)(=NH)C(CH₃)₃, -NH-SO₂-CH₃, -NH-SO₂-C₂H₅, -NH-SO₂-C₃H₇, -NH-SO₂-cyclo-C₃H₅, -NH-SO₂-CH(CH₃)₂, -NH-SO₂-C(CH₃)₃, -O-SO₂-CH₃, -O-SO₂-C₂H₅, -O-SO₂-C₃H₇, -O-SO₂-cyclo-C₃H₅, -O-SO₂-CH(CH₃)₂, -O-SO₂-C(CH₃)₃, -OCF₃, -CH₂-OCF₃, -C₂H₄-OCF₃, -C₃H₆-OCF₃, -OC₂F₅, -CH₂-OC₂F₅, -C₂H₄-OC₂F₅, -C₃H₆-OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CS-N(C₃H₇)₂, -NH-CO-NHC₃H₇, -NH-CO-N(C₃H₇)₂, -NH-CO-NH[CH(CH₃)₂], -NH-CO-NH[C(CH₃)₃], -NH-CO-N(CH₃)₂, -NH-CO-N(C₂H₅)₂, -NH-CO-NH-cyclo-C₃H₅, -NH-CO-N(cyclo-C₃H₅)₂, -NH-CO-N[CH(CH₃)₂]₂, -NH-CS-N(C₂H₅)₂, -NH-CO-N[C(CH₃)₃]₂, -NH-CS-NH₂, -NH-CS-NHCH₃, -NH-CS-N(CH₃)₂, -NH-CS-NHC₂H₅, -NH-CS-NHC₃H₇, -NH-CS-NH-cyclo-C₃H₅, -NH-CS-NH[CH(CH₃)₂], -NH-CS-NH[C(CH₃)₃], -NH-CS-N(cyclo-C₃H₅)₂, -NH-CS-N[CH(CH₃)₂]₂, -NH-CS-N[C(CH₃)₃]₂, -NH-C(=NH)-NH₂, -NH-C(=NH)-NHCH₃, -NH-C(=NH)-NHC₂H₅, -NH-C(=NH)-NHC₃H₇, -O-CO-NH-cyclo-C₃H₅, -NH-C(=NH)-NH-cyclo-C₃H₅, -NH-C(=NH)-NH[CH(CH₃)₂] -O-CO-NH[CH(CH₃)₂], -NH-C(=NH)-NH[C(CH₃)₃], -NH-C(=NH)-N(CH₃)₂, -NH-C(=NH)-N(C₂H₅)₂, -NH-C(=NH)-N(C₃H₇)₂, -NH-C(=NH)-N(cyclo-C₃H₅)₂, -O-CO-NHC₃H₇, -NH-C(=NH)-N[CH(CH₃)₂]₂, -NH-C(=NH)-N[C(CH₃)₃]₂, -O-CO-NH₂, -O-CO-NHCH₃, -O-CO-NHC₂H₅, -O-CO-NH[C(CH₃)₃], -O-CO-N(CH₃)₂, -O-CO-N(C₂H₅)₂, -O-CO-N(C₃H₇)₂, -O-CO-N(cyclo-C₃H₅)₂, -O-CO-N[CH(CH₃)₂]₂, -O-CO-N[C(CH₃)₃]₂, -O-CO-OCH₃, -O-CO-OC₂H₅, -O-CO-OC₃H₇, -O-CO-O-cyclo-C₃H₅, -O-CO-OCH(CH₃)₂, -O-CO-OC(CH₃)_{3,} -CH₂F, -CHF₂, -CF₃, -CH₂Cl, -CH₂Br, -CH₂I, -CH₂-CH₂F, -CH₂-CHF₂, -CH₂-CF₃, -CH₂-CH₂Cl, -CH₂-CH₂Br, -CH₂-CH₂I, -cyclo-C₅H₉, -cyclo-C₆H₁₁, -CH₂-cyclo-C₆H₁₁, -CH₂-CH₂-cyclo-C₆H₁₁, -cyclo-C₇H₁₃, -cyclo-C₈H₁₅, -Ph, -CH₂-Ph, -CH₂-CH₂-Ph, -CH=CH-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₇, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -C₂H₄-CH(CH₃)-CH=CH₂, -CH₂-CH(CH₃)-CH₂-CH=CH₂, -C₂H₄-CH=C(CH₃)₂, -CH(CH₃)-C₂H₄-CH=CH₂, -C₂H₄-C(CH₃)=CH-CH₃, -CH₂-CH(CH₃)-CH=CH-CH₃, -CH(CH₃)-CH₂-CH=CH-CH₃, -CH₂-CH=CH-CH(CH₃)₂, -CH₂-CH=C(CH₃)-C₂H₅, -CH₂-C(CH₃)=CH-C₂H₅, -CH(CH₃)-CH=CH-C₂H₅, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -C[C(CH₃)₃]=CH₂, -CH(CH₃)-CH₂-C(CH₃)=CH₂, -CH(CH₃)-CH(CH₃)-CH=CH₂, -CH=CH-C₂H₄-CH=CH₂, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH=CH-CH₂-CH=CH-CH₃, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[CH(CH₃)(C₂H₅)]=CH₂, -C[CH₂-CH(CH₃)₂]=CH₂, -C₂H₄-CH=CH-CH=CH₂, -CH₂-CH=CH-CH₂-CH=CH₂, -C₃H₆-C≡C-CH₃, -CH₂-CH=CH-CH=CH-CH₃, -CH=CH-CH=CH-C₂H₅, -CH₂-CH=CH-C(CH₃)=CH₂, -CH₂-CH=C(CH₃)-CH=CH₂, -CH₂-C(CH₃)=CH-CH=CH₂, -CH(CH₃)-CH₂-C≡CH, -CH(CH₃)-CH=CH-CH=CH₂, -CH=CH-CH₂-C(CH₃)=CH₂, -CH(CH₃)-C≡C-CH₃, -CH=CH-CH(CH₃)-CH=CH₂, -CH=C(CH₃)-CH₂-CH=CH₂, -C₂H₄-CH(CH₃)-C≡CH, -C(CH₃)=CH-CH₂-CH=CH₂, -CH=CH-CH=C(CH₃)₂, -CH₂-CH(CH₃)-CH₂-C≡CH, -CH=CH-C(CH₃)=CH-CH₃, -CH=C(CH₃)-CH=CH-CH₃, -CH₂-CH(CH₃)-C≡CH, -C(CH₃)=CH-CH=CH-CH₃, -CH=C(CH₃)-C(CH₃)=CH₂, -C(CH₃)=CH-C(CH₃)=CH₂, -C(CH₃)=C(CH₃)-CH=CH₂, -CH=CH-CH=CH-CH=CH₂, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -C₄H₈-C≡CH, -C₂H₄-C≡C-C₂H₅, -CH₂-C≡C-C₃H₇, -C≡C-C₄H₉, -C≡C-C(CH₃)₃, -CH(CH₃)-C₂H₄-C≡CH, -CH₂-CH(CH₃)-C≡C-CH₃, -CH(CH₃)-CH₂-C≡C-CH₃, -CH(CH₃)-C≡C-C₂H₅, -CH₂-C≡C-CH(CH₃)₂, -C≡C-CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡CH, -CH₂-CH(C≡CH)₂, -C≡C-C≡CH, -CH₂-C≡C-C≡CH, -C≡C-C≡C-CH₃, -CH(C≡CH)₂, -C₂H₄-C≡C-C≡CH, -CH₂-C≡C-CH₂-C≡CH, -C≡C-C₂H₄-C≡CH, -CH₂-C≡C-C≡C-CH₃, -C≡C-CH₂-C≡C-CH₃, -C≡C-C≡C-C₂H₅, -C(C≡CH)₂-CH₃, -C≡C-CH(CH₃)-C≡CH, -CH(CH₃)-C≡C-C≡CH, -CH(C≡CH)-CH₂-C≡CH, -CH(C≡CH)-C≡C-CH₃, 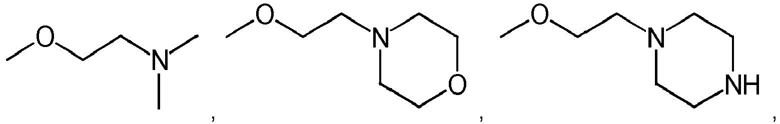 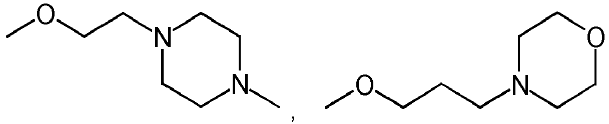 or
**R¹⁸** and **R^{18'}** or **R¹⁹** and **R^{19'}** or **R²⁰** and **R^{20'}** or **R²¹** and **R^{21'}** or **R²²** and **R^{22'}** can form together 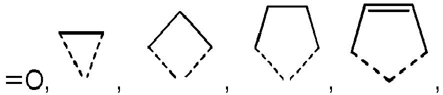 or =C**R^{23'}R^{24'}**, wherein **R^{23'}** and **R^{24'}** represent independently of each other -H, -CH₃, -C₂H₅, -CF₃, -CH₂CF₃, -C₂F₅;
**R²³-R²⁵** represent independently of each other -H, -CH₂-OCH₃, -C₂H₄-OCH₃, -C₃H₆-OCH₃, -CH₂-OC₂H₅, -C₂H₄-OC₂H₅, -C₃H₆-OC₂H₅, -CH₂-OC₃H₇, -C₂H₄-OC₃H₇, -C₃H₆-OC₃H₇, -CH₂-O-cyclo-C₃H₅, -C₂H₄-O-cyclo-C₃H₅, -C₃H₆-O-cyclo-C₃H₅, -CH₂-OCH(CH₃)₂, -C₂H₄-OCH(CH₃)₂, -C₃H₆-OCH(CH₃)₂, -CH₂-OC(CH₃)₃, -C₂H₄-OC(CH₃)₃, -C₃H₆-OC(CH₃)₃, -CH₂-OC₄H₉, -C₂H₄-OC₄H₉, -C₃H₆-OC₄H₉, -CH₂-OPh, -C₂H₄-OPh, -C₃H₆-OPh, -CH₂-OCH₂-Ph, -C₂H₄-OCH₂-Ph, -C₃H₆-OCH₂-Ph, -CH₂F, -CHF₂, -CF₃, -CH₂Cl, -CH₂Br, -CH₂I, -CH₂-CH₂F, -CH₂-CHF₂, -CH₂-CF₃, -CH₂-CH₂Cl, -CH₂-CH₂Br, -CH₂-CH₂I, -cyclo-C₈H₁₅, -Ph, -CH₂-Ph, -CH₂-CH₂-Ph, -CH=CH-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₇, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -C₂H₄-CH(CH₃)-CH=CH₂, -CH₂-CH(CH₃)-CH₂-CH=CH₂, -C₂H₄-CH=C(CH₃)₂, -CH(CH₃)-C₂H₄-CH=CH₂, -C₂H₄-C(CH₃)=CH-CH₃, -CH₂-CH(CH₃)-CH=CH-CH₃, -CH(CH₃)-CH₂-CH=CH-CH₃, -CH₂-CH=CH-CH(CH₃)₂, -CH₂-CH=C(CH₃)-C₂H₅, -CH₂-C(CH₃)=CH-C₂H₅, -CH(CH₃)-CH=CH-C₂H₅, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -C[C(CH₃)₃]=CH₂, -CH(CH₃)-CH₂-C(CH₃)=CH₂, -CH(CH₃)-CH(CH₃)-CH=CH₂, -CH=CH-C₂H₄-CH=CH₂, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH=CH-CH₂-CH=CH-CH₃, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[CH(CH₃)(C₂H₅)]=CH₂, -C[CH₂-CH(CH₃)₂]=CH₂, -C₂H₄-CH=CH-CH=CH₂, -CH₂-CH=CH-CH₂-CH=CH₂, -C₃H₆-C≡C-CH₃, -CH₂-CH=CH-CH=CH-CH₃, -CH=CH-CH=CH-C₂H₅, -CH₂-CH=CH-C(CH₃)=CH₂, -CH₂-CH=C(CH₃)-CH=CH₂, -CH₂-C(CH₃)=CH-CH=CH₂, -CH(CH₃)-CH₂-C≡CH, -CH(CH₃)-CH=CH-CH=CH₂, -CH=CH-CH₂-C(CH₃)=CH₂, -CH(CH₃)-C≡C-CH₃, -CH=CH-CH(CH₃)-CH=CH₂, -CH=C(CH₃)-CH₂-CH=CH₂, -C₂H₄-CH(CH₃)-C≡CH, -C(CH₃)=CH-CH₂-CH=CH₂, -CH=CH-CH=C(CH₃)₂, -CH₂-CH(CH₃)-CH₂-C≡CH, -CH=CH-C(CH₃)=CH-CH₃, -CH=C(CH₃)-CH=CH-CH₃, -CH₂-CH(CH₃)-C≡CH, -C(CH₃)=CH-CH=CH-CH₃, -CH=C(CH₃)-C(CH₃)=CH₂, -C(CH₃)=CH-C(CH₃)=CH₂, -C(CH₃)=C(CH₃)-CH=CH₂, -CH=CH-CH=CH-CH=CH₂, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -C₄H₈-C≡CH, -C₂H₄-C≡C-C₂H₅, -CH₂-C≡C-C₃H₇, -C≡C-C₄H₉, -C≡C-C(CH₃)₃, -CH(CH₃)-C₂H₄-C≡CH, -CH₂-CH(CH₃)-C≡C-CH₃, -CH(CH₃)-CH₂-C≡C-CH₃, -CH(CH₃)-C≡C-C₂H₅, -CH₂-C≡C-CH(CH₃)₂, -C≡C-CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡CH, -CH₂-CH(C≡CH)₂, -C≡C-C≡CH, -CH₂-C≡C-C≡CH, -C≡C-C≡C-CH₃, -CH(C≡CH)₂, -C₂H₄-C≡C-C≡CH, -CH₂-C≡C-CH₂-C≡CH, -C≡C-C₂H₄-C≡CH, -CH₂-C≡C-C≡C-CH₃, -C≡C-CH₂-C≡C-CH₃, -C≡C-C≡C-C₂H₅, -C(C≡CH)₂-CH₃, -C≡C-CH(CH₃)-C≡CH, -CH(CH₃)-C≡C-C≡CH, -CH(C≡CH)-CH₂-C≡CH, -CH(C≡CH)-C≡C-CH₃;
**R^{N}** represents -H, -CH₂-OCH₃, -C₂H₄-OCH₃, -C₃H₆-OCH₃, -CH₂-OC₂H₅, -C₂H₄-OC₂H₅, -C₃H₆-OC₂H₅, -CH₂-OC₃H₇, -C₂H₄-OC₃H₇, -C₃H₆-OC₃H₇, -CH₂-O-cyclo-C₃H₅, -C₂H₄-O-cyclo-C₃H₅, -C₃H₆-O-cyclo-C₃H₅, -CH₂-OCH(CH₃)₂, -C₂H₄-OCH(CH₃)₂, -C₃H₆-OCH(CH₃)₂, -CH₂-OC(CH₃)₃, -C₂H₄-OC(CH₃)₃, -C₃H₆-OC(CH₃)₃, -CH₂-OC₄H₉, -C₂H₄-OC₄H₉, -C₃H₆-OC₄H₉, -CH₂-OPh, -C₂H₄-OPh, -C₃H₆-OPh, -CH₂-OCH₂-Ph, -C₂H₄-OCH₂-Ph, -C₃H₆-OCH₂-Ph, -CHO, -COCH₃, -COC₂H₅, -COC₃H₇, -CO-cyclo-C₃H₅, -COCH(CH₃)₂, -COC(CH₃)₃, -COCN, -COOCH₃, -COOC₂H₅, -COOC₃H₇, -COO-cyclo-C₃H₅, -COOCH(CH₃)₂, -COOC(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -SO₂CH₃, -SO₂C₂H₅, -SO₂C₃H₇, -SO₂-cyclo-C₃H₅, -SO₂CH(CH₃)₂, -SO₂C(CH₃)₃, -CH₂-OCF₃, -C₂H₄-OCF₃, -C₃H₆-OCF₃, -OC₂F₅, -CH₂-OC₂F₅, -C₂H₄-OC₂F₅, -C₃H₆-OC₂F₅, -CH₂F, -CHF₂, -CF₃, -CH₂Cl, -CH₂Br, -CH₂I, -CH₂-CH₂F, -CH₂-CHF₂, -CH₂-CF₃, -CH₂-CH₂Cl, -CH₂-CH₂Br, -CH₂-CH₂I, -cyclo-C₈H₁₅, -Ph, -CH₂-Ph, -CH₂-CH₂-Ph, -CH=CH-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₇, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -C₂H₄-CH(CH₃)-CH=CH₂, -CH₂-CH(CH₃)-CH₂-CH=CH₂, -C₂H₄-CH=C(CH₃)₂, -CH(CH₃)-C₂H₄-CH=CH₂, -C₂H₄-C(CH₃)=CH-CH₃, -CH₂-CH(CH₃)-CH=CH-CH₃, -CH(CH₃)-CH₂-CH=CH-CH₃, -CH₂-CH=CH-CH(CH₃)₂, -CH₂-CH=C(CH₃)-C₂H₅, -CH₂-C(CH₃)=CH-C₂H₅, -CH(CH₃)-CH=CH-C₂H₅, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -C[C(CH₃)₃]=CH₂, -CH(CH₃)-CH₂-C(CH₃)=CH₂, -CH(CH₃)-CH(CH₃)-CH=CH₂, -CH=CH-C₂H₄-CH=CH₂, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH=CH-CH₂-CH=CH-CH₃, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[CH(CH₃)(C₂H₅)]=CH₂, -C[CH₂-CH(CH₃)₂]=CH₂, -C₂H₄-CH=CH-CH=CH₂, -CH₂-CH=CH-CH₂-CH=CH₂, -C₃H₆-C≡C-CH₃, -CH₂-CH=CH-CH=CH-CH₃, -CH=CH-CH=CH-C₂H₅, -CH₂-CH=CH-C(CH₃)=CH₂, -CH₂-CH=C(CH₃)-CH=CH₂, -CH₂-C(CH₃)=CH-CH=CH₂, -CH(CH₃)-CH₂-C≡CH, -CH(CH₃)-CH=CH-CH=CH₂, -CH=CH-CH₂-C(CH₃)=CH₂, -CH(CH₃)-C≡C-CH₃, -CH=CH-CH(CH₃)-CH=CH₂, -CH=C(CH₃)-CH₂-CH=CH₂, -C₂H₄-CH(CH₃)-C≡CH, -C(CH₃)=CH-CH₂-CH=CH₂, -CH=CH-CH=C(CH₃)₂, -CH₂-CH(CH₃)-CH₂-C≡CH, -CH=CH-C(CH₃)=CH-CH₃, -CH=C(CH₃)-CH=CH-CH₃, -CH₂-CH(CH₃)-C≡CH, -C(CH₃)=CH-CH=CH-CH₃, -CH=C(CH₃)-C(CH₃)=CH₂, -C(CH₃)=CH-C(CH₃)=CH₂, -C(CH₃)=C(CH₃)-CH=CH₂, -CH=CH-CH=CH-CH=CH₂, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -C₄H₈-C≡CH, -C₂H₄-C≡C-C₂H₅, -CH₂-C≡C-C₃H₇, -C≡C-C₄H₉, -C≡C-C(CH₃)₃, -CH(CH₃)-C₂H₄-C≡CH, -CH₂-CH(CH₃)-C≡C-CH₃, -CH(CH₃)-CH₂-C≡C-CH₃, -CH(CH₃)-C≡C-C₂H₅, -CH₂-C≡C-CH(CH₃)₂, -C≡C-CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₅)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡CH, -CH₂-CH(C≡CH)₂, -C≡C-C≡CH, -CH₂-C≡C-C≡CH, -C≡C-C≡C-CH₃, -CH(C≡CH)₂, -C₂H₄-C≡C-C≡CH, -CH₂-C≡-C-CH₂-C≡CH, -C≡C-C₂H₄-C≡CH, -CH₂-C≡C-C≡C-CH₃, -C≡C-CH₂-C≡C-CH₃, -C≡C-C≡C-C₂H₅, -C(C≡CH)₂-CH₃, -C≡C-CH(CH₃)-C≡CH, -CH(CH₃)-C≡C-C≡CH, -CH(C≡CH)-CH₂-C≡CH, -CH(C≡CH)-C≡C-CH₃;
**X4, X5 and X6** represent independently of each other:
   a bond, -CH₂-, -C₂H₄-, -C₃H₆-, -C₄H₈-, -C₅H₁₀-, -C₆H₁₂-, -C₇H₁₄-, -C₈H₁₆-, -C₉H₁₈-, -C₁₀H₂₀-, -CH(CH₃)-, -C[(CH₃)₂]-, -CH₂-CH(CH₃)-, -CH(CH₃)-CH₂-, -CH(CH₃)-C₂H₄-, -CH₂-CH(CH₃)-CH₂-, -C₂H₄-CH(CH₃)-, -CH₂-C[(CH₃)₂]-, -C[(CH₃)₂]-CH₂-, -CH(CH₃)-CH(CH₃)-, -C[(C₂H₅)(CH₃)]-, -CH(C₃H₇)-, -CH₂O-, -CH₂CH₂O-, -(CH₂-CH₂-O)ₘ-CH₂-CH₂-, -C(CH₃)=CH-C(CH₃)=CH-, -C₂H₄-CH=CH-CH=CH-, -CH₂-CH=CH-CH₂-CH=CH-, -C₃H₆-C≡C-CH₂-, -CH₂-CH=CH-CH=CH-CH₂-, -CH=CH-CH=CH-C₂H₄-, -CH₂-CH=CH-C(CH₃)=CH-, -CH₂-CH=C(CH₃)-CH=CH-, -CH₂-C(CH₃)=CH-CH=CH-, -CH(CH₃)-CH=CH-CH=CH-, -CH=CH-CH₂-C(CH₃)=CH-, -CH(CH₃)-C≡C-CH₂-, -CONH-, -NHCO-, -CH₂-CONH-, -CONH-CH₂-, -NHCO-CH₂-, -CH₂-NHCO-;
   wherein m is an integer from 1 to 10; or
   **X⁵-R^{C}** and **X⁶-R^{D}** can form together a cyclic ring selected from the group consisting of: 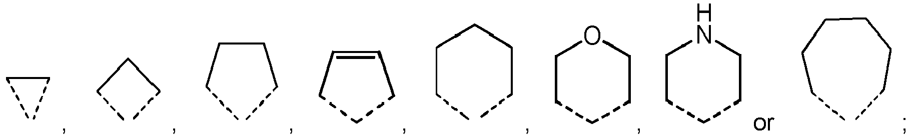 and enantiomers, stereoisomeric forms, mixtures of enantiomers, anomers, deoxy-forms, diastereomers, mixtures of diastereomers, prodrugs, tautomers, hydrates, solvates and racemates of the above mentioned compounds and pharmaceutically acceptable salts thereof.

In fact, it is a finding of this invention that a connection to the linker at R^{E}-position is of central importance for the binding to the FKBP, and FKBP-degradation. Attachment of the linker to other R-positions may result in a loss of binding affinity to the FKBP.

The term "ligand capable of binding" herein means any molecule, which is capable to bind to the target molecule with an affinity of at least 10µM, preferable <1µM, more preferable <100nM and most preferably <10nM.

In one embodiment, (FM) and has the structure of (III): 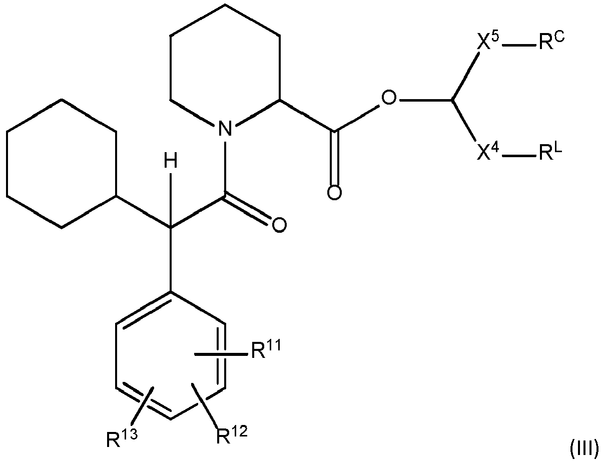 wherein
**X⁴** represents bond, -C₂H₄-, or -CH₂O-;
**X⁵, R^{C}, R^{L}, R¹¹, R¹², R¹³** having the meaning as defined above
and enantiomers, stereoisomeric forms, mixtures of enantiomers, anomers, deoxy-forms, diastereomers, mixtures of diastereomers, prodrugs, tautomers, hydrates, solvates and racemates of the above mentioned compounds and pharmaceutically acceptable salts thereof.

In another embodiment (FM) has the structure of (IVa - c): 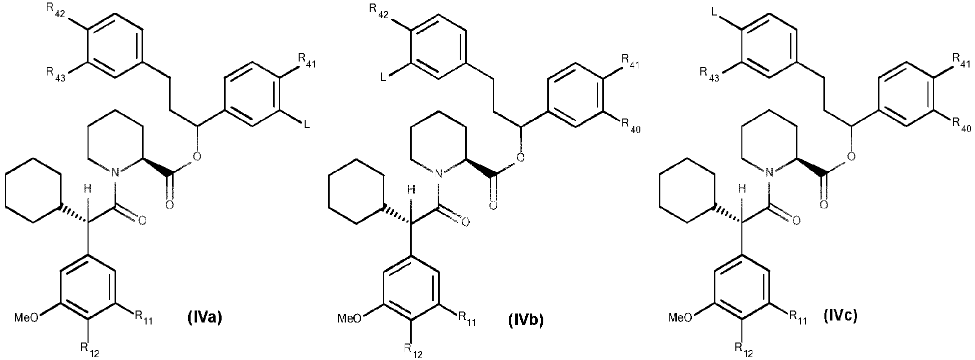
wherein **L** represents the chemical linker;
and **R¹¹, R¹², R⁴⁰, R⁴¹**, **R⁴²**, and **R⁴³** represents the same residues as defined above;
and enantiomers, stereoisomeric forms, mixtures of enantiomers, anomers, deoxy-forms, diastereomers, mixtures of diastereomers, prodrugs, tautomers, hydrates, solvates and racemates of the above mentioned compounds and pharmaceutically acceptable salts thereof.

### Linker

The linker connects the **FM** and the **EM.** It is of central importance for the present invention, since different linker lengths and structures may result in successful or non-successful binding to and/or degradation of the targeted FKBP.

The linker of PROTACs connects the E3 ligase recognition motif and the small molecule ligand for the target protein. The length and configuration of the linker can impact the efficiency of a PROTAC to degrade a target protein. An appropriate linker is usually crucial for inducing an optimal protein-protein interaction between a protein of interest and an E3-E2-ubiquitin complex to promote ubiquitination of target protein resulting in efficient proteasomal degradation.

In a preferred embodiment, the formula of the linker **L** is: 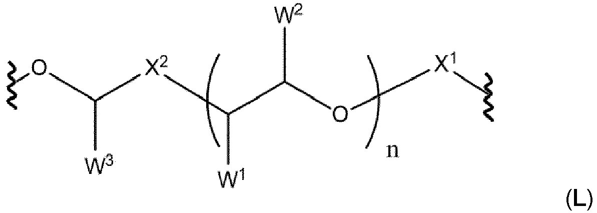
- wherein n is an integer from 0 to 10
- X1 represents a bond, -CH₂-,-(C=O)NH-,-CH2(C=O)NH-, -CH(CH₃)(C=O)NH-, - (C=O)NHCH2-,
- X2 represents 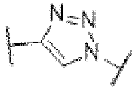 or -(C=O)NH-;
- wherein W¹, W² and W³ independently represents -H and/or -CH₃,
- wherein the "FKBP-binding moiety" (FM) represents a ligand capable of binding to the FK506 binding protein 51 (FKBP51),
- wherein the "E3-binding moiety" (EM) represents ligand capable of binding an E3 ubiquitin ligase,
and wherein, upon binding of the PROTAC to FKBP51 and the E3 ubiquitin ligase, FKBP51 is degraded by a ubiquitin mediated proteolysis pathway within a cell.

In fact, it is a finding of the present invention that the length (n) of the linker should not exceed 10 glycol units, in one embodiment it should be from 2 to 8, in one embodiment 3-5, in one embodiment 4 in order to enable a degradation of the FKBP.

Although PEGs are widely utilized polymers in drug development and nanotechnology due to their biocompatibility, an efficient PEG-chain elongation remains a challenge due to the low yield of the available ether synthesis methods.

The synthesis route and conditions were optimized for liquid phase. Diethylene glycol monoethyl ether, a 2-unit PEG chain with one end capped by ethyl, was employed as the substitute for the intermediate bound to resin.

The small cyclic sulfates, 1,3,2-Dioxathiolane 2,2-dioxide, and the 14-membered cyclic sulfates (with 4 PEG units) were applied as the electrophiles.

After the substitution with diethylene glycol monoethyl ether and the subsequent deprotection in acidic condition, the extended PEG chains were obtained with the yield of 85% and 92%, respectively.

It is therefore another finding of the present invention, that cyclic sulfate is an ideal building block to extend PEG chain into different lengths flexibly with high yield.

### E3-binding moiety (EM)

The ubiquitin-proteasome system (UPS) is one of the major pathways responsible for protein degradation for the maintenance of cellular homeostasis. This system comprises ubiquitin, proteasome, three enzymes and intracellular target proteins, designated substrates, and plays key roles in various important biological processes, such as cell cycle progression, signal transduction, maintenance of genome integrity, and tumorigenesis.

Protein ubiquitination is an ATP-dependent enzymatic reaction mediated by an ubiquitin-activating enzyme (E1), an ubiquitin-conjugating enzyme (E2), and a ubiquitin ligase (E3) (Figure 1).

The process involves three steps: first, an E1 activates an ubiquitin molecule by forming an acyl amide acid mediator at the C-terminus of the ubiquitin in an ATP-dependent reaction.

Second, the activated ubiquitin is then transferred to an E2.

Finally, an E3 ligase, which recognizes and recruits the protein substrate, catalyzes the transfer of the ubiquitin from the E2 to a lysine residue on the substrate via the formation of a covalent bond.

Ubiquitin itself contains seven lysine residues and an N-terminal methionine residue, on which polyubiquitin chains are formed after multiple cycles of this reaction. K48- or K11-linked poly-ubiquitinated proteins are predominantly recognized and destroyed by the proteasome.

By using specific binders to E3 ligases, the inventors were able to degrade specific FKBP, which are otherwise known for their cellular stability, via the UPS.

### General Formulas

The E3 ubiquitin ligase binding moieties according to some embodiments of the present invention are generally represented by the following general formulas (EM-1), (EM-2) and/or (EM-3): 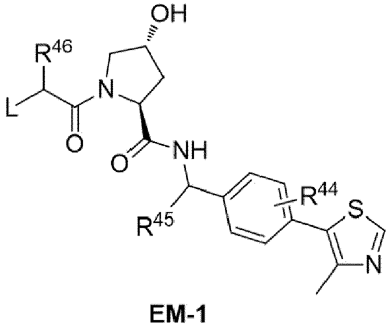 or 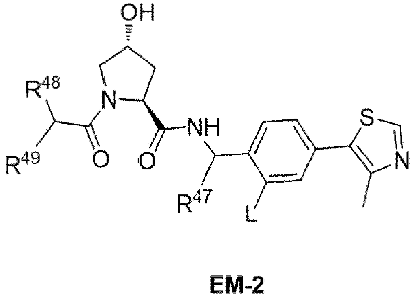 or 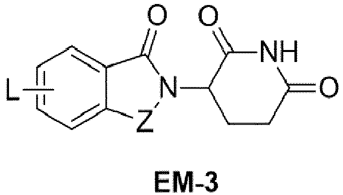 wherein
**R⁴⁴** represents -H, -OH, -SH, -NH2;
**R⁴⁵** and **R⁴⁷** represent independently -H or -CH3;
**R⁴⁶** and **R⁴⁸** represent -H, -CH3, -CH2CH3, -CH(CH3)2, -C(CH3)3, -C(SH)(CH3)2;
**R⁴⁹** represents 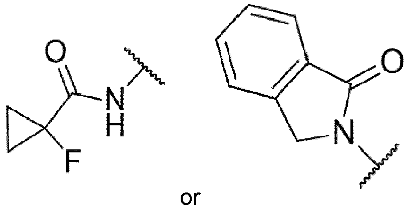
**Z** represents -CH2-, -CO-;
**L** represents the chemical linker L as depicted above;
and enantiomers, stereoisomeric forms, mixtures of enantiomers, anomers, deoxy-forms, diastereomers, mixtures of diastereomers, prodrugs, tautomers, hydrates, solvates and racemates of the above mentioned compounds and pharmaceutically acceptable salts thereof.

### Specific Molecules

In some embodiments, the following E3 ubiquitin ligase binding moieties may be used: 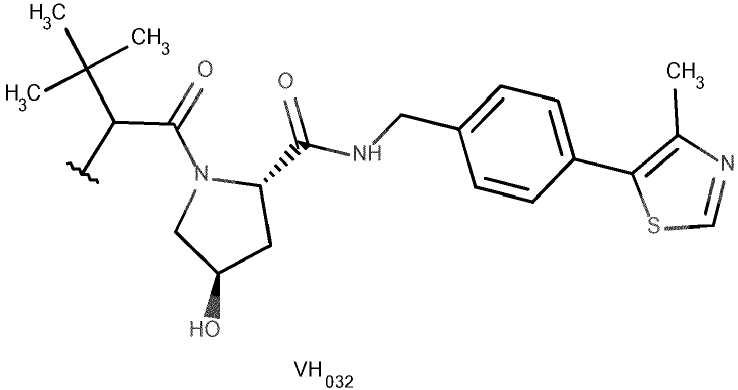 or 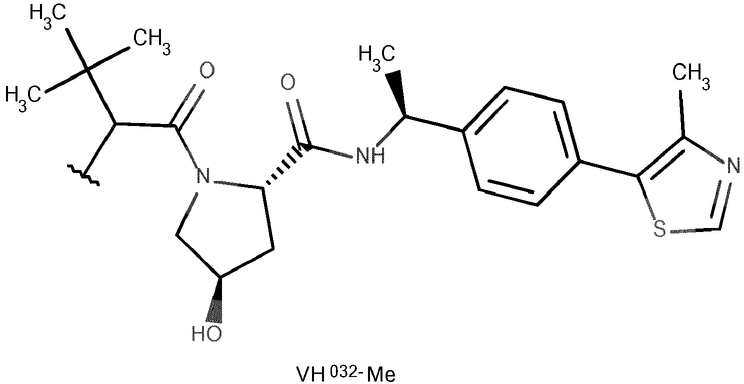 or 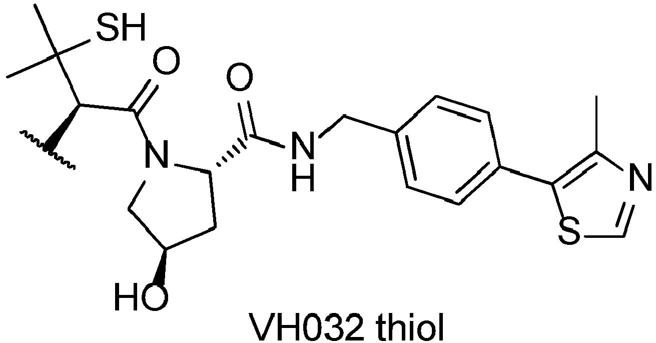 or 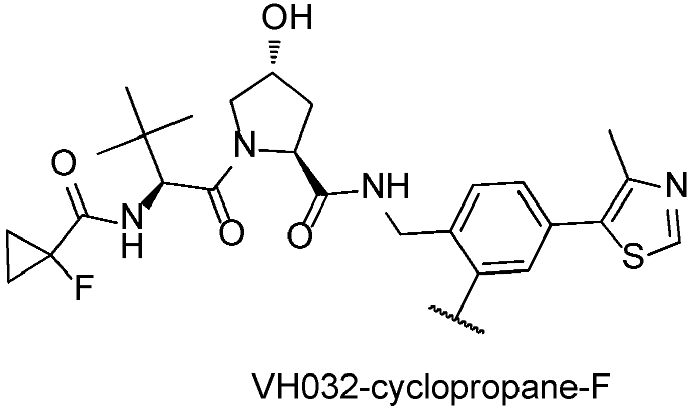 or 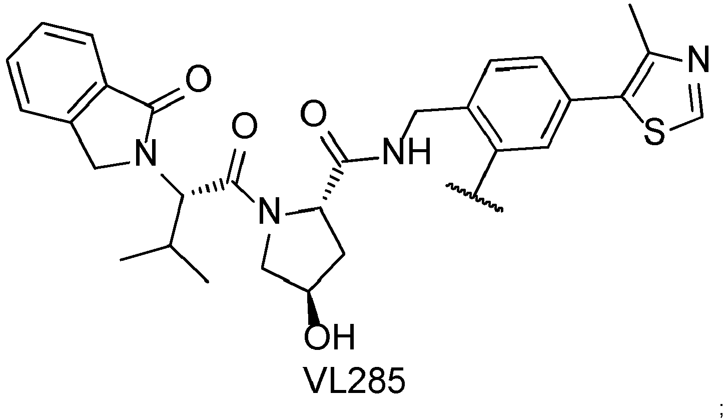 and enantiomers, stereoisomeric forms, mixtures of enantiomers, anomers, deoxy-forms, diastereomers, mixtures of diastereomers, prodrugs, tautomers, hydrates, solvates and racemates of the above mentioned compounds and pharmaceutically acceptable salts thereof.

In further embodiments EM may be selected from a group consisting of a ligand of ubiquitin E3 ligase cereblon-ligand, an ubiquitin E3 ligase VHL-ligand and/or other E3-ligase ligand; .

In even further embodiments EM may be selected from the group consisting of (4R,5S)-nutlin carboxylic acid, (S,R,S)-AHPC hydrochloride, (S,R,S)-AHPC TFA, (S,R,S)-AHPC, (S,R,S)-AHPC-Me dihydrochloride, (S,R,S)-AHPC-Me hydrochlo-ride, (S,R,S)-AHPC-Me, (S,R,S)-AHPC-propargyl, AhR Ligand 1, BC-1215, Bestatin-amido-Me, CC-885, clAP1 ligand 1, clAP1 ligand 2, E3 ligase Ligand 10, E3 ligase Lig-and 13, E3 ligase Ligand 14, E3 ligase Ligand 18, E3 ligase Ligand 8, E3 ligase Ligand 9, VH032-cyclopropane-F, Iberdomide, Lenalidomide (hemihydrate), Lenalidomide hydro-chloride, Lenalidomide, Lenalidomide-Br, MV-1-NH-Me, Nutlin carboxylic acid, NV03, Pomalidomide, TD-106, Thalidomide D4, Thalidomide fluoride, Thalidomide, Thalido-mide-O-COOH, Thalidomide-propargyl, Nutlin, Idasanutlin, Bestatin Esters, VH032, VH032 thiol, VH-298, VHL Ligand 8, VL285, and/or any combination thereof.

### Specific molecules

In one embodiment the compound of the present invention may be selected from one of the molecules represented as: 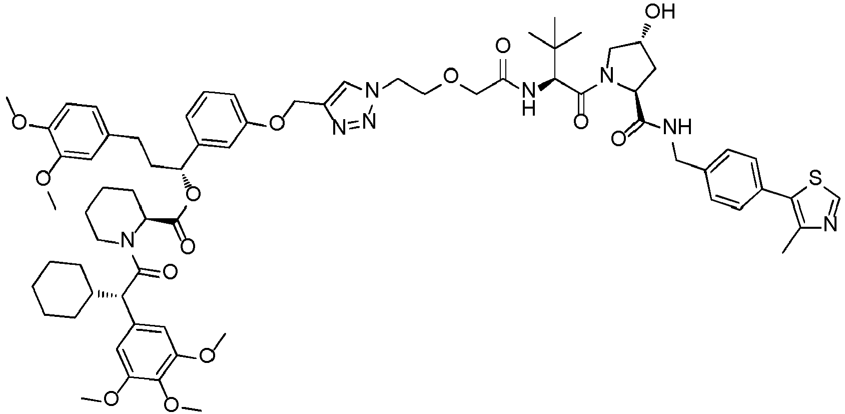 (S)-(R)-3-(3,4-Dimethoxyphenyl)-1-(3-((1-(2-(2-(((S)-1-((2S,4R)-4-hydroxy-2-((4-(4-methylthiazol-5-yl)benzyl)carbamoyl)pyrrolidin-1-yl)-3,3-dimethyl-1-oxobutan-2-yl)amino)-2-oxoethoxy)ethyl)-1H-1,2,3-triazol-4-yl)methoxy)phenyl)propyl 1-((S)-2-cyclohexyl-2-(3,4,5-trimethoxyphenyl)acetyl)piperidine-2-carboxylate, or 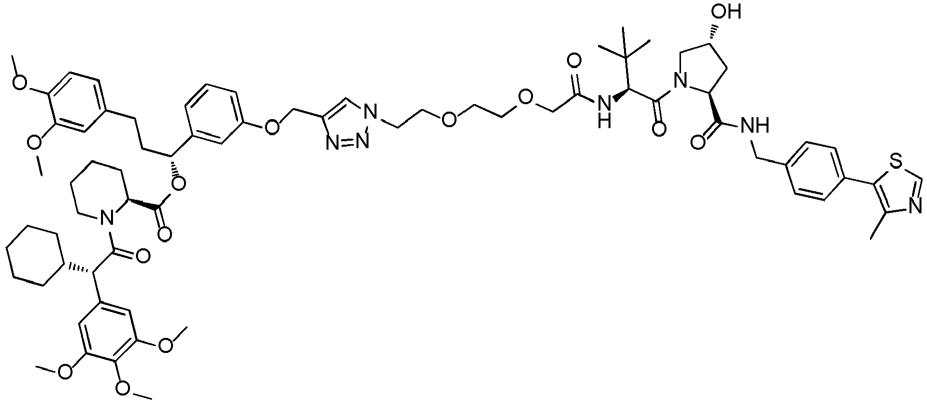 (S)-(R)-3-(3,4-Dimethoxyphenyl)-1-(3-((1-(2-(2-(2-(((S)-1-((2S,4R)-4-hydroxy-2-((4-(4-methylthiazol-5-yl)benzyl)carbamoyl)pyrrolidin-1-yl)-3,3-dimethyl-1-oxobutan-2-yl)amino)-2-oxoethoxy)ethoxy)ethyl)-1 H-1 ,2,3-triazol-4-yl)methoxy)phenyl)propyl 1-((S)-2-cyclohexyl-2-(3,4,5-trimethoxyphenyl)acetyl)piperidine-2-carboxylate,
or 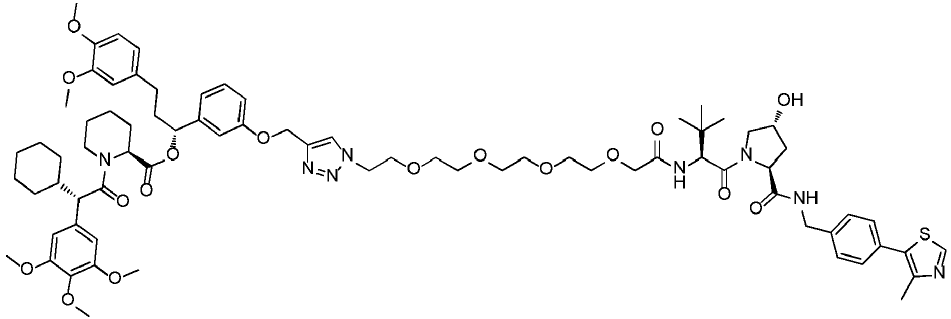 (S)-(R)-3-(3,4-Dimethoxyphenyl)-1-(3-((1-((S)-16-((2S,4R)-4-hydroxy-2-((4-(4-methylthiazol-5-yl)benzyl)carbamoyl)pyrrolidine-1-carbonyl)-17,17-dimethyl-14-oxo-3,6,9,12-tetraoxa-15-azaoctadecyl)-1H-1,2,3-triazol-4-yl)methoxy)phenyl)propyl 1-((S)-2-cyclohexyl-2-(3,4,5-trimethoxyphenyl)acetyl)piperidine-2-carboxylate, (alternative name: MTQ202) and/or any combination thereof;
and enantiomers, stereoisomeric forms, mixtures of enantiomers, anomers, deoxy-forms, diastereomers, mixtures of diastereomers, prodrugs, tautomers, hydrates, solvates and racemates of the above mentioned compounds and pharmaceutically acceptable salts thereof.

### Pharmaceutical Compositions

The present invention also comprises pharmaceutically acceptable salts of the compounds according to the general formula (I) and the subformulas (II)-(IV), all stereoisomeric forms of the compound as well as solvates, especially hydrates, tautomers or prodrugs thereof.

The expression "tautomer" is defined as an organic compound that is interconvertible by a chemical reaction called tautomerization. Tautomerization can be catalyzed preferably by bases or acids or other suitable compounds.

The expression "prodrug" is defined as a pharmacological substance, a drug, which is administered in an inactive or significantly less active form. Once administered, the prodrug is metabolized in the body in vivo into the active compound.

In case, the inventive compounds bear basic and/or acidic substituents, they may form salts with organic or inorganic acids or bases. Examples of suitable acids for such acid addition salt formation are hydrochloric acid, hydrobromic acid, sulfuric acid, phosphoric acid, acetic acid, citric acid, oxalic acid, malonic acid, salicylic acid, p-aminosalicylic acid, malic acid, fumaric acid, succinic acid, ascorbic acid, maleic acid, sulfonic acid, phosphonic acid, perchloric acid, nitric acid, formic acid, propionic acid, gluconic acid, lactic acid, tartaric acid, hydroxymaleic acid, pyruvic acid, phenylacetic acid, benzoic acid, p-aminobenzoic acid, p-hydroxybenzoic acid, methanesulfonic acid, ethanesulfonic acid, nitrous acid, hydroxyethanesulfonic acid, ethylenesulfonic acid, p-toluenesulfonic acid, naphthylsulfonic acid, sulfanilic acid, camphorsulfonic acid, mandelic acid, o-methylmandelic acid, hydrogen-benzenesulfonic acid, picric acid, adipic acid, tolyltartaric acid, tartronic acid, (o, m, p)-toluic acid, naphthylamine sulfonic acid, and other mineral or carboxylic acids well known to those skilled in the art. The salts are prepared by contacting the free base form with a sufficient amount of the desired acid to produce a salt in the conventional manner.

Examples for suitable inorganic or organic bases are, for example, NaOH, KOH, NH₄OH, tetraalkylammonium hydroxide, lysine or arginine and the like. Salts may be prepared in a conventional manner using methods well known in the art, for example by treatment of a solution of the compound of the general formula (I) and the subformulas (II)-(IV), with a solution of an acid, selected out of the group mentioned above.

Some of the compounds of the present invention may be crystallised or re-crystallised from solvents such as aqueous and organic solvents. In such cases solvates may be formed. This invention includes within its scope stoichiometric solvates including hydrates as well as compounds containing variable amounts of water that may be produced by processes such as lyophilisation.

Certain compounds of the general formula (I) and the subformulas (II)-(IV) may exist in the form of optical isomers if substituents with at least one asymmetric center are present, e.g. diastereoisomers and mixtures of isomers in all ratios, e.g. racemic mixtures. The invention includes all such forms, in particular the pure isomeric forms. The different isomeric forms may be separated or resolved one from the other by conventional methods, or any given isomer may be obtained by conventional synthetic methods or by stereospecific or asymmetric syntheses. Where a compound according to the general formula (I) and the subformulas (II)-(IV) contains an alkene moiety, the alkene can be presented as a cis- or trans-isomer or a mixture thereof.

When an isomeric form of a compound of the invention is provided substantially free of other isomers, it will preferably contain less than 5% w/w, more preferably less than 2% w/w and especially less than 1 % w/w of the other isomers.

Another aspect of the present invention relates to the use of the inventive FKBP-degradation PROTACs as drugs, i.e. as pharmaceutically active agents applicable in medicine.

Therefore, one aspect of the present invention is that the compounds according to the general formula (I) and the subformulas (II)-(IV) are suitable for use to eliminate of FKBP-function in an organism. It is preferred if said compound is suitable to eliminate the function of the FK506-binding protein 51 (FKBP51).

FKBP51 has been implicated in numerous in human diseases. Consequently, FKBP51 is a target which is addressed in order to prevent and/or treat the diseases disclosed in the literature.

Thus, FKBP51 degradation PROTACs of the present invention can be used as pharmaceutically active agent in medicine.

Preferred, the FKBP51 degradation PROTACs of the present invention can be used for treatment, or for the preparation of a pharmaceutical formulation for prophylaxis and/or treatment of these FKBP51-associated diseases.

The inventive compound of any one of general formula (I) and the subformulas (II)-(IV) is used in the manufacture of a medicament or of a pharmaceutical composition for the treatment and/or prevention of FKBP51-associated diseases.

Another aspect of the present invention relates to a method of treating FKBP51-associated diseases comprising administration a therapeutically effective amount of at least one inventive compound or a pharmaceutical composition comprising at least one inventive compound.

These FKBP51-associated diseases include psychiatric and neurodegenerative diseases, disorders and conditions, for metabolic diseases such as localized adiposity or obesity, for sleep disorders, neuroprotection or neuroregeneration, for the treatment of neurological disorders, for the treatment of diseases relating to neurodegeneration, for the treatment of cancers such as malignant melanoma or acute lymphoblastic leukaemia and especially steroid-hormone dependent cancers such as prostate cancer, for the treatment of glucocorticoid hyposensitivity syndromes and for peripheral glucocorticoid resistance, for asthma, especially steroid-resistant asthma, and for the treatment of infectious diseases, for the treatment of alopecia and promoting hair growth, for the treatment or prevention of multi-drug resistance, for stimulating neurite growth or neuroregeneration, for neuroprotection, for the use as wound healing agents for treating wounds resulting from injury or surgery; for the use in limiting or preventing hemorrhage or neovascularization for treating macular degeneration, and for treating oxidative damage to eye tissues, for treating a metabolic disorder or for pain relief.

The FKBP51 degradation PROTACs of the present invention are preferably suitable for treatment, or for the preparation of a pharmaceutical formulation for prophylaxis and treatment of psychiatric diseases. It is especially preferred if this psychiatric disease is an affective disorder (ICD-10 classification: F30-F39) or an anxiety disorder.

Among the hundreds of different neurodegenerative disorders, the attention has been given only to a handful, including Alzheimer's disease, Parkinson's Disease, and amyotrophic lateral sclerosis.

Among the glucocorticoid hyposensitivity syndromes, the attention has been given to the group of related diseases enclosing resistant asthma, eosinophilic esophagitis, AIDS, rheumatoid arthritis, hypertension.

Among the metabolic disorders, the attention has been given to obesity and diabetes.

Among the cancers, the attention has been given to malignant melanoma, acute lymphoblastic leukaemia, gliomas, idiopathic myelofibrosis, pancreatic and breast cancers, steroid-hormone dependent cancers or prostate cancer.

Among the pain indications, the attention has been given to chronic pain, neuropathic pain and to fibromyalgia.

Therefore, another aspect of the present invention is directed to pharmaceutical compositions comprising at least one compound of the present invention as active ingredient, together with at least one pharmaceutically acceptable carrier, excipient and/or diluents. The pharmaceutical compositions of the present invention can be prepared in a conventional solid or liquid carrier or diluent and a conventional pharmaceutically-made adjuvant at suitable dosage level in a known way. The preferred preparations are adapted for oral application. These administration forms include, for example, pills, tablets, film tablets, coated tablets, capsules, powders and deposits.

Furthermore, the present invention also includes pharmaceutical preparations for parenteral application, including dermal, intradermal, intragastral, intracutan, intravasal, intravenous, intramuscular, intraperitoneal, intranasal, intravaginal, intrabuccal, percutan, rectal, subcutaneous, sublingual, topical, or transdermal application, which preparations in addition to typical vehicles and/or diluents contain at least one compound according to the present invention and/or a pharmaceutical acceptable salt thereof as active ingredient.

The pharmaceutical compositions according to the present invention containing at least one compound according to the present invention, and/or a pharmaceutical acceptable salt thereof as active ingredient will typically be administered together with suitable carrier materials selected with respect to the intended form of administration, i.e. for oral administration in the form of tablets, capsules (either solid filled, semi-solid filled or liquid filled), powders for constitution, extrudates, deposits, gels, elixirs, dispersable granules, syrups, suspensions, and the like, and consistent with conventional pharmaceutical practices. For example, for oral administration in the form of tablets or capsules, the active drug component may be combined with any oral nontoxic pharmaceutically acceptable carrier, preferably with an inert carrier like lactose, starch, sucrose, cellulose, magnesium stearate, dicalcium phosphate, calcium sulfate, talc, mannitol, ethyl alcohol (liquid filled capsules) and the like. Moreover, suitable binders, lubricants, disintegrating agents and coloring agents may also be incorporated into the tablet or capsule. Powders and tablets may contain about 5 to about 95 weight % of the benzothiophene-1,1-dioxide derived compound and/or the respective pharmaceutically active salt as active ingredient.

Suitable binders include starch, gelatine, natural sugars, corn sweeteners, natural and synthetic gums such as acacia, sodium alginate, carboxymethylcellulose, polyethylene glycol and waxes. Among suitable lubricants there may be mentioned boric acid, sodium benzoate, sodium acetate, sodium chloride, and the like. Suitable disintegrants include starch, methylcellulose, guar gum, and the like. Sweetening and flavoring agents as well as preservatives may also be included, where appropriate. The disintegrants, diluents, lubricants, binders etc. are discussed in more detail below.

Moreover, the pharmaceutical compositions of the present invention may comprise an additional pharmaceutically active compound or drug. The pharmaceutically active compound or drug may belong to the group of glucocorticoids. Thus, an embodiment of the current invention comprises the administration of a compound of the current invention in addition to a co-administration of glucocorticoids.

Moreover, the pharmaceutical compositions of the present invention may be formulated in sustained release form to provide the rate controlled release of any one or more of the components or active ingredients to optimise the therapeutic effect(s), e.g. antihistaminic activity and the like. Suitable dosage forms for sustained release include tablets having layers of varying disintegration rates or controlled release polymeric matrices impregnated with the active components and shaped in tablet form or capsules containing such impregnated or encapsulated porous polymeric matrices.

Liquid form preparations include solutions, suspensions, and emulsions. As an example, there may be mentioned water or water/propylene glycol solutions for parenteral injections or addition of sweeteners and pacifiers for oral solutions, suspensions, and emulsions. Liquid form preparations may also include solutions for intranasal administration. Aerosol preparations suitable for inhalation may include solutions and solids in powder form, which may be present in combination with a pharmaceutically acceptable carrier such as an inert, compressed gas, e.g. nitrogen. For preparing suppositories, a low melting fat or wax, such as a mixture of fatty acid glycerides like cocoa butter is melted first, and the active ingredient is then dispersed homogeneously therein e.g. by stirring. The molten, homogeneous mixture is then poured into conveniently sized moulds, allowed to cool, and thereby solidified.

Also included are solid form preparations, which are intended to be converted shortly before use, to liquid form preparations for either oral or parenteral administration. Such liquid forms include solutions, suspensions, and emulsions.

The compounds according to the present invention may also be delivered transdermally. The transdermal compositions may have the form of a cream, a lotion, an aerosol and/or an emulsion and may be included in a transdermal patch of the matrix or reservoir type as is known in the art for this purpose.

The term capsule as recited herein refers to a specific container or enclosure made e.g. of methyl cellulose, polyvinyl alcohols, or denatured gelatines or starch for holding or containing compositions comprising the active ingredient(s). Capsules with hard shells are typically made of blended of relatively high gel strength gelatines from bones or pork skin. The capsule itself may contain small amounts of dyes, opaquing agents, plasticisers and/or preservatives. Under tablet a compressed or moulded solid dosage form is understood which comprises the active ingredients with suitable diluents. The tablet may be prepared by compression of mixtures or granulations obtained by wet granulation, dry granulation, or by compaction well known to a person of ordinary skill in the art.

Oral gels refer to the active ingredients dispersed or solubilised in a hydrophilic semi-solid matrix. Powders for constitution refers to powder blends containing the active ingredients and suitable diluents which can be suspended e.g. in water or in juice.

Suitable diluents are substances that usually make up the major portion of the composition or dosage form. Suitable diluents include sugars such as lactose, sucrose, mannitol, and sorbitol, starches derived from wheat, corn rice, and potato, and celluloses such as microcrystalline cellulose. The amount of diluent in the composition can range from about 5 to about 95 % by weight of the total composition, preferably from about 25 to about 75 weight %, and more preferably from about 30 to about 60 weight %.

The term disintegrants refers to materials added to the composition to support break apart (disintegrate) and release the pharmaceutically active ingredients of a medicament. Suitable disintegrants include starches, "cold water soluble" modified starches such as sodium carboxymethyl starch, natural and synthetic gums such as locust bean, karaya, guar, tragacanth and agar, cellulose derivatives such as methylcellulose and sodium carboxymethylcellulose, microcrystalline celluloses, and cross-linked microcrystalline celluloses such as sodium croscaramellose, alginates such as alginic acid and sodium alginate, clays such as bentonites, and effervescent mixtures. The amount of disintegrant in the composition may range from about 2 to about 20 weight-% of the composition, more preferably from about 5 to about 10 weight-%.

Binders are substances which bind or "glue" together powder particles and make them cohesive by forming granules, thus serving as the "adhesive" in the formulation. Binders add cohesive strength already available in the diluent or bulking agent. Suitable binders include sugars such as sucrose, starches derived from wheat corn rice and potato, natural gums such as acacia, gelatine and tragacanth, derivatives of seaweed such as alginic acid, sodium alginate and ammonium calcium alginate, cellulose materials such as methylcellulose, sodium carboxymethylcellulose and hydroxypropylmethylcellulose, polyvinylpyrrolidone, and inorganic compounds such as magnesium aluminum silicate. The amount of binder in the composition may range from about 2 to about 20 weight-% of the composition, preferably from about 3 to about 10 weight-%, and more preferably from about 3 to about 6 weight-%.

Lubricants refer to a class of substances, which are added to the dosage form to enable the tablet granules etc. after being compressed to release from the mould or die by reducing friction or wear. Suitable lubricants include metallic stearates such as magnesium stearate, calcium stearate, or potassium stearate, stearic acid, high melting point waxes, and other water soluble lubricants such as sodium chloride, sodium benzoate, sodium acetate, sodium oleate, polyethylene glycols and D-,L-leucine. Lubricants are usually added at the very last step before compression, since they must be present at the surface of the granules. The amount of lubricant in the composition may range from about 0.2 to about 5 weight-% of the composition, preferably from about 0.5 to about 2 weight-%, and more preferably from about 0.3 to about 1.5 weight-% of the composition.

Glidents are materials that prevent caking of the components of the pharmaceutical composition and improve the flow characteristics of granulate so that flow is smooth and uniform. Suitable glidents include silicon dioxide and talc. The amount of glident in the composition may range from about 0.1 to about 5 weight-% of the final composition, preferably from about 0.5 to about 2 weight-%.

Coloring agents are excipients that provide coloration to the composition or the dosage form. Such excipients can include food grade dyes adsorbed onto a suitable adsorbent such as clay or aluminum oxide. The amount of the coloring agent may vary from about 0.1 to about 5 weight-% of the composition, preferably from about 0.1 to about 1 weight-%.

Said pharmaceutical compositions may further comprise at least one FKBP51 PROTACs of the general formula (I) and the subformulas (II)-(IV).

The pharmaceutical compositions may further comprise at least one further active agent. It is preferred if this active agent is selected from the group consisting of anti-depressant and other psychotropic drugs. It is further preferred if the anti-depressant is selected from amitriptyline, amioxide clomipramine, doxepine, duloxetine, imipramine trimipramine, mirtazapine, reboxetine, citaloprame, fluoxetine, moclobemide and sertraline.

### Medical Uses

Another aspect of the invention is to provide compounds and/or pharmaceutically acceptable salts thereof, which can be used as pharmaceutically active agents, especially for the treatment of psychiatric disorders and neurodegenerative diseases, disorders and conditions, for treating vision disorders and/or improving vision; for treating memory impairment and/or enhancing memory performance and for treating alopecia, as well as compositions comprising at least one of those compounds and/or pharmaceutically acceptable salts thereof as pharmaceutically active ingredients.

A further aspect of the invention is to provide methods for preparing said compounds.

The object of the present invention is solved by the teaching of the independent claims. Further advantageous features, aspects and details of the invention are evident from the dependent claims, the description, and the examples of the present application.

### Methods of production

Another aspect of the invention is to provide methods for production of the compounds of the present invention as well as pharmaceutical compositions. The details of synthesis-steps are outlined in the example section.

### DESCRIPTION OF THE FIGURES

Figure 1: The ubiquitin-proteasome system. Ubiquitin is activated by an ubiquitin-activating enzyme (E1) via an ATP-dependent reaction and then transferred to the cysteine of an ubiquitin-conjugating enzyme (E2). A ubiquitin ligase (E3) finally catalyzes the transfer of the ubiquitin from the E2 to a substrate. The poly-ubiquitinated substrate is finally recognized and degraded by the proteasome.
Figure 2: Degradation of FKBP51 with MTQ202 in HeLa cells after 24h incubation. Upper blot: Endogeneous FKBP51. Lower blot: GAPDH.
Figure 3: Degradation of FKBP51 with MTQ202 in HEK293T cells after 48h incubation. Upper blot: Endogeneous FKBP51. Lower blot: GAPDH.
Figure 4A-C: General synthetic approach for the intermediates of the general formula (IV).

### EXAMPLES

### Example 1: General synthetic approach for the intermediates of the general formula (IV)

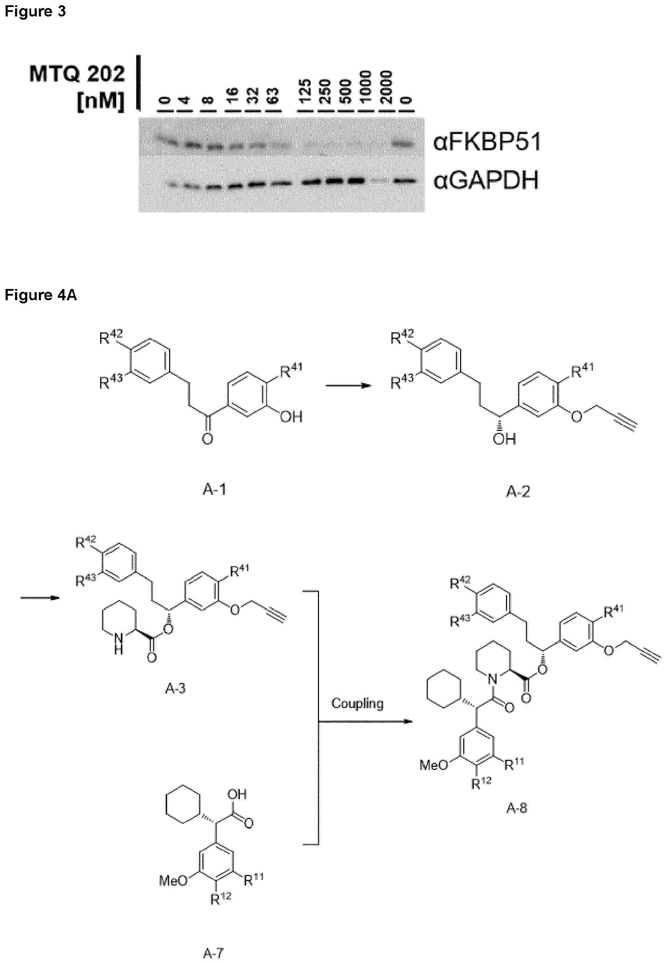

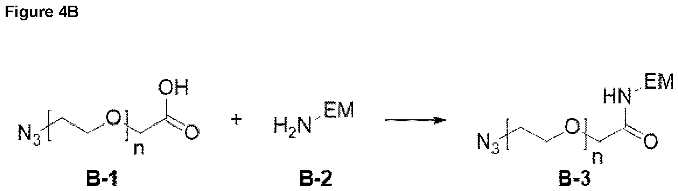

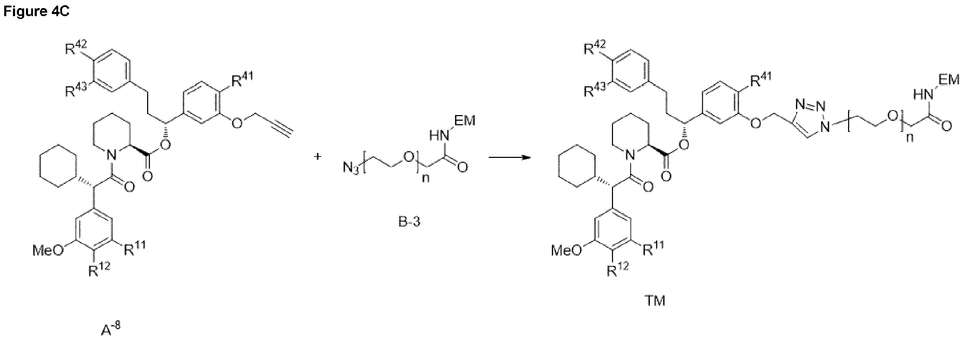

As depicted in Figures 4A-C, the free aromatic alcohol of A-1 was subsequently alkylated with propargyl bromide and reduced via stereoselective hydrogenation to afford A-2. Esterification of the chiral alcohol with commercially available N-Fmoc-L-pipecolinic acid followed by deprotection gave the desired O-propargyl top group A-3. The desired product could be obtained by coupling A-3 and bottom group A-7 with HATU.

The azido linker was coupled to the EM moiety with HATU. The resulting azido-linker-EM B-3 was conjugated with the A-8 by a copper-catalyzed click reaction to give the desired PROTACs TM.

### Example 2: Synthesis of 3-(3,4-Dimethoxyphenyl)-1-(3-(prop-2-yn-1-yloxy)phenyl)propan-1-one [Molecule (1)]

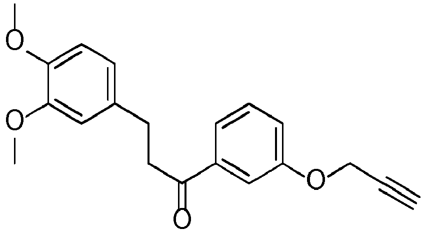

To a solution of 3-(3,4-dimethoxyphenyl)-1-(3-hydroxyphenyl)propan-1-one (3 g, 10.48 mmol, 1 eq.) and K₂CO₃ (4.35 g, 20.96 mmol, 3 eq.) in acetone (50 mL) was added 3-bromoprop-1-yne (80% wt in toluene, 1.356 mL, 13.62 mmol, 1.3 eq.) and stirred at RT for 16h. Then the mixture was filtered and the precipitate was washed with DCM. The solvent was removed under reduced pressure and the crude product was recrystallized from methanol to obtain white crystal (3.07g, 9.4 mmol, 90%).

The following data was obtained when analyzing the molecule in TLC, HPLC and NMR:
TLC [cyclohexane/EtOAc 6/4]: R_{f} = 0.63.
HPLC [0-100% Solvent B, 20 min]: Rₜ = 18.60 min, purity (220 nm) ≥99%.
¹H NMR (300 MHz, Chloroform-d) δ 7.60 - 7.53 (m, 2H), 7.41 - 7.32 (m, 1H), 7.20 - 7.12 (m, 1H), 6.81 - 6.73 (m, 3H), 4.72 (d, J = 2.3 Hz, 2H), 3.86 (s, 3H), 3.84 (s, 3H), 3.26 (t, J = 7.4 Hz, 2H), 3.00 (t, J = 7.6 Hz, 2H), 2.53 (t, J = 2.3 Hz, 1H).
¹³C NMR (75 MHz, Chloroform-d) δ 198.91, 157.76, 148.94, 147.45, 138.31, 133.83, 129.68, 121.45, 120.19, 120.16, 113.68, 111.90, 111.42, 78.09, 75.94, 55.95, 55.85, 40.76, 29.84.

### Example 3: Synthesis of (R)-3-(3,4-Dimethoxyphenyl)-1-(3-(prop-2-yn-1-yloxy)phenyl)propan-1-ol [Molecule (2)]

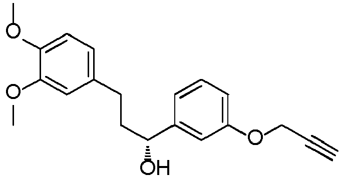

To a stirred solution of molecule (1) (250 mg, 0.77 mmol, 1 eq.) and (S)-(-)-2-methyl-CBS-oxa-zaborolidine solution (1M in toluene, 464 µl, 0.46 mmol, 0.6 eq.) in dry THF (15 mL) borane tetrahydrofuran complex solution (1M in THF, 1.16 mL, 1.16 mmol, 1.5 eq.) was added at
-40°C and the mixture was stirred at the same temperature under Ar atmosphere for 5h. Then 2 mL MeOH was added, and the mixture was warmed to room temperature. Saturated NH₄Cl solution was added and the mixture was extracted with EtOAc. The crude product was concentrated and purified by flash column chromatography (cyclohexane → 1% MeOH in DCM). **molecule (2)** was afforded as a colorless oil (253mg, 0.77 mmol, 100%).

The following data was obtained when analyzing the molecule in TLC, HPLC and NMR:
TLC [cyclohexane/EtOAc 6/4]: R_{f} = 0.49.
HPLC [0-100% Solvent B, 20 min]: Rₜ = 17.10 min, purity (220 nm) = 97%.
¹H NMR (400 MHz, Chloroform-d) δ 7.29 (t, J = 7.9 Hz, 1H), 7.01 - 6.96 (m, 2H), 6.90 (ddd, J = 8.2, 2.5, 0.9 Hz, 1H), 6.81 - 6.77 (m, 1H), 6.75 - 6.70 (m, 2H), 4.70 (d, J = 2.4 Hz, 2H), 4.68 (dd, J = 7.8, 5.4 Hz, 1H), 3.86 (s, 3H), 3.86 (s, 3H), 2.75 - 2.57 (m, 2H), 2.51 (t, J = 2.4 Hz, 1H), 2.15 - 1.96 (m, 2H).
¹³C NMR (101 MHz, Chloroform-d) δ 157.90, 149.01, 147.37, 146.57, 134.46, 129.71, 120.34, 119.28, 114.04, 112.72, 111.93, 111.43, 78.68, 75.68, 73.87, 56.08, 55.98, 55.95, 40.70, 31.74.

### Example 4: Synthesis of (S)-1-((9H-Fluoren-9-yl)methyl) 2-((R)-3-(3,4-dimethoxyphenyl)-1-(3-(prop-2-yn-1-yloxy)phenyl)propyl) piperidine-1,2-dicarboxylate [Molecule (3)]

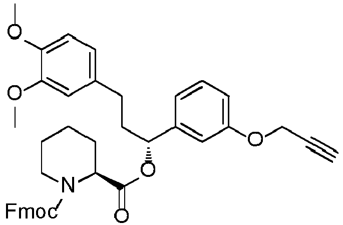

**Molecule (2)** (240 mg, 0.735 mmol, 1.0 eq.) and (S)-N-Fmoc-piperidine-2-carboxylic acid (284 mg, 0.809 mmol, 1.1 eq.) were dissolved in DCM (3.5 mL) followed by the addition of EDC (155 mg, 0.809 mmol, 1.1 eq.) and DMAP (18 mg, 0.147 mmol, 0.2 eq.) at RT. After stirring for 16h, the mixture was concentrated under reduced pressure and purified by flash column chromatography (cyclohexane:EtOAc, gradient EtOAc 0 to 15%) to give a yellow oil (419mg, 0.63 mmol, 86%)

The following data was obtained when analyzing the molecule in TLC, HPLC, MS and NMR:
TLC [cyclohexane/EtOAc 6/4]: R_{f} = 0.51.
HPLC [50-100% Solvent B, 20 min]: Rₜ = 20.91 min, purity (220 nm) ≥ 99%.
¹H NMR (500 MHz, Chloroform-*d*) δ 7.80 - 7.69 (m, 2H), 7.60 (t, J = 8.4 Hz, 1H), 7.51 - 7.17 (m, 6H), 6.98 - 6.84 (m, 3H), 6.74 (d, J = 8.1 Hz, 1H), 6.67 - 6.57 (m, 2H), 5.82 - 5.76 (m, 1H), 5.08 - 4.84 (m, 1H), 4.70 - 4.57 (m, 2H), 4.49 - 4.07 (m, 4H), 3.86 - 3.78 (m, 6H), 3.20 - 2.95 (m, 1H), 2.62 - 2.42 (m, 3H), 2.37 - 2.29 (m, 1H), 2.26 - 2.15 (m, 1H), 2.08 - 2.04 (m, 1H), 1.77 - 1.66 (m, 3H), 1.53 - 1.40 (m, 1H), 1.34 - 1.27 (m, 1H).
¹³C NMR (126 MHz, Chloroform-*d*) δ 171.01, 157.82, 156.47, 156.03, 149.01, 147.46, 144.22, 144.13, 143.98, 143.93, 141.85, 141.60, 141.39, 133.62, 133.50, 129.73, 127.76, 127.14, 125.16, 125.03, 120.22, 120.04, 119.89, 119.77, 114.43, 113.59, 113.37, 111.88, 111.81, 111.50, 78.57, 76.53, 76.26, 75.78, 75.71, 67.87, 60.45, 56.03, 55.92, 55.02, 54.65, 47.35, 42.12, 42.00, 38.16, 31.23, 27.11, 26.92, 24.89, 24.66, 21.11, 20.91, 20.80, 14.30.
MS (ESI+): m/z: found 682.09 [M+Na]⁺, calculated 682.28 [M+Na]⁺

### Example 5: Synthesis of (S)-(R)-3-(3,4-Dimethoxyphenyl)-1-(3-(prop-2-yn-1-yloxy)phenyl)propyl piperidine-2-carboxylate [Molecule (4)]

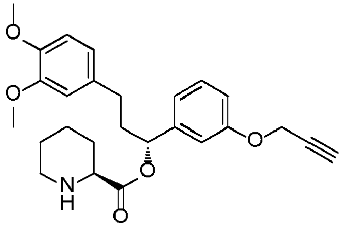

**Molecule (3)** was dissolved in 3mL 4-methylpiperidine/DCM (v/v= 1:9). The solution was stirred at RT overnight.

Then the solution was diluted with 100 ml DCM, and washed with saturated NH₄Cl solution. The organic layer was dried over magnesium sulfate, filtered and concentrated under reduced pressure. Purification by flash column chromatography (cyclohexane → cyclohexane: EtOAc =2:8 + 1% TEA) gave a yellow oil (198mg, 0.45 mmol, 73%).

The following data was obtained when analyzing the molecule in TLC, HPLC. MS and NMR:
TLC [EtOAc + 1% TEA]: R_{f} = 0.23.
HPLC [0-100% Solvent B, 20 min]: Rₜ = 14.37 min, purity (220 nm) = 96%.
¹H NMR (500 MHz, Chloroform-*d*) δ 7.19 (t, J = 8.0 Hz, 1H), 6.90 - 6.85 (m, 2H), 6.85 - 6.80 (m, 1H), 6.70 (d, J = 8.1 Hz, 1H), 6.62 - 6.56 (m, 2H), 5.70 (dd, J = 7.8, 5.7 Hz, 1H), 4.61 (d, J = 2.4 Hz, 2H), 3.78 (s, 3H), 3.77 (s, 3H), 3.29 (dd, J = 9.9, 3.2 Hz, 1H), 2.99 (dt, J = 11.0, 3.4 Hz, 1H), 2.61 - 2.45 (m, 3H), 2.44 (t, J = 2.4 Hz, 1H), 2.22 - 2.12 (m, 1H), 2.04 - 1.93 (m, 2H), 1.77 - 1.69 (m, 1H), 1.58 - 1.46 (m, 2H), 1.45 - 1.32 (m, 2H).
¹³C NMR (126 MHz, Chloroform-*d*) δ 172.92, 157.79, 149.04, 147.49, 142.06, 133.72, 129.67, 120.27, 119.86, 114.19, 113.47, 111.86, 111.50, 78.60, 75.71, 75.51, 58.87, 56.04, 55.98, 55.95, 45.78, 38.05, 31.41, 29.39, 25.96, 24.26.
MS (ESI+): m/z: found 438.02 [M+H]⁺, calculated 438.23 [M+H]⁺

### Example 6: Synthesis of (R)-5-(3-hydroxy-3-(3-methoxyphenyl)propyl)-2-methoxyphenol [Molecule (5)]

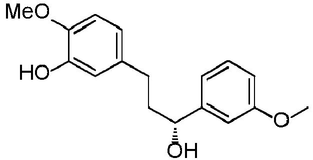

Ketone 3-(3-hydroxy-4-methoxyphenyl)-1-(3-methoxyphenyl)propan-1-one (3.32 g, 11.62 mmol) was suspended in 100 mL isopropanol and the solution was degassed with argon in an autoclave. K₂CO₃ (1.61 g, 11.62 mmol) and the catalyst RuCl₂[(*S*)-(DM-SEGPHOS)][(*S*)-DAIPEN] (0.11 g, 0.09 mmol) were added. The autoclave was closed and flushed with H₂ for three times. A pressure of 10 bar H₂ was applied and the mixture was stirred for 2 d. The solution was filtered and the filtrate was concentrated in vacuum. The crude product was purified by chromatography (cyclohexane: EtOAc, gradient EtOAc 20 to 40%) to give 3.30 g (11.44 mmol, 98.8%) **molecule (5).**

The following data was obtained when analyzing the molecule in TLC, HPLC. MS and NMR:
TLC [cyclohexane/EtOAc 6/4]: R_{f} = 0.43.
HPLC [0%-100% eluent B, 20 min]: Rt = 10.8 min, 99% purity
Enantiomeric excess (ee) = 99%
HR-MS: (ESI⁺), m/z: calculated 311.13 [C₁₇H₂₀O₄ + Na]⁺, found 311.13 [M + Na]⁺
¹H-NMR (300 MHz, Chloroform-*d*): 7.27 (t, *J* = 8.1 Hz, 1H), 6.93 (dd, *J* = 4.4, 1.8 Hz, 2H), 6.83 (ddd, *J* = 8.2, 2.5, 1.0 Hz, 1H), 6.80 - 6.75 (m, 2H), 6.68 (dd, *J* = 8.2, 2.0 Hz, 1H), 4.66 (dd, *J* = 7.7, 5.4 Hz, 1H), 3.87 (s, 3H), 3.82 (s, 3H), 2.63 (td, *J* = 9.0, 6.6 Hz, 2H), 2.16 - 1.95 (m, 2H) ppm.
¹³C-NMR (75 MHz, Chloroform-*d*): 159.77, 146.38, 145.51, 144.81, 135.11, 129.49, 119.73, 118.26, 114.75, 113.08, 111.37, 110.73, 73.71, 56.02, 55.23, 40.48, 31.38 ppm.

### Example 7: Synthesis of (R)-3-(4-methoxy-3-(prop-2-yn-1-yloxy)phenyl)-1-(3-methoxyphenyl)propan-1-ol [Molecule (6)]

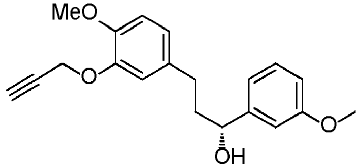

To a solution of 5 (9.98 g, 34.6 mmol) and K₂CO₃ (47.84 g, 346.1 mmol) in 150 mL acetone, 3-Bromprop-1-yne (3.71 mL, 4.94 g, 41.5 mmol) was added and stirred at RT for 4 d. The solution was filtered and the filter cake wash washed with acetone. The combined solution was concentrated in vacuum and purified by chromatography (cyclohexane: EtOAc, gradient EtOAc 30 to 40%) to give 10.5 g (32.0 mmol, 93%) **molecule (6).**

The following data was obtained when analyzing the molecule in TLC, HPLC. MS and NMR:
TLC [cyclohexane/EtOAc 8/2]: R_{f} = 0.13.
HPLC [0%-100% eluent B, 20 min]: Rt = 13.4 min, ≥99% purity
Mass: (ESI⁺), m/z: calculated 309.15 [C₂₀H₂₂O₄⁺H-H₂O]⁺, found 309.13 [M + H - H₂O]⁺
¹H-NMR (300 MHz, Chloroform-*d*): 7.25 (t, *J* = 8.1 Hz, 1H), 6.91 (dd, *J* = 4.6, 2.4 Hz, 2H), 6.88 (s, 1H), 6.85 - 6.77 (m, 3H), 4.73 (d, *J* = 2.4 Hz, 2H), 4.64 (dd, *J* = 7.8, 5.3 Hz, 1H), 3.84 (s, 3H), 3.80 (s, 3H), 2.66 (td, *J* = 8.8, 6.6 Hz, 2H), 2.50 (t, *J =* 2.4 Hz, 1H), 2.13 - 1.94 (m, 3H) ppm.
¹³C-NMR (75 MHz, Chloroform-*d*): 159.78, 147.95, 146.60, 146.41, 134.27, 129.50, 121.83, 118.23, 115.13, 112.97, 111.92, 111.48, 78.79, 75.71, 73.61, 56.81, 55.98, 55.23, 40.47, 31.47 ppm.

### Example 8: Synthesis of (S)-1-((9H-fluoren-9-yl)methyl) 2-((R)-3-(4-methoxy-3-(prop-2-yn-1-yloxy)phenyl)-1-(3-methoxyphenyl)propyl) piperidine-1,2-dicarboxylate [Molecule (7)]

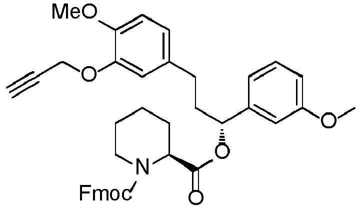

EDC-HCl (0.71 g, 3.68 mmol), DMAP (0.11 g, 0.92 mmol) and (S)-N-Fmoc-piperidine-2-carboxylic acid (1.08 g, 3.06 mmol) were dissolved in 30 mL DCM at 0°C. Alcohol 6 (1.00 g, 3.06 mmol) was added and the mixture was stirred at 0°C for 15 min and at RT overnight. The solution was concentrated in vacuum and purified by chromatography (cyclohexane: EtOAc, gradient EtOAc 0 to 20%) to afford **molecule (7)** (1.92 g, 2.91 mmol, 95%) as a yellow oil.

The following data was obtained when analyzing the molecule in TLC, HPLC. MS and NMR:
TLC [cyclohexane/EtOAc 8/2]: R_{f} = 0.23.
HPLC [50%-100% eluent B, 20 min]: Rt = 15.6 min, ≥99% purity
Mass (ESI⁺), m/z: calculated 682.28 [C₄₁H₄₁NO₇ + Na]⁺, found 682.29 [M+ Na]⁺
¹H-NMR (300 MHz, Chloroform-*d*): 7.75 (dd, *J* = 13.6, 7.5 Hz, 2H), 7.60 (t, *J* = 6.1 Hz, 1H), 7.53 - 7.16 (m, 6H), 6.91 (d, *J* = 7.7 Hz, 1H), 6.87 (d, *J* = 2.2 Hz, 1H), 6.85 - 6.62 (m, 4H), 5.76 (s, 1H), 5.08 - 4.85 (m, 1H), 4.73 - 4.67 (m, 2H), 4.51 - 4.04 (m, 4H), 3.83 (s, 3H), 3.75 (d, *J* = 18.1 Hz, 3H), 3.08 (dt, *J* = 43.4, 12.5 Hz, 1H), 2.61 - 2.40 (m, 3H), 2.38 - 2.14 (m, 2H), 2.05 (t, *J* = 7.1 Hz, 1H), 1.70 (dt, *J* = 9.7, 5.2 Hz, 3H), 1.55 - 1.15 (m, 6H) ppm.
¹³C-NMR (75 MHz, Chloroform-*d*): 170.92, 159.71, 148.09, 146.64, 144.12, 143.91, 141.67, 141.29, 133.40, 129.62, 127.66, 127.05, 125.08, 121.73, 119.95, 118.86, 118.71, 115.02, 113.35, 112.41, 112.31, 111.93, 78.71, 75.73, 67.79, 56.81, 55.96, 55.20, 54.92, 54.55, 47.25, 42.02, 41.92, 38.01, 31.00, 27.04, 26.93, 26.82, 24.82, 24.58, 20.82, 20.72 ppm.

### Example 9: Synthesis of (S)-(R)-3-(4-methoxy-3-(prop-2-yn-1-yloxy)phenyl)-1-(3-methoxyphenyl)propyl piperidine-2-carboxylate [Molecule (8)]

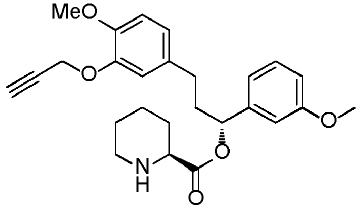

**Molecule (7)** (1.82 g, 2.76 mmol) was dissolved in 13.5 mL 4-methylpiperidine/DCM (v/v=1:9) and the mixture was stirred at RT for 2 h. Then the solution was diluted with DCM, washed with sat. NH₄Cl, dried over MgSO₄ and concentrated in vacuum. The crude product was purified by chromatography (cyclohexane: EtOAc with 1% TEA, gradient EtOAc 40 to 60%) to afford **molecule (8)** (1.07 g, 2.45 mmol, 89%).

The following data was obtained when analyzing the molecule in TLC, HPLC, MS and NMR:
TLC [cyclohexane/EtOAc 6/4]: R_{f} = 0.13.
HPLC [0%-100% eluent B, 20 min]: Rₜ = 12.0 min, 98% purity
¹H-NMR (300 MHz, Chloroform-*d*): 7.28 - 7.19 (m, 1H), 6.90 (d, *J* = 7.7 Hz, 1H), 6.87 - 6.78 (m, 4H), 6.74 (dd, *J* = 8.2, 1.9 Hz, 1H), 5.75 (dd, *J* = 7.9, 5.7 Hz, 1H), 4.74 (d, *J* = 2.4 Hz, 2H), 3.83 (s, 3H), 3.79 (s, 3H), 3.37 (dd, *J* = 9.7, 3.2 Hz, 1H), 3.06 (dt, *J* = 12.0, 3.4 Hz, 1H), 2.69 - 2.53 (m, 3H), 2.51 (t, *J* = 2.4 Hz, 1H), 2.32 - 2.20 (m, 2H), 2.12 - 1.99 (m, 3H), 1.83 - 1.75 (m, 1H), 1.63 - 1.40 (m, 4H) ppm.
¹³C-NMR (75 MHz, Chloroform-*d*): 172.73, 159.68, 148.13, 146.66, 141.82, 133.51, 129.54, 121.78, 118.79, 115.11, 113.16, 112.40, 111.95, 78.72, 75.76, 75.48, 58.68, 56.88, 55.97, 55.23, 45.59, 37.86, 31.18, 29.21, 25.78, 24.10 ppm.

### Example 10: General Method A

To a solution of carboxylic acid (1 eq.) in DCM/DMF (2/1), HATU (1.1 eq.), DIPEA (3 eq.) and amine (1 eq.) were added. After stirring for 16 h, the mixture was poured into brine and extracted 3 times with Et₂O. The combined organic layer was washed 3 times with brine, dried over magnesium sulfate, filtered and condensed. The crude product was purified by flash chromatography.

### Example 11: Synthesis of (S)-(R)-3-(3,4-Dimethoxyphenyl)-1-(3-(prop-2-yn-1-yloxy)phenyl)propyl 1-((S)-2-cyclohexyl-2-(3,4,5-trimethoxyphenyl)acetyl)piperidine-2-carboxylate [Molecule (9)]

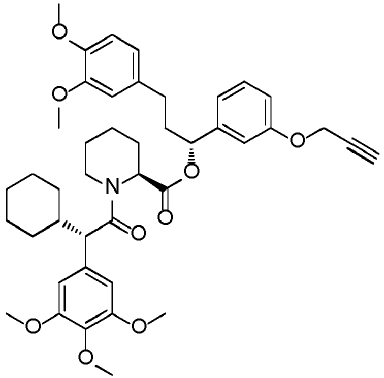

**Molecule** (9) was synthesized from **molecule (4)** (180 mg, 0. 41 mmol) and (S)-2-cyclohexyl-2-(3,4,5-trimethoxyphenyl)acetic acid according to the General Method A. Purification by flash column chromatography (cyclohexane: EtOAc, gradient EtOAc 0 to 20%) gave a white solid (200mg, 0.275 mmol, 67%).

The following data was obtained when analyzing the molecule in TLC, HPLC. HRMS and NMR:
TLC [cyclohexane/EtOAc 6/4]: R_{f} = 0.45.
HPLC [0%-100% eluent B, 20 min]: Rt = 18.5 min, ≥99% purity
¹H NMR (500 MHz, DMSO-*d*₆, 353K) δ 7.27 (m, 1H), 7.06 - 6.58 (m, 8H), 5.58 (dd, J = 7.9, 5.6 Hz, 1H), 5.30 (dd, J = 6.0, 2.7 Hz, 1H), 4.84 - 4.72 (m, 2H), 4.16 (d, J = 13.8 Hz, 1H), 3.84 - 3.59 (m, 16H), 3.46 - 3.35 (m, 1H), 2.89 - 2.75 (m, 1H), 2.62 (m, 1H), 2.50 - 2.33 (m, 2H), 2.29 - 2.12 (m, 1H), 2.12 - 1.76 (m, 3H), 1.73 - 1.37 (m, 6H), 1.34 - 0.66 (m, 8H).
¹³C NMR (126 MHz, DMSO-*d*₆, 353K) δ 172.04, 170.21, 157.33, 152.67, 149.15, 147.54, 141.90, 137.02, 133.63, 133.59, 129.30, 120.29, 118.87, 114.22, 113.19, 113.07, 112.98, 106.84, 79.18, 77.71, 74.92, 59.85, 56.35-55.50, 52.97, 51.84, 42.99, 40.96, 37.32, 31.69, 30.35, 29.82, 26.33-25.02, 20.43.
HRMS: m/z: found 728.37910 [M+H]⁺, calculated 728.37931 [M+H]⁺

### Example 12: Synthesis of (S)-(R)-3-(4-methoxy-3-(prop-2-yn-1-yloxy)phenyl)-1-(3-methoxyphenyl)propyl 1-((S)-2-cyclohexyl-2-(3,4,5-trimethoxyphenyl)acetyl)piperidine-2-carboxylate [molecule (10)]

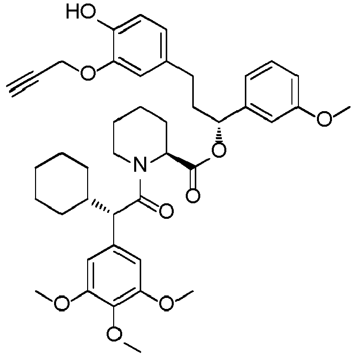

**Molecule (10)** was synthesized from **8** (500 mg, 1.15 mmol) (S)-2-cyclohexyl-2-(3,4,5-trimethoxyphenyl)acetic acid according to the General Method A. Purification by flash column chromatography (cyclohexane:EtOAc, gradient EtOAc 0 to 40%) gave a white solid (546 mg, 0.75 mmol, 65%).

The following data was obtained when analyzing the molecule in TLC, HPLC, HRMS and NMR:
TLC [cyclohexane/EtOAc 6/4]: R_{f} = 0.60.
HPLC [50%-100% eluent B, 20 min]: Rₜ = 12.4 min, 98% purity
Mass (ESI⁺): m/z: calculated 728.38 [C₄₃H₅₃NO₉ + H]⁺, found 728.23 [M+ H]⁺
¹H NMR (500 MHz, Chloroform-*d*) (3:1 mixture of rotamers) δ 7.29 (t, *J* = 7.9 Hz, 0.25H), 7.10 (t, *J* = 7.9 Hz, 0.75H), 6.95 - 6.71 (m, 4H), 6.69 (dd, *J* = 8.2, 1.9 Hz, 0.75H), 6.63 - 6.60 (m, 0.75H), 6.49 (s, 1.5H), 6.42 (d, *J* = 5.6 Hz, 1H), 5.83 - 5.76 (m, 0.25H), 5.55 (dd, *J* = 7.9, 5.9 Hz, 0.75H), 5.47 (d, *J* = 5.0 Hz, 0.75H), 4.74 - 4.72 (m, 2H), 4.70 (d, *J* = 2.3 Hz, 0.25H), 4.55 (d, *J* = 13.7 Hz, 0.25H), 3.95 (d, *J* = 14.0 Hz, 0.75H), 3.86 - 3.77 (m, 7H), 3.75 (d, *J* = 7.5 Hz, 4H), 3.71 (s, 4H), 3.37 (d, *J* = 9.9 Hz, 0.75H), 2.96 (d, *J* = 9.7 Hz, 0.25H), 2.78 (td, *J* = 13.4, 2.7 Hz, 1H), 2.63 - 2.34 (m, 3H), 2.28 (d, *J* = 9.8 Hz, 1H), 2.13 - 2.07 (m, 1H), 2.00 - 1.76 (m, 3H), 1.72 - 1.51 (m, 6H), 1.43 - 1.26 (m, 3H), 1.22 - 1.03 (m, 3H), 1.00 - 0.64 (m, 2H).
¹³C NMR (126 MHz, CDCl₃) (major) δ 172.36, 170.60, 159.56, 153.16, 148.19, 146.74, 141.88, 137.02, 133.72, 133.47, 129.58, 122.00, 118.51, 115.26, 113.13, 112.51, 112.04, 105.92, 78.87, 75.89, 75.68, 60.84, 56.95, 56.15, 56.08, 55.24, 55.10, 52.10, 43.75, 41.49, 37.98, 32.89, 30.94, 30.77, 26.83, 26.70, 26.31, 26.27, 25.69, 21.07.
HRMS: m/z: found 728.37899 [M+H]⁺, calculated 728.37931 [M+H]⁺

### Example 13: The synthesis of exemplary FM-L-EM - General Method B

To a solution of **L-EM** (1 eq) and **FM** (1 eq) in t-BuOH-H₂O (1:1,1 mL) at RT were added copper(II) sulfate pentahydrate (1M in H₂O, 5.5 µl, 5.5 µmol) and (+)-Sodium L-ascorbate (1M in H₂O, 5.5 µl, 5.5 µmol). The reaction mixture was stirred at RT overnight. Then the mixture was diluted by DCM (50mL) and washed with brine (25 mL x 2). The organic phase was dried over magnesium sulfate, filtered and concentrated under reduced pressure. The crude product was purified by flash column chromatography to giva **FM-L-EM.**

### Example 14: Synthesis of (S)-(R)-3-(3,4-Dimethoxyphenyl)-1-(3-((1-(2-(2-(((S)-1-((2S,4R)-4-hydroxy-2-((4-(4-methylthiazol-5-yl)benzyl)carbamoyl)pyrrolidin-1-yl)-3,3-dimethyl-1-oxo-butan-2-yl)amino)-2-oxoethoxy)ethyl)-1H-1,2,3-triazol-4-yl)methoxy)phenyl)propyl 1-((S)-2-cyclohexyl-2-(3,4,5-trimethoxyphenyl)acetyl)piperidine-2-carboxylate (MTQ199)

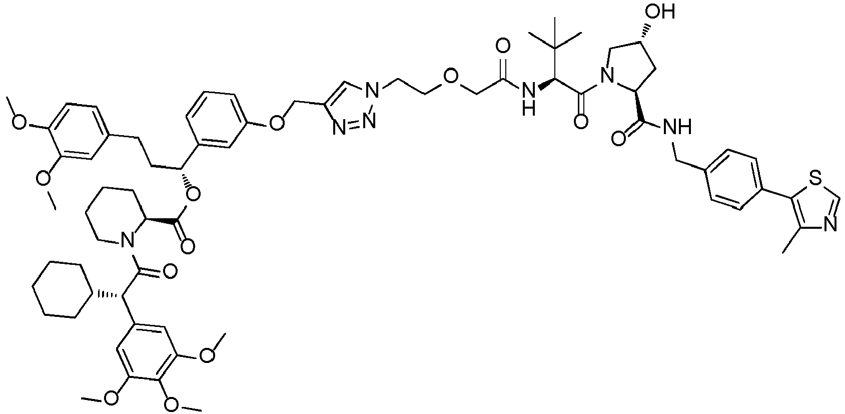

**MTQ199** was synthesized from **molecule (9)** (7 mg, 0. 0137 mmol) according to the General Method B. Purification by flash column chromatography (0% to 5% MeOH in DCM) gave a white solid (13 mg, 0.010 mmol, 74%).

The following data was obtained when analyzing the molecule in TLC, HPLC, HRMS and NMR:
TLC [DCM:MeOH 95:5]: Rf= 0.25.
HPLC [0-100% Solvent B, 20 min]: Rₜ = 17.4 min, purity (220 nm) = 99%.
¹H NMR (300 MHz, Chloroform-*d*) δ 8.69 (s, 1H), 7.79 (s, 0.75H), 7.74 (s, 0.25H), 7.42 - 7.26 (m, 5H), 7.20 - 7.04 (m, 2H), 7.02 - 6.73 (m, 4H), 6.69 - 6.58 (m, 2H), 6.50 - 6.46 (m, 2H), 5.58 (dd, *J* = 7.8, 5.7 Hz, 1H), 5.44 (d, *J* = 3.5 Hz, 1H), 5.17 (s, 0.5H), 5.14 (s, 1.5H), 4.79 - 4.20 (m, 8H), 4.06 - 3.86 (m, 6H), 3.85 - 3.80 (m, 8H), 3.77 (s, 2H), 3.71 (s, 4H), 3.64 - 3.57 (m, 1H), 3.38 (d, *J* = 9.9 Hz, 0.75H), 2.97 (d, *J* = 9.6 Hz, 0.25H), 2.90 - 2.70 (m, 1H), 2.64 - 2.53 (m, 1H), 2.51 (s, 3H), 2.49 - 2.22 (m, 4H), 2.13 - 1.95 (m, 4H), 1.88 - 1.79 (m, 4H), 1.74 - 1.68 (m, 2H), 1.62 - 1.50 (m, 3H), 1.37 - 1.28 (m, 2H), 1.22 - 1.06 (m, 3H), 0.93 (s, 9H).

### Example 15: Synthesis of (S)-(R)-3-(3,4-Dimethoxyphenyl)-1-(3-((1-(2-(2-(2-(((S)-1-((2S,4R)-4-hydroxy-2-((4-(4-methylthiazol-5-yl)benzyl)carbamoyl)pyrrolidin-1-yl)-3,3-dimethyl-1-oxobutan-2-yl)amino)-2-oxoethoxy)ethoxy)ethyl)-1H-1,2,3-triazol-4-yl)methoxy)phenyl)propyl 1-((S)-2-cyclohexyl-2-(3,4,5-trimethoxyphenyl)acetyl)piperidine-2-carboxylate (MTQ200)

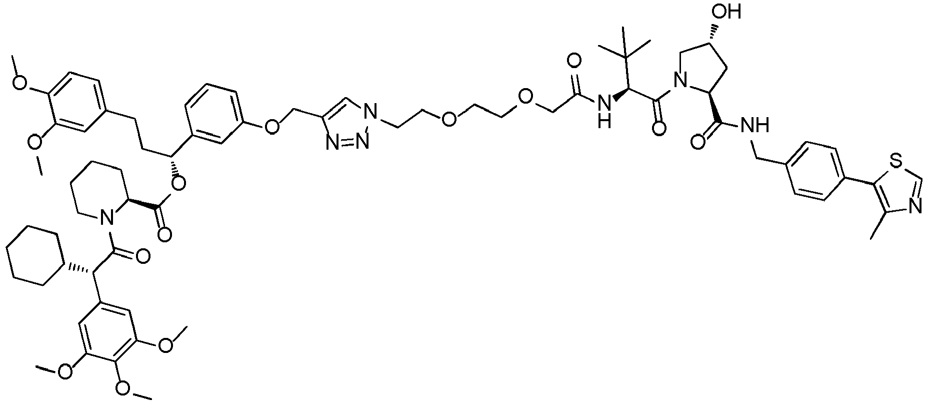

**MTQ200** was synthesized from **molecule (9)** (8 mg, 0. 0137 mmol) according to the General Method B. Purification by flash column chromatography (0% to 5% MeOH in DCM) gave a white solid (13 mg, 0.010 mmol, 74%).

The following data was obtained when analyzing the molecule in TLC, HPLC. HRMS and NMR:
TLC [DCM:MeOH 95:5]: Rf= 0.24.
HPLC [0-100% Solvent B, 20 min]: Rₜ = 17.6 min, purity (220 nm) = 99%.
¹H NMR (500 MHz, Chloroform-*d*) δ 8.66 (s, 1H), 7.97 (s, 0.75H), 7.85 (s, 0.25H), 7.36-7.31 (m, 3H), 7.30 - 7.26 (m, 2H), 7.25 - 7.08 (m, 2H), 6.99 - 6.96 (m, 0.5H), 6.86 - 6.73 (m, 2.75H), 6.69 - 6.59 (m, 2H), 6.49 (s, 1.5H), 6.46 - 6.41 (m, 1.25H), 5.54 (dd, *J* = 8.0, 5.7 Hz, 1H), 5.45 (d, *J* = 5.0 Hz, 1H), 5.17 (s, 0.5H), 5.14 (s, 1.5H), 4.70 - 4.40 (m, 7H), 4.34 - 4.24 (m, 1H), 4.05 - 3.92 (m, 3H), 3.91 - 3.86 (m, 2H), 3.85 - 3.81 (m, 9H), 3.77 (s, 2H), 3.71 (s, 4H), 3.67 - 3.56 (m, 5H), 3.38 (d, *J* = 9.8 Hz, 0.75H), 2.99 (d, *J* = 9.6 Hz, 0.25H), 2.78 (td, *J* = 13.4, 2.7 Hz, 1H), 2.64 - 2.52 (m, 1H), 2.50 (s, 3H), 2.49 - 2.23 (m, 4H), 2.06 - 1.92 (m, 2H), 1.90 - 1.80 (m, 2H), 1.66 - 1.51 (m, 5H), 1.48 - 1.37 (m, 1H), 1.34 - 1.28 (m, 2H), 1.21 - 1.09 (m, 3H), 0.96 (s, 9H), 0.91 - 0.52 (m, 3H).
HRMS: m/z: 1329.64806 [M+H]⁺, calculated 1329.64756 [M+H]⁺

### Example 16: Synthesis of (S)-(R)-3-(3,4-Dimethoxyphenyl)-1-(3-((1-((S)-16-((2S,4R)-4-hydroxy-2-((4-(4-methylthiazol-5-yl)benzyl)carbamoyl)pyrrolidine-1-carbonyl)-17,17-dimethyl-14-oxo-3,6,9,12-tetraoxa-15-azaoctadecyl)-1H-1,2,3-triazol-4-yi)methoxy)phenyl)propyl 1-((S)-2-cyclohexyl-2-(3,4,5-trimethoxyphenyl)acetyl)piperidine-2-carboxylate (MTQ202)

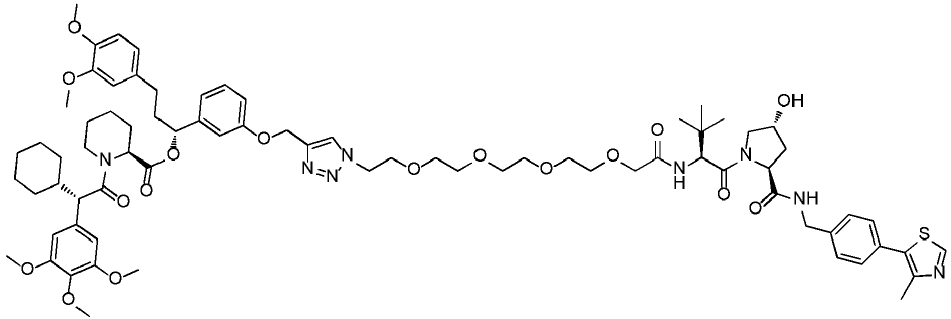

**MTQ202** was synthesized from **9** (10 mg, 0. 0137 mmol) according to the General Method B. Purification by flash column chromatography (0% to 5% MeOH in DCM) gave a white solid (17 mg, 0.012 mmol, 89%).

The following data was obtained when analyzing the molecule in TLC, HPLC, HRMS and NMR:
TLC [DCM:MeOH 95:5]: Rf= 0.29.
HPLC [0-100% Solvent B, 20 min]: Rₜ = 17.6 min, purity (220 nm) = 99%.
¹H NMR (500 MHz, Chloroform-*d*) δ 8.66 (s, 1H), 7.81 (s, 1H), 7.39 - 7.28 (m, 5.5H), 7.23 (d, *J* = 8.7 Hz, 1H), 7.13 - 6.95 (m, 1.5H), 6.87 - 6.78 (m, 1H), 6.77 - 6.74 (m, 1.5H), 6.70 - 6.59 (m, 2H), 6.49 (s, 1.5H), 6.46 - 6.41 (m, 1H), 5.58 (dd, *J* = 7.9, 5.7 Hz, 1H), 5.46 (d, *J* = 4.7 Hz, 1H), 5.17 (s, 0.5H), 5.13 (s, 1.5H), 4.73 - 4.69 (m, 1H), 4.57 - 4.48 (m, 5H), 4.37 - 4.30 (m, 1H), 4.07 (d, *J* = 11.4 Hz, 1H), 4.03 - 3.90 (m, 3H), 3.87 - 3.81 (m, 10H), 3.76 (s, 2H), 3.70 (s, 4H), 3.66 - 3.55 (m, 14H), 3.38 (d, *J* = 9.8 Hz, 1H), 2.78 (td, *J* = 13.4, 2.7 Hz, 1H), 2.64 - 2.51 (m, 2H), 2.50 (s, 3H), 2.49 - 2.33 (m, 2H), 2.31 - 2.23 (m, 1H), 2.14 - 2.06 (m, 2H), 1.99 - 1.81 (m, 3H), 1.66 - 1.54 (m, 5H), 1.48 - 1.28 (m, 3H), 1.22 - 1.05 (m, 3H), 0.95 (s, 9H), 0.88 - 0.53 (m, 2H).
HRMS: m/z: 1417.69922 [M+H]⁺, calculated 1417.69999 [M+H]⁺

### Example 17: Binding affinities of exemplary compounds

Binding to FKBP51 is an important prerequisite in order to allow degradation of FKBP51. Thus, the binding affinities of the newly synthesized molecules (table 1) were tested. The results are shown in table 2.

**Table 1:**

| **Name of molecule:** | **IUPAC-Nomenclature:** |
|---|---|
| **MTQ199** | (S)-(R)-3-(3,4-Dimethoxyphenyl)-1-(3-((1-(2-(2-(((S)-1-((2S,4R)-4-hydroxy-2-((4-(4-methylthiazol-5-yl)benzyl)carbamoyl)pyrrolidin-1-yl)-3,3-dimethyl-1-oxobutan-2-yl)amino)-2-oxoethoxy)ethyl)-1H-1,2,3-triazol-4-yl)methoxy)phenyl)propyl 1-((S)-2-cyclohexyl-2-(3,4,5-trimethoxyphenyl)acetyl)piperidine-2-carboxylate |
| **MTQ200** | (S)-(R)-3-(3,4-Dimethoxyphenyl)-1-(3-((1-(2-(2-(2-((S)-1-((2S,4R)-4-hydroxy-2-((4-(4-methylthiazol-5-yl)benzyl)carbamoyl)pyrrolidin-1-yl)-3,3-dimethyl-1-oxobutan-2-yl)amino)-2-oxoethoxy)ethoxy)ethyl)-1H-1,2,3-triazol-4-yl)methoxy)phenyl)propyl 1-((S)-2-cyclohexyl-2-(3,4,5-trimethoxyphenyl)acetyl)piperidine-2-carboxylate |
| **MTQ201** | (S)-(R)-3-(3,4-Dimethoxyphenyl)-1-(3-((1-((S)-13-((2S,4R)-4-hydroxy-2-((4-(4-methylthiazol-5-yl)benzyl)carbamoyl)pyrrolidine-1-carbonyl)-14,14-dimethyl-11-oxo-3,6,9-trioxa-12-azapentadecyl)-1H-1,2,3-triazol-4-yl)methoxy)phenyl)propyl 1-((S)-2-cyclohexyl-2-(3,4,5-trimethoxyphenyl)acetyl)piperidine-2-carboxylate |
| **MTQ202** | (S)-(R)-3-(3,4-Dimethoxyphenyl)-1-(3-((1-((S)-16-((2S,4R)-4-hydroxy-2-((4-(4-methylthiazol-5-yl)benzyl)carbamoyl)pyrrolidine-1-carbonyl)-17,17-dimethyl-14-oxo-3,6,9,12-tetraoxa-15-azaoctadecyl)-1H-1,2,3-triazol-4-yl)methoxy)phenyl)propyl 1-((S)-2-cyclohexyl-2-(3,4,5-trimethoxyphenyl)acetyl)piperidine-2-carboxylate |
| **MTQ203** | (S)-(R)-3-(3,4-Dimethoxyphenyl)-1-(3-((1-((S)-19-((2S,4R)-4-hydroxy-2-((4-(4-methylthiazol-5-yl)benzyl)carbamoyl)pyrrolidine-1-carbonyl)-20,20-dimethyl-17-oxo-3,6,9,12,15-pentaoxa-18-azahenicosyl)-1H-1,2,3-triazol-4-yl)methoxy)phenyl)propyl 1-((S)-2-cyclohexyl-2-(3,4,5-trimethoxyphenyl)acetyl)piperidine-2-carboxylate |
| **MTQ204** | (25)-(1R)-3-(3,4-Dimethoxyphenyl)-1-(3-((1-(2-(2-((2-(2,6-dioxopiperidin-3-yl)-1,3-dioxoisoindolin-4-yl)amino)-2-oxoethoxy)ethyl)-1H-1,2,3-triazol-4-yl)methoxy)phenyl)propyl1-((S)-2-cyclohexyl-2-(3,4,5-trimethoxyphenyl)acetyl)piperidine-2-carboxylate |
| **MTQ205** | (2S)-(1R)-3-(3,4-Dimethoxyphenyl)-1-(3-((1-(2-(2-(2-((2-(2,6-dioxopiperidin-3-yl)-1,3-dioxoisoindolin-4-yl)amino)-2-oxoethoxy)ethoxy)ethyl)-1H-1,2,3-triazol-4-yl)methoxy)phenyl)propyl 1-((S)-2-cyclohexyl-2-(3,4,5-trimethoxyphenyl)acetyl)piperidine-2-carboxylate |
| **MTQ206** | (2S)-(1R)-3-(3,4-Dimethoxyphenyl)-1-(3-((1-(2-(2-(2-(2-((2-(2,6-dioxopiperidin-3-yl)-1,3-dioxoisoindolin-4-yl)amino)-2-oxoethoxy)ethoxy)ethoxy)ethyl)-1H-1,2,3-triazol-4-yl)methoxy)phenyl)propyl 1-((S)-2-cyclohexyl-2-(3,4,5-trimethoxyphenyl)acetyl)piperidine-2-carboxylate |
| MTQ207 | (2S)-(1R)-3-(3,4-Dimethoxyphenyl)-1-(3-((1-(14-((2-(2,6-dioxopiperidin-3-yl)-1,3-dioxoisoindolin-4-yl)amino)-14-oxo-3,6,9,12-tetraoxatetradecyl)-1H-1,2,3-triazol-4-yl)methoxy)phenyl)propyl 1-((S)-2-cyclohexyl-2-(3,4,5-trimethoxyphenyl)acetyl)piperidine-2-carboxylate |
| **MTQ208** | (2S)-(1R)-3-(3,4-Dimethoxyphenyl)-1-(3-((1-(17-((2-(2,6-dioxopiperidin-3-yl)-1,3-dioxoisoindolin-4-yl)amino)-17-oxo-3,6,9,12,15-pentaoxaheptadecyl)-1H-1,2,3-triazol-4-yl)methoxy)phenyl)propyl 1-((S)-2-cyclohexyl-2-(3,4,5-trimethoxyphenyl)acetyl)piperidine-2-carboxylate |
| **MTQ328** | (S)-(R)-3-(4-((1-(2-(2-(((S)-1-((2S,4R)-4-Hydroxy-2-((4-(4-methylthiazol-5-yl)benzyl)carbamoyl)pyrrolidin-1-yl)-3,3-dimethyl-1-oxobutan-2-yl)amino)-2-oxoethoxy)ethyl)-1H-1,2,3-triazol-4-yl)methoxy)-3-methoxyphenyl)-1-(3-methoxyphenyl)propyl 1-((S)-2-cyclohexyl-2-(3,4,5-trimethoxyphenyl)acetyl)piperidine-2-carboxylate |
| **MTQ329** | (S)-(R)-3-(4-((1-(2-(2-(2-(((S)-1-((2S,4R)-4-Hydroxy-2-((4-(4-methylthiazol-5-yl)benzyl)carbamoyl)pyrrolidin-1-yl)-3,3-dimethyl-1-oxobutan-2-yl)amino)-2-oxoethoxy)ethoxy)ethyl)-1H-1,2,3-triazol-4-yl)methoxy)-3-methoxyphenyl)-1-(3-methoxyphenyl)propyl 1-((S)-2-cyclohexyl-2-(3,4,5-trimethoxyphenyl)acetyl)piperidine-2-carboxylate |
| **MTQ330** | (S)-(R)-3-(4-((1-((S)-13-((2S,4R)-4-Hydroxy-2-((4-(4-methylthiazol-5-yl)benzyl)carbamoyl)pyrrolidine-1-carbonyl)-14,14-dimethyl-11-oxo-3,6,9-trioxa-12-azapentadecyl)-1H-1,2,3-triazol-4-yl)methoxy)-3-methoxyphenyl)-1-(3-methoxyphenyl)propyl 1-((S)-2-cyclohexyl-2-(3,4,5-trimethoxyphenyl)acetyl)piperidine-2-carboxylate |
| **MTQ331** | (S)-(R)-3-(4-((1-((5)-16-((25,4R)-4-Hydroxy-2-((4-(4-methylthiazol-5-yl)benzyl)carbamoyl)pyrrolidine-l-carbonyl)-17,17-dimethyl-14-oxo-3,6,9,12-tetraoxa-15-azaoctadecyl)-1H-1,2,3-triazol-4-yl)methoxy)-3-methoxyphenyl)-1-(3-methoxyphenyl)propyl 1-((S)-2-cyclohexyl-2-(3,4,5-trimethoxyphenyl)acetyl)piperidine-2-carboxylate |
| **MTQ332** | (S)-(R)-3-(4-((1-((S)-19-((2S,4R)-4-Hydroxy-2-((4-(4-methylthiazo1-5-yl)benzyl)carbamoyl)pyrrolidine-1-carbonyl)-20,20-dimethyl-17-oxo-3,6,9,12,15-pentaoxa-18-azahenicosyl)-1H-1,2,3-triazol-4-yl)methoxy)-3-methoxyphenyl)-1-(3-methoxyphenyl)propyl 1-((S)-2-cyclohexyl-2-(3,4,5-trimethoxyphenyl)acetyl)piperidine-2-carboxylate |
| **MTQ333** | (25)-(1R)-3-(4-((1-(2-(2-((2-(2,6-Dioxopiperidin-3-yl)-1,3-dioxoisoindolin-4-yl)amino)-2-oxoethoxy)ethyl)-1H-1,2,3-triazol-4-yl)methoxy)-3-methoxyphenyl)-1-(3-methoxyphenyl)propyl 1-((S)-2-cyclohexyl-2-(3,4,5-trimethoxyphenyl)acetyl)piperidine-2-carboxylate |
| **MTQ334** | (25)-(1R)-3-(4-((1-(2-(2-(2-((2-(2,6-Dioxopiperidin-3-yl)-1,3-dioxoisoindolin-4-yl)amino)-2-oxoethoxy)ethoxy)ethyl)-1H-1,2,3-triazol-4-yl)methoxy)-3-methoxyphenyl)-1-(3-methoxyphenyl)propyl 1-((S)-2-cyclohexyl-2-(3,4,5-trimethoxyphenyl)acetyl)piperidine-2-carboxylate |

**Table 2:**

| **Name of molecule:** | **Kᵢ (nM)** |
|---|---|
| | **FKBP51FK1** |
| **Molecule (9)** | 32 ± 7 |
| **Molecule (10)** | 149 ± 39 |
| **MTQ199** | 25 ± 6 |
| **MTQ200** | 24 ± 7 |
| **MTQ201** | 23 ± 7 |
| **MTQ202** | 24 ± 8 |
| **MTQ203** | 12 ± 3 |
| **MTQ204** | 19 ± 5 |
| **MTQ205** | 16 ± 5 |
| **MTQ206** | 22 ± 6 |
| **MTQ207** | 18 ± 4 |
| **MTQ208** | 5 ± 1 |
| **MTQ229** | 36 ± 9 |
| **MTQ235** | 32 ± 12 |
| **MTQ328** | 47 ± 19 |
| **MTQ329** | 60 ± 16 |
| **MTQ330** | 86 ± 26 |
| **MTQ331** | 59 ± 22 |
| **MTQ332** | 34 ± 10 |
| **MTQ333** | 22 ± 6 |
| **MTQ334** | 21 ± 10 |

### Example 18: Degradation of FKBP51 with MTQ202 - (HeLa cells // 24h)

HeLa cells were incubated with the PROTAC MTQ202. 35,000 cells per well in a 24 well format. Figure 2 - Upper blot: Endogeneous FKBP51. Figure 2 - Lower blot: GAPDH. Cells were treated with the compound for 24 h before lysis. All samples have been exposed to a constant DMSO concentration. Lysate protein concentration was quantified via BCA assay and normalized with Lämmli buffer. Equal amounts of total protein were subjected to SDS-Page and Western Blot afterwards. The degradation is clearly visible, but not complete. This blot shows duplicates.

### Example 19: Degradation of FKBP51 with MTQ202 - (HEK293T cells // 48h)

HEK293T cells were incubated with the PROTAC MTQ202. 35,000 cells per well in a 24 well format. Figure 3-Upper blot: Endogeneous FKBP51. Figure 3-Lower blot: GAPDH. Cells were treated with the compound for 48 h before lysis. All samples have been exposed to a constant DMSO concentration. Lysate protein concentration was quantified via BCA assay and normalized with Lämmli buffer. Equal amounts of total protein were subjected to SDS-Page and Western Blot afterwards. The degradation is clearly visible, but not complete.

## Claims

1. A proteolysis-targeting chimera (PROTAC) for degradation of FK506 binding proteins (FKBPs), comprising a FKBP-binding moiety and E3-binding moiety connected via a Linker (L), represented by the following general formula I: 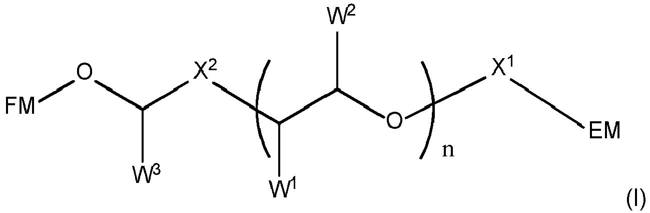
wherein **n** is an integer from 0 to 10,
**X1** represents a bond, -CH₂(C=O)NH-,-CH₂(C=O)NMe-, -CH(CH₃)(C=O)NH-,-CH(CH₃)(C=O)NMe-, -CH₂-,-C(O)-, -(C=O)NH-, -NH(C=O)-, -(C=O)NHCH₂-, - (C=O)NMeCH₂-, -NH(C=O)CH₂-;
**X2** represents 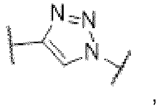 -(C=O)NH- or-CH2NH(C=O)-; -CH₂O-, -CH(CH₃)O-, wherein **W¹, W²** and **W³** independently represents -H and/or -CH₃;
wherein the "FKBP-binding moiety" **(FM)** represents a ligand capable of binding to FK506 binding proteins (FKBPs);
wherein the "E3-binding moiety" **(EM)** represents ligand capable of binding an E3 ubiquitin ligase;
and wherein, upon binding of the PROTAC to the FKBP and the E3 ubiquitin ligase, the FKBP is degraded by a ubiquitin mediated proteolysis pathway within a cell;
and enantiomers, stereoisomeric forms, mixtures of enantiomers, anomers, deoxy-forms, diastereomers, mixtures of diastereomers, prodrugs, tautomers, hydrates, solvates and racemates of the above mentioned compounds and pharmaceutically acceptable salts thereof.

2. The compound according to any of the previous claims, wherein **FM** has the structure of: 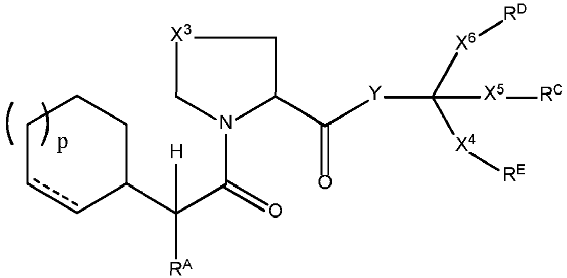 **wherein**
**X3** represents -CH₂₋, -CH₂CH₂-, -CH=CH-, -CH₂-S-, or -S-CH₂-;
**Y** represents -NH-, or -O-;
**p** is an integer of 0 or 1;
" 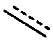 " represents a C=C bond or a C-C bond;
wherein **R^{E}** represents **R^{L;}**
and **R^{A}** represents 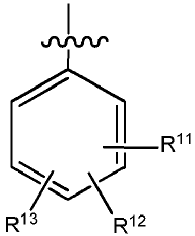
**R^{L}** represents: 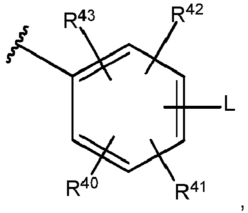 wherein L represents the chemical linker L as defined above;
**R^{C}** represents: **R²⁷** or 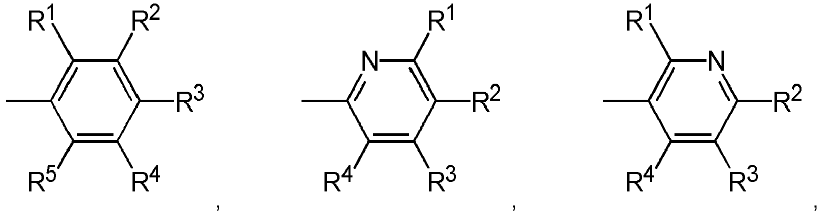 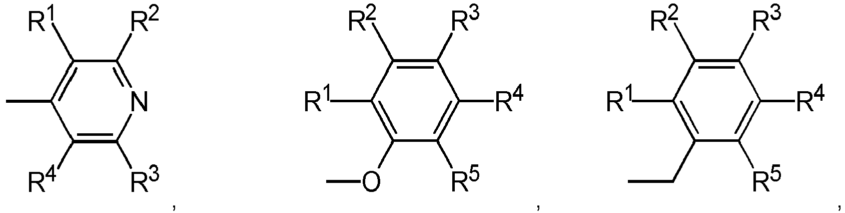 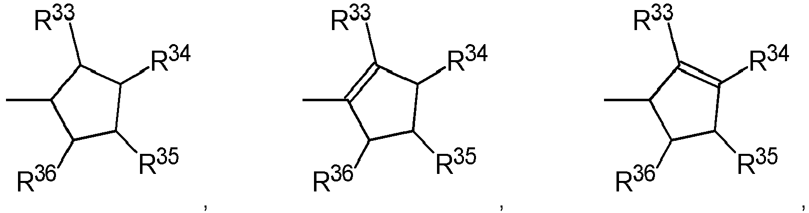 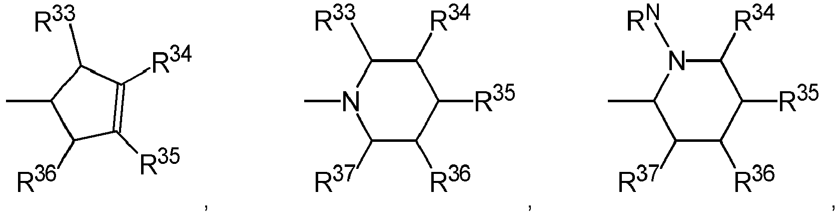 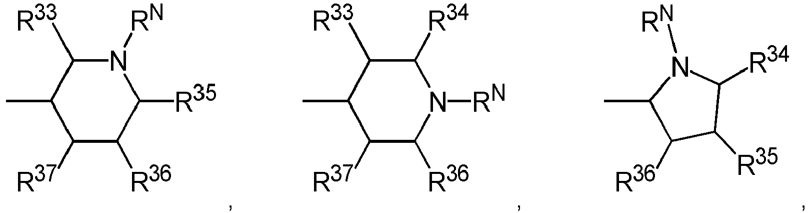 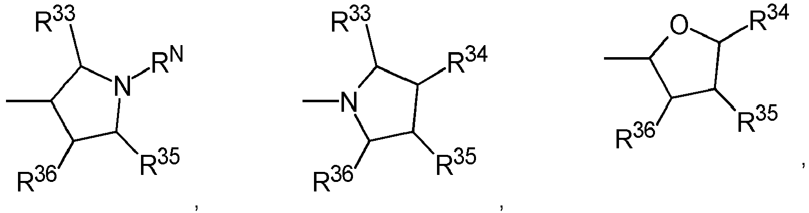 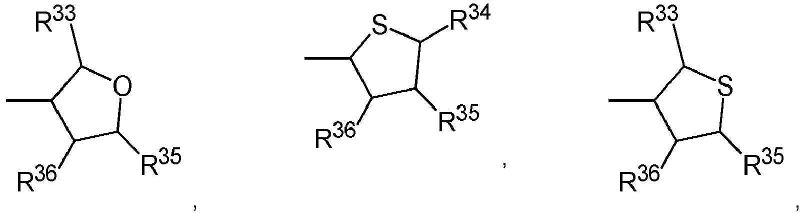 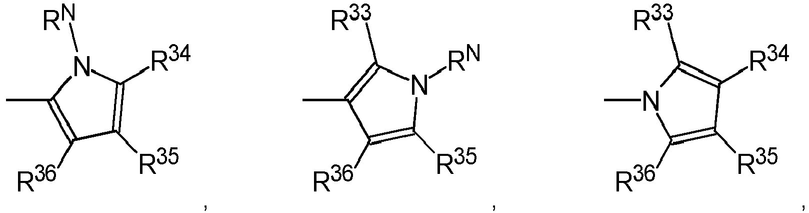 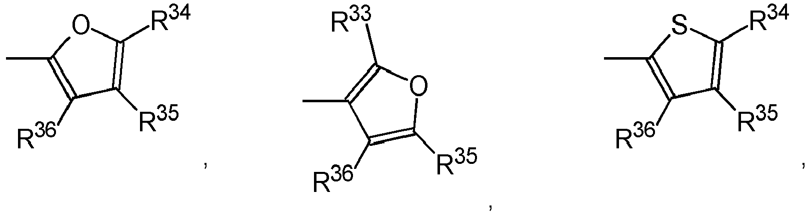 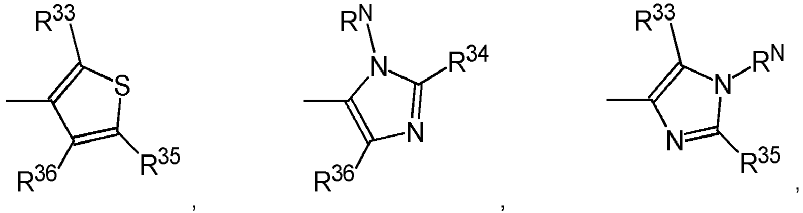 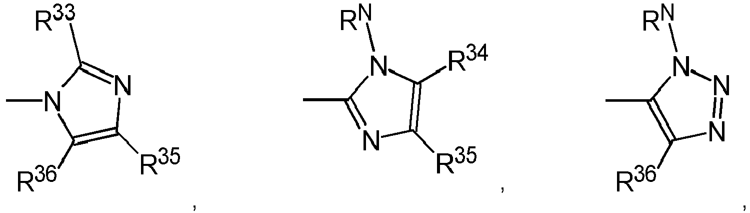 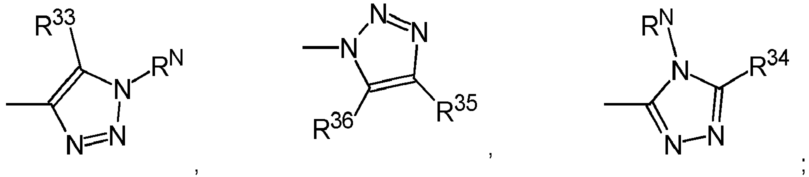
**R^{D}** represents: **R²⁸** or 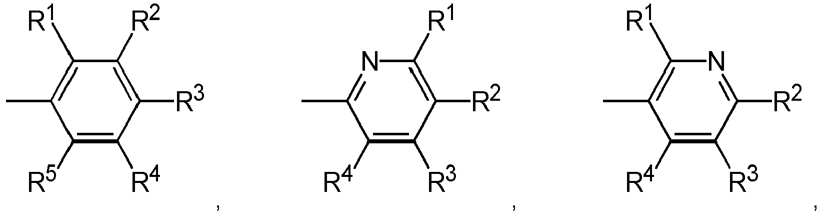 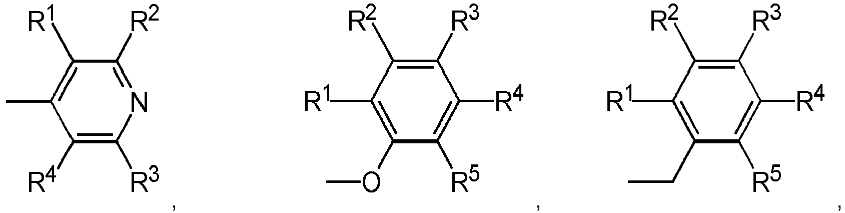 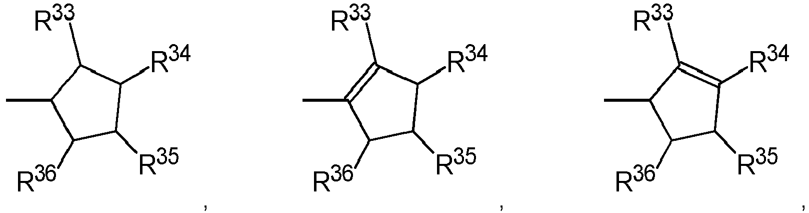 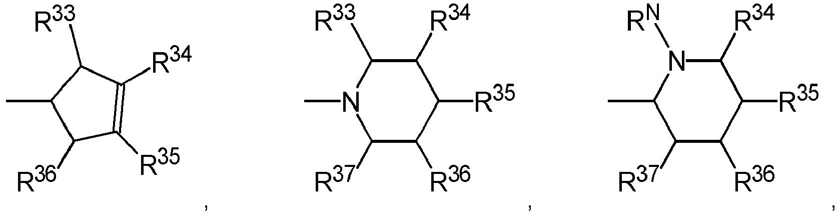 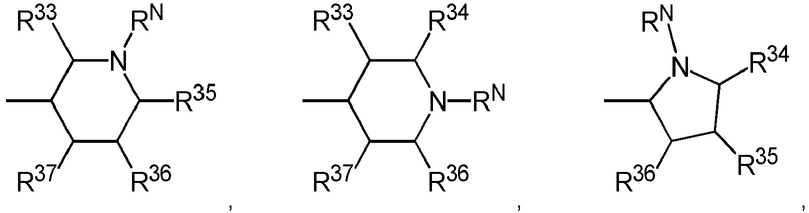 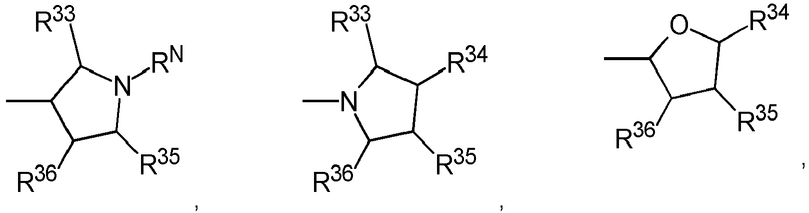 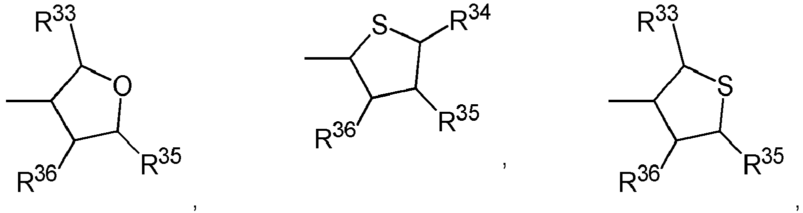 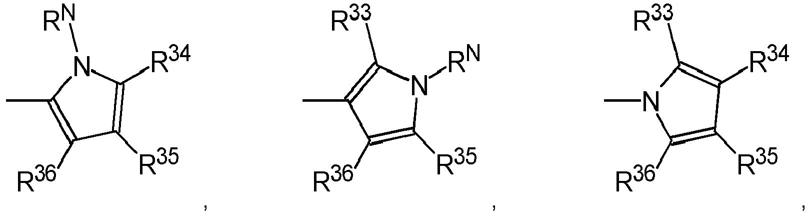 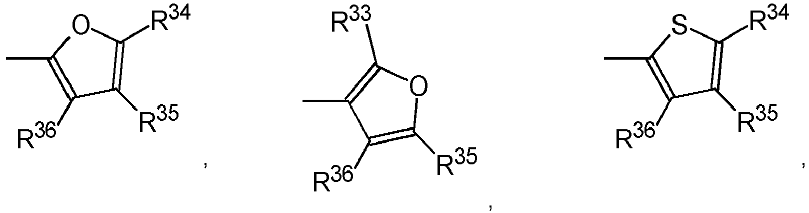 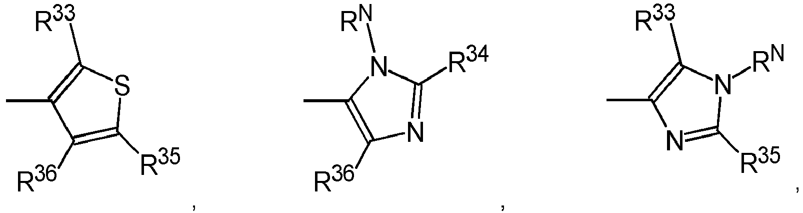 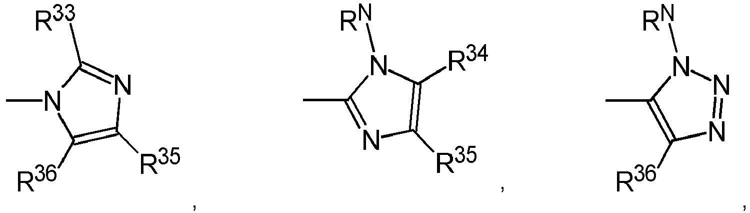 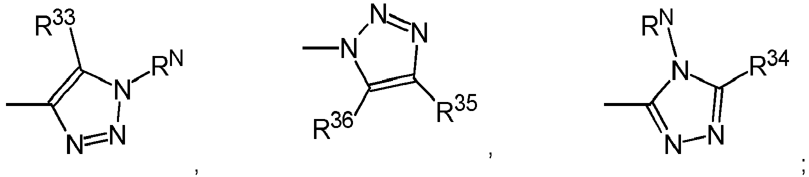
**R¹- R²², R^{18'}** - **R^{22'}, R²⁶ - R⁴³** represent independently of each other -H, -OH, -OCH₃, -OC₂H₅, -OC₃H₇, -O-cyclo-C₃H₅, -OCH(CH₃)₂, -OC(CH₃)₃, -OC₄H₉, -OCH₂-COOH, -OPh, -OCH₂-Ph, -OCPh₃, -CH₂-OH, -C₂H₄-OH, -C₃H₆-OH, -CH(OH)-CH₂-OH, -CH₂-OCH₃, -C₂H₄-OCH₃, -C₃H₆-OCH₃, -CH₂-OC₂H₅, -C₂H₄-OC₂H₅, -C₃H₆-OC₂H₅, -CH₂-OC₃H₇, -C₂H₄-OC₃H₇, -C₃H₆-OC₃H₇, -CH₂-O-cyclo-C₃H₅, -C₂H₄-O-cyclo-C₃H₅, -C₃H₆-O-cyclo-C₃H₅, -CH₂-OCH(CH₃)₂, -C₂H₄-OCH(CH₃)₂, -C₃H₆-OCH(CH₃)₂, -CH₂-OC(CH₃)₃, -C₂H₄-OC(CH₃)₃, -C₃H₆-OC(CH₃)₃, -CH₂-OC₄H₉, -C₂H₄-OC₄H₉, -C₃H₆-OC₄H₉, -CH₂-OPh, -C₂H₄-OPh, -C₃H₆-OPh, -CH₂-OCH₂-Ph, -C₂H₄-OCH₂-Ph, -C₃H₆-OCH₂-Ph, -SH, -SCH₃, -SC₂H₅, -SC₃H₇, -S-cyclo-C₃H₅, -SCH(CH₃)₂, -SC(CH₃)₃, -NO₂, -F, -CI, -Br, -I, -P(O)(OH)₂, -P(O)(OCH₃)₂, -P(O)(OC₂H₅)₂, -P(O)(OCH(CH₃)₂)₂, -C(OH)[P(O)(OH)₂]₂, -Si(CH₃)₂(C(CH₃)₃), -Si(C₂H₅)₃, -Si(CH₃)₃, -N₃, -CN, -OCN, -NCO, -SCN, -NCS, -CHO, -COCH₃, -COC₂H₅, -COC₃H₇, -CO-cyclo-C₃H₅, -COCH(CH₃)₂, -COC(CH₃)₃, -COOH, -COCN, -COOCH₃, -COOC₂H₅, -COOC₃H₇, -COO-cyclo-C₃H₅, -COOCH(CH₃)₂, -COOC(CH₃)₃, -OOC-CH₃, -OOC-C₂H₅, -OOC-C₃H₇, -OOC-cyclo-C₃H₅, -OOC-CH(CH₃)₂, -OOC-C(CH₃)₃, -CONH₂, -CH₂-CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -NHCOCH₃, -NHCOC₂H₅, -NHCOC₃H₇, -NHCO-cyclo-C₃H₅, -NHCO-CH(CH₃)₂, -NHCO-C(CH₃)₃, -NHCO-OCH₃, -NHCO-OC₂H₅, -NHCO-OC₃H₇, -NHCO-O-cyclo-C₃H₅, -NHCO-OCH(CH₃)₂, -NHCO-OC(CH₃)₃, -NH₂, -NHCH₃, -NHC₂H₅, -NHC₃H₇, -NH-cyclo-C₃H₅, -NHCH(CH₃)₂, -NHC(CH₃)₃, -N(CH₃)₂, -N(C₂H₅)₂, -N(C₃H₇)₂, -N(cyclo-C₃H₅)₂, -N[CH(CH₃)₂]₂, -N[C(CH₃)₃]₂, -SOCH₃, -SOC₂H₅, -SOC₃H₇, -SO-cyclo-C₃H₅, -SOCH(CH₃)₂, -SOC(CH₃)₃, -SO₂CH₃, -SO₂C₂H₅, -SO₂C₃H₇, -SO₂-cyclo-C₃H₅, -SO₂CH(CH₃)₂, -SO₂C(CH₃)₃, -SO₃H, -SO₃CH₃, -SO₃C₂H₅, -SO₃C₃H₇, -SO₃-cyclo-C₃H₅, -SO₃CH(CH₃)₂, -SO₃C(CH₃)₃, -SO₂NH₂, -SO₂NHCH₃, -SO₂NHC₂H₅, -SO₂NHC₃H₇, -SO₂NH-cyclo-C₃H₅, -SO₂NHCH(CH₃)₂, -SO₂NHC(CH₃)₃, -SO₂N(CH₃)₂, -SO₂N(C₂H₅)₂, -SO₂N(C₃H₇)₂, -SO₂N(cyclo-C₃H₅)₂, -SO₂N[CH(CH₃)₂]₂, -SO₂N[C(CH₃)₃]₂, -O-S(=O)CH₃, -O-S(=O)C₂H₅, -O-S(=O)C₃H₇, -O-S(=O)-cyclo-C₃H₅, -O-S(=O)CH(CH₃)₂, -O-S(=O)C(CH₃)₃, -S(=O)(=NH)CH₃, -S(=O)(=NH)C₂H₅, -S(=O)(=NH)C₃H₇, -S(=O)(=NH)-cyclo-C₃H₅, -S(=O)(=NH)CH(CH₃)₂, -S(=O)(=NH)C(CH₃)₃, -NH-SO₂-CH₃, -NH-SO₂-C₂H₅, -NH-SO₂-C₃H₇, -NH-SO₂-cyclo-C₃H₅, -NH-SO₂-CH(CH₃)₂, -NH-SO₂-C(CH₃)₃, -O-SO₂-CH₃, -O-SO₂-C₂H₅, -O-SO₂-C₃H₇, -O-SO₂-cyclo-C₃H₅, -O-SO₂-CH(CH₃)₂, -O-SO₂-C(CH₃)₃, -OCF₃, -CH₂-OCF₃, -C₂H₄-OCF₃, -C₃H₆-OCF₃, -OC₂F₅, -CH₂-OC₂F₅, -C₂H₄-OC₂F₅, -C₃H₆-OC₂F₅, -O-COOCH₃, -O-COOC₂H₅, -O-COOC₃H₇, -O-COO-cyclo-C₃H₅, -O-COOCH(CH₃)₂, -O-COOC(CH₃)₃, -NH-CO-NH₂, -NH-CO-NHCH₃, -NH-CO-NHC₂H₅, -NH-CS-N(C₃H₇)₂, -NH-CO-NHC₃H₇, -NH-CO-N(C₃H₇)₂, -NH-CO-NH[CH(CH₃)₂], -NH-CO-NH[C(CH₃)₃], -NH-CO-N(CH₃)₂, -NH-CO-N(C₂H₅)₂, -NH-CO-NH-cyclo-C₃H₅, -NH-CO-N(cyclo-C₃H₅)₂, -NH-CO-N[CH(CH₃)₂]₂, -NH-CS-N(C₂H₅)₂, -NH-CO-N[C(CH₃)₃]₂, -NH-CS-NH₂, -NH-CS-NHCH₃, -NH-CS-N(CH₃)₂, -NH-CS-NHC₂H₅, -NH-CS-NHC₃H₇, -NH-CS-NH-cyclo-C₃H₅, -NH-CS-NH[CH(CH₃)₂], -NH-CS-NH[C(CH₃)₃], -NH-CS-N(cyclo-C₃H₅)₂, -NH-CS-N[CH(CH₃)₂]₂, -NH-CS-N[C(CH₃)₃]₂, -NH-C(=NH)-NH₂, -NH-C(=NH)-NHCH₃, -NH-C(=NH)-NHC₂H₅, -NH-C(=NH)-NHC₃H₇, -O-CO-NH-cyclo-C₃H₅, -NH-C(=NH)-NH-cyclo-C₃H₅, -NH-C(=NH)-NH[CH(CH₃)₂] -O-CO-NH[CH(CH₃)₂], -NH-C(=NH)-NH[C(CH₃)₃], -NH-C(=NH)-N(CH₃)₂, -NH-C(=NH)-N(C₂H₅)₂, -NH-C(=NH)-N(C₃H₇)₂, -NH-C(=NH)-N(cyclo-C₃H₅)₂, -O-CO-NHC₃H₇, -NH-C(=NH)-N[CH(CH₃)₂]₂, -NH-C(=NH)-N[C(CH₃)₃]₂, -O-CO-NH₂, -O-CO-NHCH₃, -O-CO-NHC₂H₅, -O-CO-NH[C(CH₃)₃], -O-CO-N(CH₃)₂, -O-CO-N(C₂H₅)₂, -O-CO-N(C3H₇)₂, -O-CO-N(cyclo-C₃H₅)₂, -O-CO-N[CH(CH₃)₂]₂, -O-CO-N[C(CH₃)₃]₂, -O-CO-OCH₃, -O-CO-OC₂H₅, -O-CO-OC₃H₇, -O-CO-O-cyclo-C₃H₅, -O-CO-OCH(CH₃)₂, -O-CO-OC(CH₃)₃, -CH₂F, -CHF₂, -CF₃, -CH₂Cl, -CH₂Br, -CH₂I, -CH₂-CH₂F, -CH₂-CHF₂, -CH₂-CF₃, -CH₂-CH₂Cl, -CH₂-CH₂Br, -CH₂-CH₂I, -cyclo-C₅H₉, -cyclo-C₆H₁₁, -CH₂-cyclo-C₆H₁₁, -CH₂-CH₂-cyclo-C₆H₁₁, -cyclo-C₇H₁₃, -cyclo-C₈H₁₅, -Ph, -CH₂-Ph, -CH₂-CH₂-Ph, -CH=CH-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₇, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -C₂H₄-CH(CH₃)-CH=CH₂, -CH₂-CH(CH₃)-CH₂-CH=CH₂, -C₂H₄-CH=C(CH₃)₂, -CH(CH₃)-C₂H₄-CH=CH₂, -C₂H₄-C(CH₃)=CH-CH₃, -CH₂-CH(CH₃)-CH=CH-CH₃, -CH(CH₃)-CH₂-CH=CH-CH₃, -CH₂-CH=CH-CH(CH₃)₂, -CH₂-CH=C(CH₃)-C₂H₅, -CH₂-C(CH₃)=CH-C₂H₅, -CH(CH₃)-CH=CH-C₂H₅, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -C[C(CH₃)₃]=CH₂, -CH(CH₃)-CH₂-C(CH₃)=CH₂, -CH(CH₃)-CH(CH₃)-CH=CH₂, -CH=CH-C₂H₄-CH=CH₂, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH=CH-CH₂-CH=CH-CH₃, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[CH(CH₃)(C₂H₅)]=CH₂, -C[CH₂-CH(CH₃)₂]=CH₂, -C₂H₄-CH=CH-CH=CH₂, -CH₂-CH=CH-CH₂-CH=CH₂, -C₃H₆-C≡C-CH₃, -CH₂-CH=CH-CH=CH-CH₃, -CH=CH-CH=CH-C₂H₅, -CH₂-CH=CH-C(CH₃)=CH₂, -CH₂-CH=C(CH₃)-CH=CH₂, -CH₂-C(CH₃)=CH-CH=CH₂, -CH(CH₃)-CH₂-C≡CH, -CH(CH₃)-CH=CH-CH=CH₂, -CH=CH-CH₂-C(CH₃)=CH₂, -CH(CH₃)-C≡C-CH₃, -CH=CH-CH(CH₃)-CH=CH₂, -CH=C(CH₃)-CH₂-CH=CH₂, -C₂H₄-CH(CH₃)-C≡CH, -C(CH₃)=CH-CH₂-CH=CH₂, -CH=CH-CH=C(CH₃)₂, -CH₂-CH(CH₃)-CH₂-C≡CH, -CH=CH-C(CH₃)=CH-CH₃, -CH=C(CH₃)-CH=CH-CH₃, -CH₂-CH(CH₃)-C≡CH, -C(CH₃)=CH-CH=CH-CH₃, -CH=C(CH₃)-C(CH₃)=CH₂, -C(CH₃)=CH-C(CH₃)=CH₂, -C(CH₃)=C(CH₃)-CH=CH₂, -CH=CH-CH=CH-CH=CH₂, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₃, -C≡C-C₃H₇, -CH(CH₃)-C≡CH, -C₄H₈-C≡CH, -C₂H₄-C≡C-C₂H₅, -CH₂-C≡C-C₃H₇, -C≡C-C₄H₉, -C≡C-C(CH₃)₃, -CH(CH₃)-C₂H₄-C≡CH, -CH₂-CH(CH₃)-C≡C-CH₃, -CH(CH₃)-CH₂-C≡C-CH₃, -CH(CH₃)-C≡C-C₂H₅, -CH₂-C≡C-CH(CH₃)₂, -C≡C-CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₃)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡CH, -CH₂-CH(C≡CH)₂, -C≡C-C≡CH, -CH₂-C≡C-C≡CH, -C≡C-C≡C-CH₃, -CH(C≡CH)₂, -C₂H₄-C≡C-C≡CH, -CH₂-C≡C-CH₂-C≡CH, -C≡C-C₂H₄-C≡CH, -CH₂-C≡C-C≡C-CH₃, -C≡C-CH₂-C≡C-CH₃, -C≡C-C≡C-C₂H₅, -C(C≡CH)₂-CH₃, -C≡C-CH(CH₃)-C≡CH, -CH(CH₃)-C≡C-C≡CH, -CH(C≡CH)-CH₂-C≡CH, -CH(C≡CH)-C≡C-CH₃, 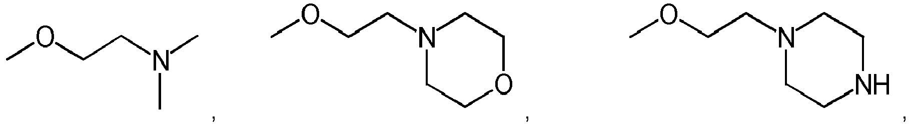 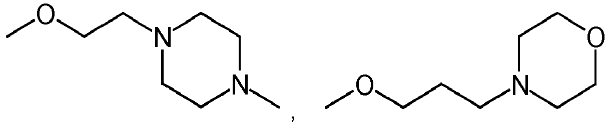 or
**R¹⁸** and **R^{18'}** or **R¹⁹** and **R^{19'}** or **R²⁰** and **R^{20'}** or **R²¹** and **R^{21'}** or **R²²** and **R^{22'}** can form together 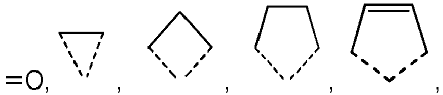 or =C**R^{23'}R^{24'}**, wherein **R^{23'}** and **R^{24'}** represent of each other -H, -CH₃, -C₂H₅, -CF₃, -CH₂CF₃, -C₂F₅;
**R²³-R²⁵** represent independently of each other -H, -CH₂-OCH₃, -C₂H₄-OCH₃, -C₃H₆-OCH₃, -CH₂-OC₂H₅, -C₂H₄-OC₂H₅, -C₃H₆-OC₂H₅, -CH₂-OC₃H₇, -C₂H₄-OC₃H₇, -C₃H₆-OC₃H₇, -CH₂-O-cyclo-C₃H₅, -C₂H₄-O-cyclo-C₃H₅, -C₃H₆-O-cyclo-C₃H₅, -CH₂-OCH(CH₃)₂, -C₂H₄-OCH(CH₃)₂, -C₃H₆-OCH(CH₃)₂, -CH₂-OC(CH₃)₃, -C₂H₄-OC(CH₃)₃, -C₃H₆-OC(CH₃)₃, -CH₂-OC₄H₉, -C₂H₄-OC₄H₉, -C₃H₆-OC₄H₉, -CH₂-OPh, -C₂H₄-OPh, -C₃H₆-OPh, -CH₂-OCH₂-Ph, -C₂H₄-OCH₂-Ph, -C₃H₆-OCH₂-Ph, -CH₂F, -CHF₂, -CF₃, -CH₂Cl, -CH₂Br, -CH₂I, -CH₂-CH₂F, -CH₂-CHF₂, -CH₂-CF₃, -CH₂-CH₂Cl, -CH₂-CH₂Br, -CH₂-CH₂I, -cyclo-C₈H₁₅, -Ph, -CH₂-Ph, -CH₂-CH₂-Ph, -CH=CH-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₇, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -C₂H₄-CH(CH₃)-CH=CH₂, -CH₂-CH(CH₃)-CH₂-CH=CH₂, -C₂H₄-CH=C(CH₃)₂, -CH(CH₃)-C₂H₄-CH=CH₂, -C₂H₄-C(CH₃)=CH-CH₃, -CH₂-CH(CH₃)-CH=CH-CH₃, -CH(CH₃)-CH₂-CH=CH-CH₃, -CH₂-CH=CH-CH(CH₃)₂, -CH₂-CH=C(CH₃)-C₂H₅, -CH₂-C(CH₃)=CH-C₂H₅, -CH(CH₃)-CH=CH-C₂H₅, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -C[C(CH₃)₃]=CH₂, -CH(CH₃)-CH₂-C(CH₃)=CH₂, -CH(CH₃)-CH(CH₃)-CH=CH₂, -CH=CH-C₂H₄-CH=CH₂, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH=CH-CH₂-CH=CH-CH₃, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[CH(CH₃)(C₂H₅)]=CH₂, -C[CH₂-CH(CH₃)₂]=CH₂, -C₂H₄-CH=CH-CH=CH₂, -CH₂-CH=CH-CH₂-CH=CH₂, -C₃H₆-C≡C-CH₃, -CH₂-CH=CH-CH=CH-CH₃, -CH=CH-CH=CH-C₂H₅, -CH₂-CH=CH-C(CH₃)=CH₂, -CH₂-CH=C(CH₃)-CH=CH₂, -CH₂-C(CH₃)=CH-CH=CH₂, -CH(CH₃)-CH₂-C≡CH, -CH(CH₃)-CH=CH-CH=CH₂, -CH=CH-CH₂-C(CH₃)=CH₂, -CH(CH₃)-C≡C-CH₃, -CH=CH-CH(CH₃)-CH=CH₂, -CH=C(CH₃)-CH₂-CH=CH₂, -C₂H₄-CH(CH₃)-C≡CH, -C(CH₃)=CH-CH₂-CH=CH₂, -CH=CH-CH=C(CH₃)₂, -CH₂-CH(CH₃)-CH₂-C≡CH, -CH=CH-C(CH₃)=CH-CH₃, -CH=C(CH₃)-CH=CH-CH₃, -CH₂-CH(CH₃)-C≡CH, -C(CH₃)=CH-CH=CH-CH₃, -CH=C(CH₃)-C(CH₃)=CH₂, -C(CH₃)=CH-C(CH₃)=CH₂, -C(CH₃)=C(CH₃)-CH=CH₂, -CH=CH-CH=CH-CH=CH₂, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -C₄H₈-C≡CH, -C₂H₄-C≡C-C₂H₅, -CH₂-C≡C-C₃H₇, -C≡C-C₄H₉, -C≡C-C(CH₃)₃, -CH(CH₃)-C₂H₄-C≡CH, -CH₂-CH(CH₃)-C≡C-CH₃, -CH(CH₃)-CH₂-C≡C-CH₃, -CH(CH₃)-C≡C-C₂H₅, -CH₂-C≡C-CH(CH₃)₂, -C≡C-CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₃)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡CH, -CH₂-CH(C≡CH)₂, -C≡C-C≡CH, -CH₂-C≡C-C≡CH, -C≡C-C≡C-CH₃, -CH(C≡CH)₂, -C₂H₄-C≡C-C≡CH, -CH₂-C≡C-CH₂-C≡CH, -C≡C-C₂H₄-C≡CH, -CH₂-C≡C-C≡C-CH₃, -C≡C-CH₂-C≡C-CH₃, -C≡C-C≡C-C₂H₅, -C(C≡CH)₂-CH₃, -C≡C-CH(CH₃)-C≡CH, -CH(CH₃)-C≡C-C≡CH, -CH(C≡CH)-CH₂-C≡CH, -CH(C≡CH)-C≡C-CH₃;
**R^{N}** represents -H, -CH₂-OCH₃, -C₂H₄-OCH₃, -C₃H₆-OCH₃, -CH₂-OC₂H₅, -C₂H₄-OC₂H₅, -C₃H₆-OC₂H₅, -CH₂-OC₃H₇, -C₂H₄-OC₃H₇, -C₃H₆-OC₃H₇, -CH₂-O-cyclo-C₃H₅, -C₂H₄-O-cyclo-C₃H₅, -C₃H₆-O-cyclo-C₃H₅, -CH₂-OCH(CH₃)₂, -C₂H₄-OCH(CH₃)₂, -C₃H₆-OCH(CH₃)₂, -CH₂-OC(CH₃)₃, -C₂H₄-OC(CH₃)₃, -C₃H₆-OC(CH₃)₃, -CH₂-OC₄H₉, -C₂H₄-OC₄H₉, -C₃H₆-OC₄H₉, -CH₂-OPh, -C₂H₄-OPh, -C₃H₆-OPh, -CH₂-OCH₂-Ph, -C₂H₄-OCH₂-Ph, -C₃H₆-OCH₂-Ph, -CHO, -COCH₃, -COC₂H₅, -COC₃H₇, -CO-cyclo-C₃H₅, -COCH(CH₃)₂, -COC(CH₃)₃, -COCN, -COOCH₃, -COOC₂H₅, -COOC₃H₇, -COO-cyclo-C₃H₅, -COOCH(CH₃)₂, -COOC(CH₃)₃, -CONH₂, -CONHCH₃, -CONHC₂H₅, -CONHC₃H₇, -CONH-cyclo-C₃H₅, -CONH[CH(CH₃)₂], -CONH[C(CH₃)₃], -CON(CH₃)₂, -CON(C₂H₅)₂, -CON(C₃H₇)₂, -CON(cyclo-C₃H₅)₂, -CON[CH(CH₃)₂]₂, -CON[C(CH₃)₃]₂, -SO₂CH₃, -SO₂C₂H₅, -SO₂C₃H₇, -SO₂-cyclo-C₃H₅, -SO₂CH(CH₃)₂, -SO₂C(CH₃)₃, -CH₂-OCF₃, -C₂H₄-OCF₃, -C₃H₆-OCF₃, -OC₂F₅, -CH₂-OC₂F₅, -C₂H₄-OC₂F₅, -C₃H₆-OC₂F₅, -CH₂F, -CHF₂, -CF₃, -CH₂Cl, -CH₂Br, -CH₂I, -CH₂-CH₂F, -CH₂-CHF₂, -CH₂-CF₃, -CH₂-CH₂Cl, -CH₂-CH₂Br, -CH₂-CH₂I, -cyclo-C₈H₁₅, -Ph, -CH₂-Ph, -CH₂-CH₂-Ph, -CH=CH-Ph, -CPh₃, -CH₃, -C₂H₅, -C₃H₇, -CH(CH₃)₂, -C₄H₉, -CH₂-CH(CH₃)₂, -CH(CH₃)-C₂H₅, -C(CH₃)₃, -C₅H₁₁, -CH(CH₃)-C₃H₇, -CH₂-CH(CH₃)-C₂H₅, -CH(CH₃)-CH(CH₃)₂, -C(CH₃)₂-C₂H₅, -CH₂-C(CH₃)₃, -CH(C₂H₅)₂, -C₂H₄-CH(CH₃)₂, -C₆H₁₃, -C₇H₁₅, -C₈H₁₇, -C₃H₆-CH(CH₃)₂, -C₂H₄-CH(CH₃)-C₂H₅, -CH(CH₃)-C₄H₉, -CH₂-CH(CH₃)-C₃H₇, -CH(CH₃)-CH₂-CH(CH₃)₂, -CH(CH₃)-CH(CH₃)-C₂H₅, -CH₂-CH(CH₃)-CH(CH₃)₂, -CH₂-C(CH₃)₂-C₂H₅, -C(CH₃)₂-C₃H₇, -C(CH₃)₂-CH(CH₃)₂, -C₂H₄-C(CH₃)₃, -CH(CH₃)-C(CH₃)₃, -CH=CH₂, -CH₂-CH=CH₂, -C(CH₃)=CH₂, -CH=CH-CH₃, -C₂H₄-CH=CH₂, -CH₂-CH=CH-CH₃, -CH=CH-C₂H₅, -CH₂-C(CH₃)=CH₂, -CH(CH₃)-CH=CH, -CH=C(CH₃)₂, -C(CH₃)=CH-CH₃, -CH=CH-CH=CH₂, -C₃H₆-CH=CH₂, -C₂H₄-CH=CH-CH₃, -CH₂-CH=CH-C₂H₅, -CH=CH-C₃H₇, -CH₂-CH=CH-CH=CH₂, -CH=CH-CH=CH-CH₃, -CH=CH-CH₂-CH=CH₂, -C(CH₃)=CH-CH=CH₂, -CH=C(CH₃)-CH=CH₂, -CH=CH-C(CH₃)=CH₂, -C₂H₄-C(CH₃)=CH₂, -CH₂-CH(CH₃)-CH=CH₂, -CH(CH₃)-CH₂-CH=CH₂, -CH₂-CH=C(CH₃)₂, -CH₂-C(CH₃)=CH-CH₃, -CH(CH₃)-CH=CH-CH₃, -CH=CH-CH(CH₃)₂, -CH=C(CH₃)-C₂H₅, -C(CH₃)=CH-C₂H₅, -C(CH₃)=C(CH₃)₂, -C(CH₃)₂-CH=CH₂, -CH(CH₃)-C(CH₃)=CH₂, -C(CH₃)=CH-CH=CH₂, -CH=C(CH₃)-CH=CH₂, -CH=CH-C(CH₃)=CH₂, -C₄H₈-CH=CH₂, -C₃H₆-CH=CH-CH₃, -C₂H₄-CH=CH-C₂H₅, -CH₂-CH=CH-C₃H₇, -CH=CH-C₄H₉, -C₃H₆-C(CH₃)=CH₂, -C₂H₄-CH(CH₃)-CH=CH₂, -CH₂-CH(CH₃)-CH₂-CH=CH₂, -C₂H₄-CH=C(CH₃)₂, -CH(CH₃)-C₂H₄-CH=CH₂, -C₂H₄-C(CH₃)=CH-CH₃, -CH₂-CH(CH₃)-CH=CH-CH₃, -CH(CH₃)-CH₂-CH=CH-CH₃, -CH₂-CH=CH-CH(CH₃)₂, -CH₂-CH=C(CH₃)-C₂H₅, -CH₂-C(CH₃)=CH-C₂H₅, -CH(CH₃)-CH=CH-C₂H₅, -CH=CH-CH₂-CH(CH₃)₂, -CH=CH-CH(CH₃)-C₂H₅, -CH=C(CH₃)-C₃H₇, -C(CH₃)=CH-C₃H₇, -CH₂-CH(CH₃)-C(CH₃)=CH₂, -C[C(CH₃)₃]=CH₂, -CH(CH₃)-CH₂-C(CH₃)=CH₂, -CH(CH₃)-CH(CH₃)-CH=CH₂, -CH=CH-C₂H₄-CH=CH₂, -CH₂-C(CH₃)₂-CH=CH₂, -C(CH₃)₂-CH₂-CH=CH₂, -CH₂-C(CH₃)=C(CH₃)₂, -CH(CH₃)-CH=C(CH₃)₂, -C(CH₃)₂-CH=CH-CH₃, -CH=CH-CH₂-CH=CH-CH₃, -CH(CH₃)-C(CH₃)=CH-CH₃, -CH=C(CH₃)-CH(CH₃)₂, -C(CH₃)=CH-CH(CH₃)₂, -C(CH₃)=C(CH₃)-C₂H₅, -CH=CH-C(CH₃)₃, -C(CH₃)₂-C(CH₃)=CH₂, -CH(C₂H₅)-C(CH₃)=CH₂, -C(CH₃)(C₂H₅)-CH=CH₂, -CH(CH₃)-C(C₂H₅)=CH₂, -CH₂-C(C₃H₇)=CH₂, -CH₂-C(C₂H₅)=CH-CH₃, -CH(C₂H₅)-CH=CH-CH₃, -C(C₄H₉)=CH₂, -C(C₃H₇)=CH-CH₃, -C(C₂H₅)=CH-C₂H₅, -C(C₂H₅)=C(CH₃)₂, -C[CH(CH₃)(C₂H₅)]=CH₂, -C[CH₂-CH(CH₃)₂]=CH₂, -C₂H₄-CH=CH-CH=CH₂, -CH₂-CH=CH-CH₂-CH=CH₂, -C₃H₆-C≡C-CH₃, -CH₂-CH=CH-CH=CH-CH₃, -CH=CH-CH=CH-C₂H₅, -CH₂-CH=CH-C(CH₃)=CH₂, -CH₂-CH=C(CH₃)-CH=CH₂, -CH₂-C(CH₃)=CH-CH=CH₂, -CH(CH₃)-CH₂-C≡CH, -CH(CH₃)-CH=CH-CH=CH₂, -CH=CH-CH₂-C(CH₃)=CH₂, -CH(CH₃)-C≡C-CH₃, -CH=CH-CH(CH₃)-CH=CH₂, -CH=C(CH₃)-CH₂-CH=CH₂, -C₂H₄-CH(CH₃)-C≡CH, -C(CH₃)=CH-CH₂-CH=CH₂, -CH=CH-CH=C(CH₃)₂, -CH₂-CH(CH₃)-CH₂-C≡CH, -CH=CH-C(CH₃)=CH-CH₃, -CH=C(CH₃)-CH=CH-CH₃, -CH₂-CH(CH₃)-C≡CH, -C(CH₃)=CH-CH=CH-CH₃, -CH=C(CH₃)-C(CH₃)=CH₂, -C(CH₃)=CH-C(CH₃)=CH₂, -C(CH₃)=C(CH₃)-CH=CH₂, -CH=CH-CH=CH-CH=CH₂, -C≡CH, -C≡C-CH₃, -CH₂-C≡CH, -C₂H₄-C≡CH, -CH₂-C≡C-CH₃, -C≡C-C₂H₅, -C₃H₆-C≡CH, -C₂H₄-C≡C-CH₃, -CH₂-C≡C-C₂H₅, -C≡C-C₃H₇, -CH(CH₃)-C≡CH, -C₄H₈-C≡CH, -C₂H₄-C≡C-C₂H₅, -CH₂-C≡C-C₃H₇, -C≡C-C₄H₉, -C≡C-C(CH₃)₃, -CH(CH₃)-C₂H₄-C≡CH, -CH₂-CH(CH₃)-C≡C-CH₃, -CH(CH₃)-CH₂-C≡C-CH₃, -CH(CH₃)-C≡C-C₂H₅, -CH₂-C≡C-CH(CH₃)₂, -C≡C-CH(CH₃)-C₂H₅, -C≡C-CH₂-CH(CH₃)₂, -CH(C₂H₅)-C≡C-CH₃, -C(CH₃)₂-C≡C-CH₃, -CH(C₂H₃)-CH₂-C≡CH, -CH₂-CH(C₂H₅)-C≡CH, -C(CH₃)₂-CH₂-C≡CH, -CH₂-C(CH₃)₂-C≡CH, -CH(CH₃)-CH(CH₃)-C≡CH, -CH(C₃H₇)-C≡CH, -C(CH₃)(C₂H₅)-C≡CH, -CH₂-CH(C≡CH)₂, -C≡C-C≡CH, -CH₂-C≡C-C≡CH, -C≡C-C≡C-CH₃, -CH(C≡CH)₂, -C₂H₄-C≡C-C≡CH, -CH₂-C≡C-CH₂-C≡CH, -C≡C-C₂H₄-C≡CH, -CH₂-C≡C-C≡C-CH₃, -C≡C-CH₂-C≡C-CH₃, -C≡C-C≡C-C₂H₅, -C(C≡CH)₂-CH₃, -C≡C-CH(CH₃)-C≡CH, -CH(CH₃)-C≡C-C≡CH, -CH(C≡CH)-CH₂-C≡CH, -CH(C≡CH)-C≡C-CH₃;
X4, X5 and X6 represent independently of each other:
a bond, -CH₂-, -C₂H₄-, -C₃H₆-, -C₄H₈-, -C₅H₁₀-, -C₆H₁₂-, -C₇H₁₄-, -C₈H₁₆-, -C₉H₁₈-, -C₁₀H₂₀-, -CH(CH₃)-, -C[(CH₃)₂]-, -CH₂-CH(CH₃)-, -CH(CH₃)-CH₂-, -CH(CH₃)-C₂H₄-, -CH₂-CH(CH₃)-CH₂-, -C₂H₄-CH(CH₃)-, -CH₂-C[(CH₃)₂]-, -C[(CH₃)₂]-CH₂-, -CH(CH₃)-CH(CH₃)-, -C[(C₂H₅)(CH₃)]-, -CH(C₃H₇)-, -CH₂CH₂O-, -(CH₂-CH₂-O)ₘ-CH₂-CH₂-, -C(CH₃)=CH-C(CH₃)=CH-, -C₂H₄-CH=CH-CH=CH-, -CH₂-CH=CH-CH₂-CH=CH-, -C₃H₆-C≡C-CH₂-, -CH₂-CH=CH-CH=CH-CH₂-, -CH=CH-CH=CH-C₂H₄-, -CH₂-CH=CH-C(CH₃)=CH-, -CH₂-CH=C(CH₃)-CH=CH-, -CH₂-C(CH₃)=CH-CH=CH-, -CH(CH₃)-CH=CH-CH=CH-, -CH=CH-CH₂-C(CH₃)=CH-, -CH(CH₃)-C≡C-CH₂-, -CONH-, -NHCO-, -CH₂-CONH-, -CONH-CH₂-, -NHCO-CH₂-, -CH₂-NHCO-;
wherein m is an integer from 1 to 10; or
**X⁵-R^{C}** and **X⁶-R^{D}** can form together a cyclic ring selected from the group consisting of: 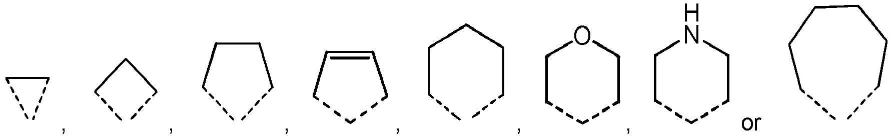 and enantiomers, stereoisomeric forms, mixtures of enantiomers, anomers, deoxy-forms, diastereomers, mixtures of diastereomers, prodrugs, tautomers, hydrates, solvates and racemates of the above mentioned compounds and pharmaceutically acceptable salts thereof.

3. The compound according to any of the previous claims, wherein FM has the structure of: 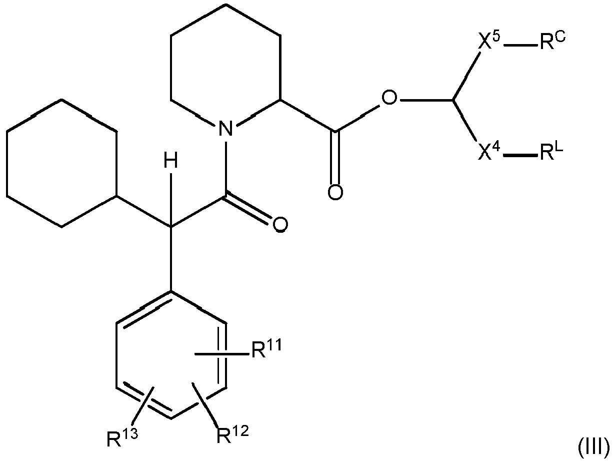 wherein
**X⁴** represents bond, -C₂H₄-, or -CH₂O-;
and **X⁵, R^{C}, R^{L}, R¹¹, R¹², R¹³** represents the same residues as defined in claim 1.
and enantiomers, stereoisomeric forms, mixtures of enantiomers, anomers, deoxy-forms, diastereomers, mixtures of diastereomers, prodrugs, tautomers, hydrates, solvates and racemates of the above mentioned compounds and pharmaceutically acceptable salts thereof.

4. The compound according to any of the previous claims, wherein **FM** has the structure of: 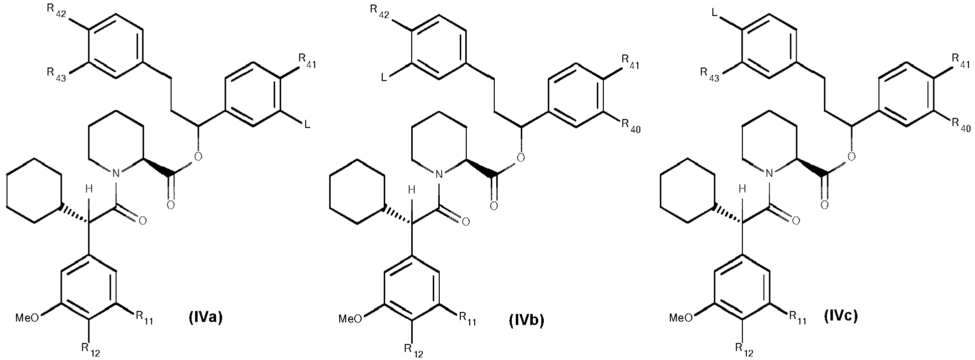
wherein L represents the chemical linker;
and **R¹¹, R¹², R⁴⁰, R⁴¹**, **R⁴²,** and **R⁴³** represents the same residues as defined in claim 1;
and enantiomers, stereoisomeric forms, mixtures of enantiomers, anomers, deoxy-forms, diastereomers, mixtures of diastereomers, prodrugs, tautomers, hydrates, solvates and racemates of the above mentioned compounds and pharmaceutically acceptable salts thereof.

5. The compound according to any of the previous claims, wherein **EM** is represented by one of the following general Formulas (EM-1), (EM-2) and/or (EM-3): 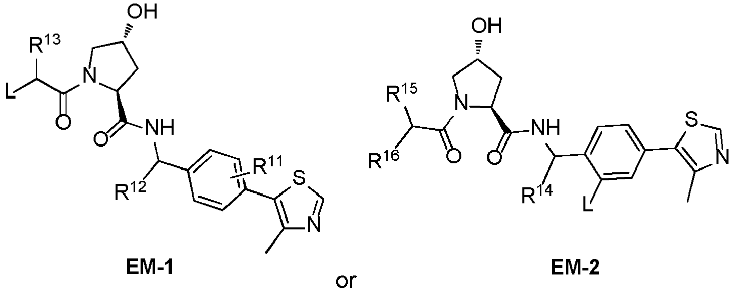 or 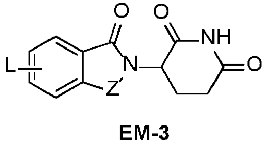 wherein
**R¹²** and **R¹⁴** represent independently -H or -CH3;
**R¹³** and **R¹⁵** represent -H, -CH3, -CH2CH3, -CH(CH3)2, -C(CH3)3, -C(SH)(CH3)2;
**R¹⁶** represents 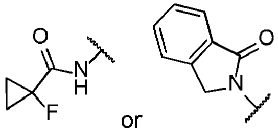
**Z** represents -CH2-, -CO-;
L represents the chemical linker L as defined in claim 1;
and enantiomers, stereoisomeric forms, mixtures of enantiomers, anomers, deoxy-forms, diastereomers, mixtures of diastereomers, prodrugs, tautomers, hydrates, solvates and racemates of the above mentioned compounds and pharmaceutically acceptable salts thereof.

6. The compound of any of the previous claims, wherein **EM** is selected from a group consisting of a ligand of ubiquitin E3 ligase cereblon-ligand, an ubiquitin E3 ligase VHL-ligand and/or other E3-ligase ligand.

7. The compound of any of the previous claims, wherein **EM** is selected from the group consisting of (4R,5S)-nutlin carboxylic acid, (S,R,S)-AHPC hydrochloride, (S,R,S)-AHPC TFA, (S,R,S)-AHPC, (S,R,S)-AHPC-Me dihydrochloride, (S,R,S)-AHPC-Me hydrochloride, (S,R,S)-AHPC-Me, (S,R,S)-AHPC-propargyl, AhR Ligand 1, BC-1215, Bestatin-amido-Me, CC-885, clAP1 ligand 1, clAP1 ligand 2, E3 ligase Ligand 10, E3 ligase Ligand 13, E3 ligase Ligand 14, E3 ligase Ligand 18, E3 ligase Ligand 8, E3 ligase Ligand 9, VH032-cyclopropane-F, Iberdomide, Lenalidomide (hemihydrate), Lenalidomide hydrochloride, Lenalidomide, Lenalidomide-Br, MV-1-NH-Me, Nutlin carboxylic acid, NV03, Pomalidomide, TD-106, Thalidomide D4, Thalidomide fluoride, Thalidomide, Thalidomide-O-COOH, Thalidomide-propargyl, Nutlin, Idasanutlin, Bestatin Esters, VH032, VH032 thiol, VH-298, VHL Ligand 8, VL285, and/or any combination thereof.

8. The compound of any of the previous claims, wherein the PROTAC leads to a degradation of a FKBP, selected from FKBP51 , or any combination thereof.

9. The compound of any of the previous claims, represented by one of the following formulas: 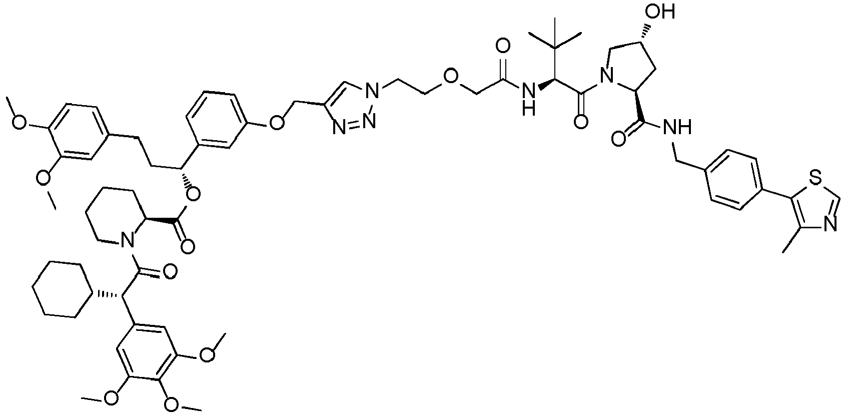 (S)-(R)-3-(3,4-Dimethoxyphenyl)-1-(3-((1-(2-(2-(((S)-1-((2S,4R)-4-hydroxy-2-((4-(4-methylthiazol-5-yl)benzyl)carbamoyl)pyrrolidin-1-yl)-3,3-dimethyl-1-oxobutan-2-yl)amino)-2-oxoethoxy)ethyl)-1H-1,2,3-triazol-4-yl)methoxy)phenyl)propyl 1-((S)-2-cyclohexyl-2-(3,4,5-trimethoxyphenyl)acetyl)piperidine-2-carboxylate, or 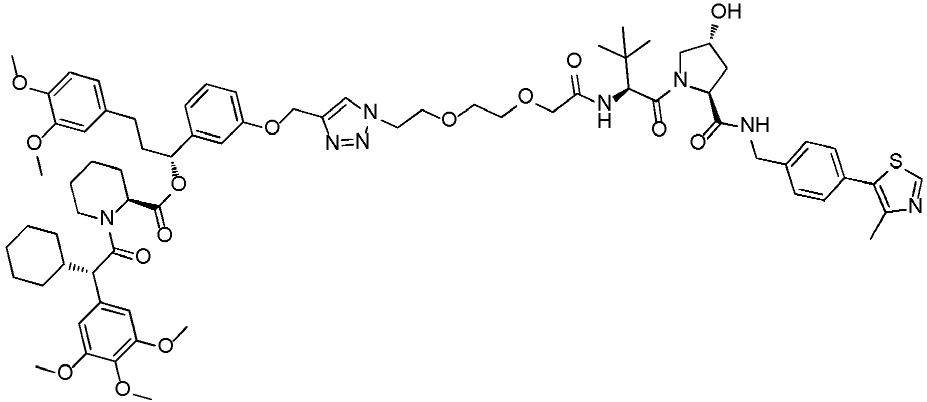 (S)-(R)-3-(3,4-Dimethoxyphenyl)-1-(3-((1-(2-(2-(2-(((S)-1-((2S,4R)-4-hydroxy-2-((4-(4-methylthiazol-5-yl)benzyl)carbamoyl)pyrrolidin-1-yl)-3,3-dimethyl-1-oxobutan-2-yl)amino)-2-oxoethoxy)ethoxy)ethyl)-1 H-1 ,2,3-triazol-4-yl)methoxy)phenyl)propyl 1-((S)-2-cyclohexyl-2-(3,4,5-trimethoxyphenyl)acetyl)piperidine-2-carboxylate,
or 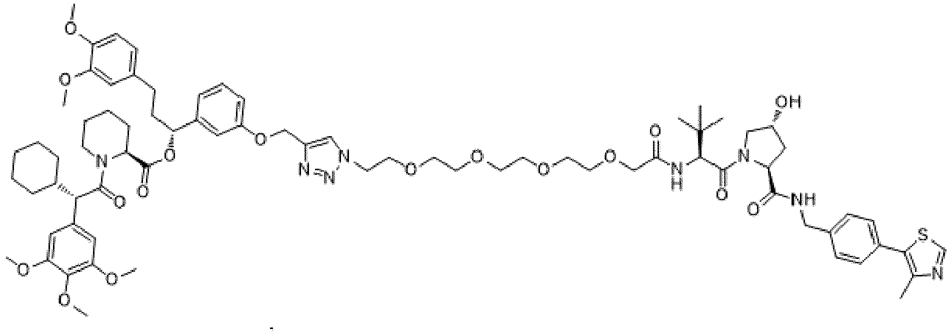 IUPAC nomenclature: (S)-(R)-3-(3,4-Dimethoxyphenyl)-1-(3-((1-((S)-16-((2S,4R)-4-hydroxy-2-((4-(4-methylthiazol-5-yl)benzyl)carbamoyl)pyrrolidine-1-carbonyl)-17,17-dimethyl-14-oxo-3,6,9,12-tetraoxa-15-azaoctadecyl)-1H-1,2,3-triazol-4-yl)methoxy)phenyl)propyl 1-((S)-2-cyclohexyl-2-(3,4,5-trimethoxyphenyl)acetyl)piperidine-2-carboxylate;
and enantiomers, stereoisomeric forms, mixtures of enantiomers, anomers, deoxy-forms, diastereomers, mixtures of diastereomers, prodrugs, tautomers, hydrates, solvates and racemates of the above mentioned compounds and pharmaceutically acceptable salts thereof.

10. Use of compound of any of the previous claims for treatment or prophylaxis of a disease selected from psychiatric disorder such as depression and posttraumatic stress disorder, cancers such glioblastoma, prostate cancer and malignant carcinoma, metabolic disorders such as obesity and diabetes, pain disorders such as neuropathic pain or fibromyalgia, glucocorticoid hyposensitivity syndrome or asthma, and for neuroprotection, neuroregeneration, stimulating neurite growth, wound healing,

11. Use according to claim 8, wherein the psychiatric disorder is an affective disorder or an anxiety disorder;
wherein the affective disorder is selected from the group consisting of depression, bipolar disorder, mania, substance induced mood disorder and seasonal affective disorder (SAD); and
wherein the anxiety disorder is selected from the group consisting of generalized anxiety disorder, panic disorder, panic disorder with agoraphobia, phobias, obsessive-compulsive disorder, post-traumatic stress disorder, separation anxiety and childhood anxiety disorders.

12. Pharmaceutical composition comprising at least one compound according to claims 1 to 9 together with at least one pharmaceutically acceptable carrier, solvent or excipient.

13. Pharmaceutical composition according to any of the previous claims further comprising at least one active agent selected from the group consisting of an anti-depressant and other psychotropic drugs.

14. Pharmaceutical composition according to of any of the previous claims, wherein the anti-depressant is selected from amitriptyline, amioxide clomipramine, doxepine, duloxetine, imipramine trimipramine, mirtazapine, reboxetine, citaloprame, fluoxetine, moclobemide and sertraline.

15. Method for producing the compound of any of claims 1 to 9 or the pharmaceutical composition of any of claims 12 to 14.
